# EUROPEAN PATENT APPLICATION

(11) **EP 4 647 491 A2**
(43) Date of publication of application: **12.11.2025**
(21) Application number: 25191454.5
(22) Date of filing: 14.05.2020
(51) Int. Cl.: C12N 5/07

(54) **HIGH-DENSITY MICROCHANNELS**

(30) Priority: 14.05.2019 US 201962847771 P
(62) Divisional of application: 20806078.0
(71) Applicant: Spiderwort Biotechnologies Inc., Ottawa, Ontario K1Y 2C5 (CA)
(72) Inventor: FERKO, Maxime-Alexandre, Kitchener, N2E 3C2 (CA); CUERRIER, Charles, Gatineau, J9J 0S7 (CA); LEBLANC LATOUR, Maxime, Gatineau, J9J 0S7 (CA); HANSON, Stephen, Ottawa, K2G 6V2 (CA); MODULEVSKY, Daniel, Ottawa, K2M 2Z8 (CA); PELLING, Andrew E., Ottawa, K1J 6A4 (CA); SZERESZEWSKI, Kama, Ottawa, K4A 3T4 (CA)
(74) Representative: Brann AB

(57) **Abstract**

Provided herein are scaffold biomaterials including at least one bundle of microchannels, the bundle of microchannels having a plurality of decellularised microchannels isolated from plant or fungal tissue, the decellularised microchannels being arranged substantially parallel to each other within the bundle. Also provided are methods and uses of such scaffold biomaterials and bundles, as well as methods for the production of such scaffold biomaterials and bundles.

## Description

### FIELD OF INVENTION

The present invention relates generally to scaffold biomaterials. More specifically, the present invention relates to high-density decellularised microchannel bundles derived from plant tissue, and uses thereof as scaffold biomaterials.

### BACKGROUND

The biomaterials industry is estimated to have a market value of $90 Billion USD and is driven by novel materials derived from natural sources, synthetic polymers, metals, and ceramics. These materials can form three dimensional high porosity scaffolds possessing nano/microscale structures that are biocompatible and promote the growth of living cells. There is intense interest in novel biomaterials which support the invasion and proliferation of living cells for potential applications in tissue engineering and regenerative medicine, for example.

Biomaterial scaffolds have applications in multiple sectors, including dental and cosmetic surgery, clinical and medical therapies (such as regenerative medicine, wound healing, tissue engineering and repair, etc.), and research & development (including industry and academic research in the biomedical sciences).

Commercial biomaterials often require complicated and time consuming production methods, which leads to a high cost to the end user, even if they are not approved for human use. In addition, most commercial biomaterials are derived from human/animal origin, resulting in potential rejection by the body and/or adverse immune responses and/or risk of disease transmission. The source materials can also have negative environmental impact, and can also lead to problems with unethical sourcing. Also, some commercial biomaterials lose their shape after implantation, which can result in reduced success of the tissue repair/replacement.

In the field, there is a general lack of viable commercial biomaterial solutions to replace directional, channel-like tissues and organs for promoting tissue repair and/or regeneration. In the field of spinal cord injuries (SCI), for example, most academic research is currently focused on injectable hydrogels containing various factors intended to modulate and promote neuronal growth. However, hydrogels often lack directionality and neuronal guiding ability.

Alternative, additional, and/or improved scaffold biomaterials, and methods for the production thereof, are desirable.

### SUMMARY OF INVENTION

Provided herein are materials (biomaterials) that may be used to mimic the directional, channel-like microscopic structures of certain tissues in humans, plants, and animals. Examples of such directional tissues may include the extracellular matrices (ECM) of the spinal cord, vascular channels, lymphatic tissue, nervous tissue, and many others. It is an object of the present invention to provide biocompatible materials possessing directionality and/or channel-like structure, as well as production methods and methods and uses thereof.

In an embodiment, there is provided herein a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle.

In another embodiment of the above scaffold biomaterial, the decellularised microchannels may comprise decellularised xylem and/or phloem channels.

In another embodiment of any of the above scaffold biomaterials, the decellularised xylem and/or phloem channels may be individually isolated from plant or fungal tissue, may be grouped in one or more vascular bundles isolated from plant or fungal tissue, or any combination thereof.

In another embodiment of any of the above scaffold biomaterials, the decellularised microchannels are physically bundled substantially without glue, for example by entanglement.

In still another embodiment of any of the above scaffold biomaterials, the plurality of decellularised microchannels may be glued together within the bundle.

In yet another embodiment of any of the above scaffold biomaterials, the plurality of decellularised microchannels may be glued together within the bundle by a biocompatible, optionally biodegradable, optionally low-volume expansion, glue.

In another embodiment of any of the above scaffold biomaterials, the biocompatible glue may comprise a PEG-based, polyurethane-based, gelatin-based, or a fibrin-based glue.

In still another embodiment of any of the above scaffold biomaterials, the biocompatible glue may comprise a fibrin-based glue.

In yet another embodiment of any of the above scaffold biomaterials, the decellularised microchannels may be cellulose-based, chitin-based, lignin-based, hemicellulose-based, or pectin-based, or any combination thereof.

In another embodiment of any of the above scaffold biomaterials, a density of decellularised microchannels within the bundle may be greater than a density of microchannels within the plant or fungal tissue.

In still another embodiment of any of the above scaffold biomaterials, the plant or fungal tissue may comprise a microchannel-containing tissue from apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, a rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue, or a genetically altered tissue produced via direct genome modification or through selective breeding, or any combinations thereof.

In yet another embodiment of any of the above scaffold biomaterials, the plant or fungal tissue may comprise celery, asparagus, or both.

In another embodiment of any of the above scaffold biomaterials, the scaffold biomaterial may further comprise living cells, in particular non-native cells, on and/or within at least one of the decellularised microchannels.

In still another embodiment of any of the above scaffold biomaterials, the living cells may be animal cells.

In yet another embodiment of any of the above scaffold biomaterials, the living cells may be mammalian cells.

In another embodiment of any of the above scaffold biomaterials, the living cells may be human cells.

In another embodiment of any of the above scaffold biomaterials, the decellularised microchannels may be separated from plant or fungal matter by a liquid-based extraction.

In another embodiment any of the above scaffold biomaterials, separation is may be by liquid-based extraction of the microchannels from the plant or fungal tissue.

In another embodiment any of the above scaffold biomaterials, liquid-based extraction may comprise at least one of acid extraction, acid and peroxide extraction, salt extraction or alkaline extraction.

In another embodiment any of the above scaffold biomaterials, acid and peroxide extraction may comprise heating the plant or fungal tissue in an acid and peroxide solution.

In another embodiment any of the above scaffold biomaterials, the acid and peroxide solution may comprise glacial acetic acid and 30% hydrogen peroxide in a ratio of 5:1 to 1:5, such as 3:1 to 1:3.

In another embodiment any of the above scaffold biomaterials, the acid and peroxide solution may comprise 1:1 glacial acetic acid:hydrogen peroxide solution (30% v/v).

In another embodiment any of the above scaffold biomaterials, heating may comprise heating for a period of up to 30 minutes or more.

In another embodiment any of the above scaffold biomaterials, heating may comprise heating for 30 minutes.

In another embodiment any of the above scaffold biomaterials, heating may comprise boiling the acid and peroxide solution.

In another embodiment any of the above scaffold biomaterials, the acid and peroxide solution may comprise 1:1 glacial acetic acid:hydrogen peroxide solution (30% v/v) and the solution is heated to boiling for 30 minutes.

In another embodiment any of the above scaffold biomaterials, the liquid-based extraction may further comprise mechanically agitating, for example stirring, the plant or fungal tissue in the acid and peroxide solution.

In another embodiment any of the above scaffold biomaterials, salt extraction may comprise heating the plant or fungal tissue in a salt solution.

In another embodiment any of the above scaffold biomaterials, the salt solution may comprise LiCl or NaCl.

In another embodiment any of the above scaffold biomaterials, the salt solution may comprise a salt concentration of about 0.5M-10M, such as 0.5M-3M.

In another embodiment any of the above scaffold biomaterials, the salt solution may comprise LiCl or NaCl at a salt concentration of about 3M.

In another embodiment any of the above scaffold biomaterials, heating may comprise heating up to a period of 30 minutes or more.

In another embodiment any of the above scaffold biomaterials, heating may comprise heating for 30 minutes.

In another embodiment any of the above scaffold biomaterials, heating may comprise boiling the salt solution.

In another embodiment any of the above scaffold biomaterials, the salt solution may comprise about 3M LiCl and the solution may be heated to boiling for 30 minutes.

In another embodiment any of the above scaffold biomaterials, the salt solution may comprise about 3M NaCl and the solution may be heated to boiling for 15 minutes.

In another embodiment any of the above scaffold biomaterials, the liquid-based extraction may further comprises mechanically agitating, for example stirring, the plant or fungal tissue in the salt solution.

In another embodiment any of the above scaffold biomaterials, alkaline extraction may comprise heating the plant or fungal tissue in an alkaline solution.

In another embodiment any of the above scaffold biomaterials, the alkaline solution may comprise sodium hydroxide (NaOH).

In another embodiment any of the above scaffold biomaterials, the alkaline solution may comprise an alkaline concentration of about 0.5-10M, such as 0.5M-3M.

In another embodiment any of the above scaffold biomaterials, the alkaline solution may comprise sodium hydroxide at an alkaline concentration of about 0.5M-1M.

In another embodiment any of the above scaffold biomaterials, heating may comprise heating up to 30 minutes.

In another embodiment any of the above scaffold biomaterials, heating may comprise heating for 30 minutes.

In another embodiment any of the above scaffold biomaterials, heating may comprise boiling the alkaline solution.

In another embodiment any of the above scaffold biomaterials, the alkaline solution may comprise about 0.5M NaOH and the solution is heated to boiling for 5 minutes.

In another embodiment any of the above scaffold biomaterials, the liquid-based extraction may further comprise mechanically agitating, for example stirring, the plant or fungal tissue in the alkaline solution.

In another embodiment any of the above scaffold biomaterials, acid extraction may comprise heating the plant or fungal tissue in an acid solution comprising acid.

In another embodiment any of the above scaffold biomaterials, the acid solution may comprise acidic acid or hydrochloric acid.

In another embodiment any of the above scaffold biomaterials, the acid solution may comprise 50% acetic acid.

In another embodiment any of the above scaffold biomaterials, heating may comprise heating for a period of up to 30 minutes or more.

In another embodiment any of the above scaffold biomaterials, heating may comprise heating for 30 minutes.

In another embodiment any of the above scaffold biomaterials, heating may comprise boiling the acid solution.

In another embodiment any of the above scaffold biomaterials, the acid solution may comprise 50% acetic acid and the solution is heated to boiling for 30 minutes.

In another embodiment any of the above scaffold biomaterials, the liquid-based extraction may further comprise mechanically agitating, for example stirring, the plant or fungal tissue in the acid solution.

In another embodiment any of the above scaffold biomaterials, the step of decellularising may occur before the step of separating.

In another embodiment, there is provided herein a use of any of the scaffold biomaterials as described herein for supporting animal or plant cell growth; promoting tissue regeneration; promoting angiogenesis; treatment and/or repair of spinal cord injury; repair or reconstruction or replacement of plant or animal tissue; solution or material filtration and/or separation; plant modification or growth modulation; material transport; assembly for mimicking a desired shape or object; and/or microfluidics; or any suitable application in which biocompatible micrometer-scale channels may be of use.

In another embodiment of the above use, the scaffold biomaterial may be for repair or reconstruction or replacement of plant or animal tissue, wherein the plant or animal tissue may comprise damaged microvasculature, or plant tissue damaged by vascular disease (such as wilt disease), or damaged vascular structure of trees infested with an insect (such as emerald ash borer).

In another embodiment of any of the above uses, the scaffold biomaterial may be for repair or reconstruction of plant or animal tissue, wherein the plant or animal tissue may comprise damaged microvasculature, wherein angiogenesis is impaired or compromised.

In still another embodiment of any of the above uses, the scaffold biomaterial may be for repair or reconstruction of animal tissue, wherein the animal is human.

In yet another embodiment of any of the above uses, the scaffold biomaterial may be for plant modification or growth modulation, wherein the modification may be modification of a grafting process for accelerated growth.

In another embodiment of any of the above uses, the scaffold biomaterial may be for material transport, wherein the material transport is a drug delivery application.

In still another embodiment of any of the above uses, the scaffold biomaterial may be for heat transfer or heat exchange microfluidics.

In another embodiment, there is provided herein a method for supporting cell growth, said method comprising:
providing any of the scaffold biomaterials as described herein; and
introducing one or more cells to the scaffold biomaterial.

In another embodiment, there is provided herein a method for promoting tissue regeneration; promoting angiogenesis; treatment and/or repair of spinal cord injury; or repair or reconstruction or replacement of plant or animal tissue, said method comprising:
providing any of the scaffold biomaterials as described herein; and
implanting said scaffold biomaterial at a site in need thereof.

In another embodiment there is provided herein a method for solution or material filtration and/or separation, said method comprising:
providing any of the scaffold biomaterials as described herein; and
passing a solution or material through the scaffold biomaterial so as to filter and/or separate components from the solution based on size exclusion.

In another embodiment there is provided herein a method for plant modification or growth modulation, said method comprising:
providing any of the scaffold biomaterials as described herein; and
grafting the scaffold biomaterial into the plant to provide for accelerated growth.

In another embodiment, there is provided herein a method for material transport, said method comprising:
providing any of the scaffold biomaterials as described herein; and
using the scaffold biomaterial to transport a material to a site in need thereof.

In another embodiment of the above method, the material may be a drug, and the scaffold biomaterial may be used for drug delivery.

In another embodiment, there is provided herein a method for preparing a structure mimicking a desired shape or object, said method comprising:
providing any of the scaffold biomaterials as described herein;
carving or arranging the scaffold biomaterial so as to mimic the desired shape or object; and
optionally, gluing the scaffold biomaterial for structural reinforcement.

In another embodiment, there is provided herein a method for exchanging or transferring heat in a microfluidics process, said method comprising:
providing any of the scaffold biomaterials as described herein; and
using the microchannels of the scaffold biomaterial to carry one or more fluids, wherein the one or more fluids are in close proximity so as to allow for heat exchange or heat transfer.

In another embodiment there is provided herein a method for isolation and decellularisation of microchannels from a plant or fungal tissue, said method comprising:
separating microchannels from the plant or fungal tissue; and
decellularising the microchannels; and
optionally, sterilizing the microchannels.

In another embodiment of the above method, the step of separating the microchannels may comprise mechanical separation of the microchannels, or vascular bundles containing the microchannels, or both, from surrounding plant or fungal tissue.

In yet another embodiment of the above methods, the step of separating may be performed by gentle peeling, or by cutting.

In another embodiment of any of the above method or methods, the decellularised microchannels may be separated from plant or fungal matter by a liquid-based extraction.

In another embodiment any of the above method or methods, separation is may be by liquid-based extraction of the microchannels from the plant or fungal tissue.

In another embodiment any of the above method or methods, liquid-based extraction may comprise at least one of acid extraction, acid and peroxide extraction, salt extraction or alkaline extraction.

In another embodiment any of the above method or methods, acid and peroxide extraction may comprise heating the plant or fungal tissue in an acid and peroxide solution.

In another embodiment any of the above method or methods, the acid and peroxide solution may comprise glacial acetic acid and 30% hydrogen peroxide in a ratio of 5:1 to 1:5, such as 3:1 to 1:3.

In another embodiment any of the above method or methods, the acid and peroxide solution may comprise 1:1 glacial acetic acid:hydrogen peroxide solution (30%).

In another embodiment any of the above method or methods, heating may comprise heating for a period of up to 30 minutes or more, such as up to 45 minutes or up to 1 hour.

In another embodiment any of the above method or methods, heating may comprise heating for 30 minutes.

In another embodiment any of the above method or methods, heating may comprise boiling the acid and peroxide solution.

In another embodiment any of the above method or methods, the acid and peroxide solution may comprise 1:1 glacial acetic acid:hydrogen peroxide solution (30% v/v) and the solution is heated to boiling for 30 minutes.

In another embodiment any of the above method or methods, the liquid-based extraction may further comprise mechanically agitating, for example stirring, the plant or fungal tissue in the acid and peroxide solution.

In another embodiment any of the above method or methods, salt extraction may comprise heating the plant or fungal tissue in a salt solution.

In another embodiment any of the above method or methods, the salt solution may comprise LiCl or NaCl.

In another embodiment any of the above method or methods, the salt solution may comprise a salt concentration of about 0.5M-10M, such as 0.5M-3M.

In another embodiment any of the above method or methods, the salt solution may comprise LiCl or NaCl at a salt concentration of about 3M.

In another embodiment any of the above method or methods, heating may comprise heating up to a period of 30 minutes or more, such as up to 45 minutes or up to 1 hour.

In another embodiment any of the above method or methods, heating may comprise heating for 30 minutes.

In another embodiment any of the above method or methods, heating may comprise boiling the salt solution.

In another embodiment any of the above method or methods, the salt solution may comprise about 3M LiCl and the solution may be heated to boiling for 30 minutes.

In another embodiment any of the above method or methods, the salt solution may comprise about 3M NaCl and the solution may be heated to boiling for 15 minutes.

In another embodiment any of the above method or methods, the liquid-based extraction may further comprises mechanically agitating, for example stirring, the plant or fungal tissue in the salt solution.

In another embodiment any of the above method or methods, alkaline extraction may comprise heating the plant or fungal tissue in an alkaline solution.

In another embodiment any of the above method or methods, the alkaline solution may comprise sodium hydroxide (NaOH).

In another embodiment any of the above method or methods, the alkaline solution may comprise an alkaline concentration of about 0.5-10M, such as 0.5M-3M.

In another embodiment any of the above method or methods, the alkaline solution may comprise sodium hydroxide at an alkaline concentration of about 0.5M-1M.

In another embodiment any of the above method or methods, heating may comprise heating up to 30 minutes or more, such as up to 45 minutes or up to 1 hour.

In another embodiment any of the above method or methods, heating may comprise heating for 30 minutes, or more such as up to 45 minutes or up to 1 hour.

In another embodiment any of the above method or methods, heating may comprise boiling the alkaline solution.

In another embodiment any of the above method or methods, the alkaline solution may comprise about 0.5M NaOH and the solution is heated to boiling for 5 minutes.

In another embodiment any of the above method or methods, the liquid-based extraction may further comprise mechanically agitating, for example stirring, the plant or fungal tissue in the alkaline solution.

In another embodiment any of the above method or methods, acid extraction may comprise heating the plant or fungal tissue in an acid solution comprising acid.

In another embodiment any of the above method or methods, the acid solution may comprise acidic acid or hydrochloric acid.

In another embodiment any of the above method or methods, the acid solution may comprise 50% acetic acid.

In another embodiment any of the above method or methods, heating may comprise heating for a period of up to 30 minutes or more.

In another embodiment any of the above method or methods, heating may comprise heating for 30 minutes.

In another embodiment any of the above method or methods, heating may comprise boiling the acid solution.

In another embodiment any of the above method or methods, the acid solution may comprise 50% acetic acid and the solution is heated to boiling for 30 minutes.

In another embodiment any of the above method or methods, the liquid-based extraction may further comprise mechanically agitating, for example stirring, the plant or fungal tissue in the acid solution.

In another embodiment any of the above method or methods, the step of decellularising may occur before the step of separating.

In another embodiment, there is provided herein a method for preparing a scaffold biomaterial, said method comprising:
separating microchannels from a plant or fungal tissue, or providing microchannels already separated from a plant or fungal tissue;
decellularising the microchannels;
bundling the microchannels together, the microchannels being arranged substantially parallel to each other;
thereby providing a scaffold biomaterial comprising bundled microchannels.

In yet another embodiment of the above method, the step of separating the microchannels may comprise mechanical separation of the microchannels, or vascular bundles containing the microchannels, or both, from surrounding plant or fungal tissue.

In another embodiment of any of the above method or methods, the step of separating may be performed by gentle peeling, or by cutting.

In another embodiment of any of the above method or methods, the decellularised microchannels may be separated from plant or fungal matter by a liquid-based extraction.

In another embodiment any of the above method or methods, separation is may be by liquid-based extraction of the microchannels from the plant or fungal tissue.

In another embodiment any of the above method or methods, liquid-based extraction may comprise at least one of acid extraction, acid and peroxide extraction, salt extraction or alkaline extraction.

In another embodiment any of the above method or methods, acid and peroxide extraction may comprise heating the plant or fungal tissue in an acid and peroxide solution.

In another embodiment any of the above method or methods, the acid and peroxide solution may comprise glacial acetic acid and 30% hydrogen peroxide in a ratio of 5:1 to 1:5, such as 3:1 to 1:3.

In another embodiment any of the above method or methods, the acid and peroxide solution may comprise 1:1 glacial acetic acid:hydrogen peroxide solution (30%).

In another embodiment any of the above method or methods, heating may comprise heating for a period of up to 30 minutes or more, such as up to 45 minutes or up to 1 hour.

In another embodiment any of the above method or methods, heating may comprise heating for 30 minutes.

In another embodiment any of the above method or methods, heating may comprise boiling the acid and peroxide solution.

In another embodiment any of the above method or methods, the acid and peroxide solution may comprise 1:1 glacial acetic acid:hydrogen peroxide solution (30% v/v) and the solution is heated to boiling for 30 minutes.

In another embodiment any of the above method or methods, the liquid-based extraction may further comprise mechanically agitating, for example stirring, the plant or fungal tissue in the acid and peroxide solution.

In another embodiment any of the above method or methods, salt extraction may comprise heating the plant or fungal tissue in a salt solution.

In another embodiment any of the above method or methods, the salt solution may comprise LiCl or NaCl.

In another embodiment any of the above method or methods, the salt solution may comprise a salt concentration of about 0.5M-10M, such as 0.5M-3M.

In another embodiment any of the above method or methods, the salt solution may comprise LiCl or NaCl at a salt concentration of about 3M.

In another embodiment any of the above method or methods, heating may comprise heating up to a period of 30 minutes or more, such as up to 45 minutes or up to 1 hour.

In another embodiment any of the above method or methods, heating may comprise heating for 30 minutes.

In another embodiment any of the above method or methods, heating may comprise boiling the salt solution.

In another embodiment any of the above method or methods, the salt solution may comprise about 3M LiCl and the solution may be heated to boiling for 30 minutes.

In another embodiment any of the above method or methods, the salt solution may comprise about 3M NaCl and the solution may be heated to boiling for 15 minutes.

In another embodiment any of the above method or methods, the liquid-based extraction may further comprises mechanically agitating, for example stirring, the plant or fungal tissue in the salt solution.

In another embodiment any of the above method or methods, alkaline extraction may comprise heating the plant or fungal tissue in an alkaline solution.

In another embodiment any of the above method or methods, the alkaline solution may comprise sodium hydroxide (NaOH).

In another embodiment any of the above method or methods, the alkaline solution may comprise an alkaline concentration of about 0.5M-10M, such as 0.5M-3M.

In another embodiment any of the above method or methods, the alkaline solution may comprise sodium hydroxide at an alkaline concentration of about 0.5M-1M.

In another embodiment any of the above method or methods, heating may comprise heating up to 30 minutes or more, such as up to 45 minutes or up to 1 hour.

In another embodiment any of the above method or methods, heating may comprise heating for 30 minutes.

In another embodiment any of the above method or methods, heating may comprise boiling the alkaline solution.

In another embodiment any of the above method or methods, the alkaline solution may comprise about 0.5M NaOH and the solution is heated to boiling for 5 minutes.

In another embodiment any of the above method or methods, the liquid-based extraction may further comprise mechanically agitating, for example stirring, the plant or fungal tissue in the alkaline solution.

In another embodiment any of the above method or methods, acid extraction may comprise heating the plant or fungal tissue in an acid solution comprising acid.

In another embodiment any of the above method or methods, the acid solution may comprise acidic acid or hydrochloric acid.

In another embodiment any of the above method or methods, the acid solution may comprise 50% acetic acid.

In another embodiment any of the above method or methods, heating may comprise heating for a period of up to 30 minutes or more.

In another embodiment any of the above method or methods, heating may comprise heating for 30 minutes.

In another embodiment any of the above method or methods, heating may comprise boiling the acid solution.

In another embodiment any of the above method or methods, the acid solution may comprise 50% acetic acid and the solution is heated to boiling for 30 minutes.

In another embodiment any of the above method or methods, the liquid-based extraction may further comprise mechanically agitating, for example stirring, the plant or fungal tissue in the acid solution.

In another embodiment any of the above method or methods, the step of decellularising may occur before the step of separating.

In another embodiment of any of the above scaffold biomaterials, the decellularised microchannels are physically bundled substantially without glue, for example by entanglement.

In another embodiment of any of the above method or methods, the step of bundling may comprise gluing the microchannels together.

In yet another embodiment of any of the above method or methods, the microchannels may be glued together by a biocompatible, optionally biodegradable, glue.

In still another embodiment of any of the above method or methods, the biocompatible glue may comprise a PEG-based, polyurethane-based, gelatin-based, or a fibrin-based glue.

In another embodiment of any of the above method or methods, the biocompatible glue may comprise a fibrin-based glue.

In yet another embodiment of any of the above method or methods, the step of bundling may comprise molding the microchannels.

In still another embodiment of any of the above method or methods, the molding may be performed using a mold into which the microchannels are packed.

In yet another embodiment of any of the above method or methods, the mold may comprise biomedical-grade silicone.

In another embodiment of any of the above method or methods, the mold may comprise a passage for receiving the microchannels therein, the passage having cross-sectional dimensions which are imparted to the microchannels being molded therein.

In still another embodiment of any of the above method or methods, the mold may be divided into two or more sections, which may be assembled around the microchannels for molding.

In another embodiment of any of the above method or methods, glue may be added to a surface of the mold facing the microchannels, and the microchannels may be packed in the mold and held therein while the glue cures.

In still another embodiment of any of the above method or methods, the mold may comprise a first section and a second section, and the molding may comprise adding glue to a surface of the first section facing the microchannels and to a surface of the second section facing the microchannels; placing the microchannels in the first section and the second section in contact with the glue; adding glue to an exposed surface of the microchannels in the first section, the microchannels in the second section, or both; installing the second section with microchannels over the exposed surface of the microchannels of the first section, opposite the first section, thereby assembling the mold; allowing the glue to substantially cure; and removing the mold.

In another embodiment of any of the above method or methods, the method may further comprise a step of cutting the bundled microchannels to a desired length.

In another embodiment of any of the above method or methods, the method may further comprise a step of cutting the bundled microchannels to a desired length, wherein the step of cutting may be performed before the mold is removed.

In another embodiment of any of the above method or methods, the method may further comprise a step of sterilizing the microchannels.

In still another embodiment of any of the above method or methods, the step of sterilizing may comprise exposing the microchannels to ethanol, or a mixture of ethanol and water.

In yet another embodiment of any of the above method or methods, the microchannels may comprise xylem and/or phloem channels.

In still another embodiment of any of the above method or methods, the decellularised microchannels may be cellulose-based, chitin-based, lignin-based, lingo-cellulosic polymer-based, hemicellulose-based, or pectin-based, or any combination thereof.

In another embodiment of any of the above method or methods, a density of decellularised microchannels within the bundle may be greater than a density of microchannels within the plant or fungal tissue.

In another embodiment of any of the above method or methods, the plant or fungal tissue may comrpise a microchannel-containing tissue from apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, a rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue, or a genetically altered tissue produced via direct genome modification or through selective breeding, or any combinations thereof.

In still another embodiment of any of the above method or methods,, the plant or fungal tissue may comprise celery, asparagus, or both.

In another embodiment of any of the above method or methods, the step of decellularising the microchannels may comprise decellularising by thermal shock, treatment with detergent, osmotic shock, lyophilisation, physical lysing, electrical disruption, or enzymatic digestion, or any combination thereof, thereby removing cellular materials and nucleic acids to provide decellularised microchannels.

In another embodiment of any of the above method or methods, the step of decellularising may comprise treatment with sodium dodecyl sulphate (SDS).

In yet another embodiment of any of the above method or methods, residual SDS may be removed by using an aqueous divalent salt solution to precipitate a salt residue containing SDS micelles out of the microchannels.

In another embodiment of any of the above method or methods, dH₂O, DI water, acetic acid, DMSO, or sonication treatment, or any combination thereof, may be used to remove the aqueous divalent salt solution, the salt residue, and/or the SDS micelles.

In another embodiment of any of the above method or methods, the divalent salt of the aqueous divalent salt solution may comprise MgCl₂ or CaCl₂.

In another embodiment of any of the above method or methods, the step of decellularising may comprise treatment with an SDS solution of about 0.1% or about 1% SDS in water, and the residual SDS may be removed following decellularisation using an aqueous CaCl₂ solution at a concentration of about 100mM, followed by incubation in dH₂O or DI water.

In yet another embodiment of any of the above method or methods, the method may further comprise a step of introducing living plant or animal cells to the microchannels.

In another embodiment of any of the above method or methods, the living cells may be mammalian cells.

In another embodiment of any of the above method or methods, the living cells may be human cells.

In another embodiment, there is provided herein a decellularised microchannel produced by the any of the method or methods described herein.

In another embodiment, there is provided herein a scaffold biomaterial produced by any of the method or methods described herein.

In another embodiment of the above scaffold biomaterial, the scaffold biomaterial may be or comprise a scaffold biomaterial as described herein.

In another embodiment, there is provided herein a kit comprising any one, two, three, or more of:
a mold;
a glue;
one or more microchannels;
one or more decellularisation agents;
a scalpel or microtome;
sterile measuring device;
sterile saline;
tweezers; and/or
instructions for performing any of the method or methods as described herein.

### BRIEF DESCRIPTION OF DRAWINGS

These and other features will become better understood with regard to the following description and accompanying drawings, wherein:
FIGURE 1 shows (Left): Isolated asparagus (AS) vascular bundles, approx. 3 cm length. (Right): Microscopic cross-sectional view of an AS vascular bundle, with outlines of xylem channels clearly visible against the background;
FIGURE 2 shows (Left): Isolated celery (CL) vascular bundles, variable length 5 - 10 cm. (Right): Microscopic cross-sectional view of a CL vascular bundle, with outlines of xylem channels clearly visible against the ground tissue background;
FIGURE 3 shows (Left): Biomedical-grade silicone mold (5-mm internal diameter, 1-cm length) for vascular bundle packing. Mold halves are pressed together. (Right): 2 mm-thick slice of mold containing glued CL vascular bundles;
FIGURE 4 shows AS channel bundles during the glue curing step; mold halves are pressed together;
FIGURE 5 shows CL channel bundles during the glue curing step; mold halves are pressed together;
FIGURE 6 shows (Left): HDMC (AS) bundles stored in DPBS. (Right): Individual HDMC (AS) immediately after removal from silicone mold;
FIGURE 7 shows (Left): HDMC (CL) bundles stored in DPBS. (Right): Individual HDMC (CL) immediately after removal from silicone mold;
FIGURE 8 shows Confocal Laser-Scanning Micrographs of HDMC (AS-based) sample after 1 week of GFP-3T3 cell culture followed by fixing in 4% paraformaldehyde. Cells (green-green fluorescent protein) visible in and around xylem microchannels (blue-autofluorescence). (Left): cross-sectional view of single CL vascular channel within a glued bundle, with cells visible within xylem channels. Note cell density surrounding openings of xylem and phloem channels. (Right): cross-sectional (partial) view of 2 vascular channels within a glued bundle, cells scattered throughout;
FIGURE 9 shows Confocal Laser-Scanning Micrographs of HDMC (CL-based) sample after 1 week of GFP-3T3 cell culture followed by fixing in 4% paraformaldehyde. Cells (green-green fluorescent protein) visible in and around xylem microchannels (blue-autofluorescence). (Left): cross-sectional view of single CL vascular channel within a glued bundle, with cells visible within xylem channels. Note the cell adherence to a fibrin glue deposit located to the right of the vascular channel. (Right): cross-sectional view of 2 vascular channels within a glued bundle, cells scattered throughout;
FIGURE 10 shows a quartered AS section with visible vascular bundles indicated by red arrows;
FIGURE 11 shows isolated AS vascular bundles in a DPBS-containing weighing boat;
FIGURE 12 shows isolated CL vascular bundles in a DPBS-containing weighing boat;
FIGURE 13 shows AS HDMC bundles during glue setting period using PEG-based glue;
FIGURE 14 shows AS HDMCs in DPBS storage solution, glued with PEG-based glue. VBs were loosely bonded and the HDMCs and did not retain a cylindrical shape in the mold-type experimental conditions used;
FIGURE 15 shows CL HDMC bundles during glue setting period, where volume expansion of the PEG-based Coseal glue caused silicone mold halves to separate;
FIGURE 16 shows CL HDMC bundles glue with PEG-based Coseal in DPBS storage solution, with partial debonding of individual VBs visible;
FIGURE 17 shows a side view of AS strip bundle glued with cyanoacrylate-based glue;
FIGURE 18 shows a radial view of glued (cyanoacrylate glue) AS strip bundle, with individual channels faintly visible;
FIGURE 19 shows AS channels in joined silicone mold haves during curing period using biodegradable fibrin sealant (TISSEEL);
FIGURE 20 shows AS channels within silicone molds during glue curing step using fibrin glue;
FIGURE 21 shows a view of fibrin-glued AS channels after removal from silicone molds;
FIGURE 22 shows a perspective view of AS sample after 1 week of GFP-3T3 cell culture followed by fixing in 4% paraformaldehyde (left panel) and a top view of the same sample (right panel);
FIGURE 23 shows CLSM imagery of AS HDMC bundle sample after 1 week of GFP-3T3 cell culture followed by fixing in 4% paraformaldehyde. Cells (green) are visible in and around xylem microchannels (blue);
FIGURE 24 shows a view of AS channels within food-grade silicone straw molds during glue (fibrin) curing step;
FIGURE 25 shows a view of glued (fibrin) AS channel samples stored in sterile DPBS after cutting to 5 mm length;
FIGURE 26 shows a side view of CL channel bundle glued with cyanoacrylate glue;
FIGURE 27 shows a radial view of CL channel bundle glued with cyanoacrylate glue, in which individual channels are faintly visible;
FIGURE 28 shows CL channels in joined silicone mold halves during curing period with fibrin sealant;
FIGURE 29 shows a view of CL channels within silicone molds during glue curing step with Fibrin sealant;
FIGURE 30 shows a view of fibrin-glued CL channel bundle after removal from silicone mold;
FIGURE 31 shows a perspective view of CL bundle sample after 1 week of GFP-3T3 cell culture followed by fixing in 4% paraformaldehyde (left panel) and a top view of the same sample (right panel);
Figure 32 shows CLSM imagery of CL bundle sample after 1 week of GFP-3T3 cell culture followed by fixing in 4% paraformaldehyde. Cells (green) are visible in and around xylem microchannels (blue);
FIGURE 33 shows a view of CL channels within food-grade silicone molds during glue (fibrin) curing step;
FIGURE 34 shows a view of glued CL channels after removal of top silicone mold half;
FIGURE 35 shows a view of glued CL channel samples, stored in sterile Dulbecco's Phosphate Buffered Saline (DPBS), after cutting to 5 mm length;

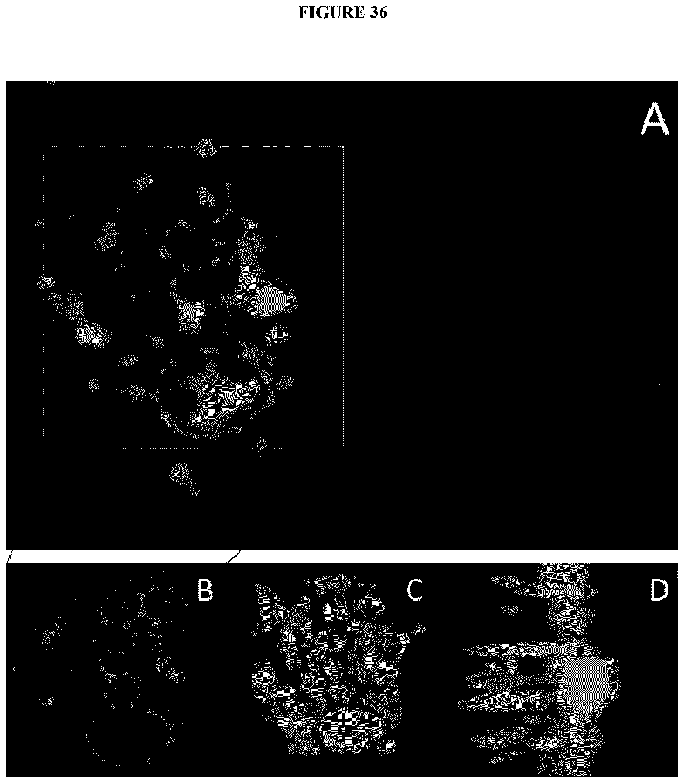

FIGURE 36 shows A) Confocal laser-scanning fluorescence micrograph (Z-stack projection) of xylem channels and fibroblast cells within HDMC/AS. Channels are visualized in red (autofluorescence; false color), GFP-3T3 fibroblasts are visualized in green (green fluorescent protein; false color). B) Close-up view of red-outlined multi-channel in panel A. C) 3D-volume rendering (Fiji/ImageJ; 3D Viewer Plugin) of green fluorescent protein and xylem autofluorescence signals from the perspective of panel B. D) Rotated view of 3D rendering, displaying directionality of cells along the length of the channels;

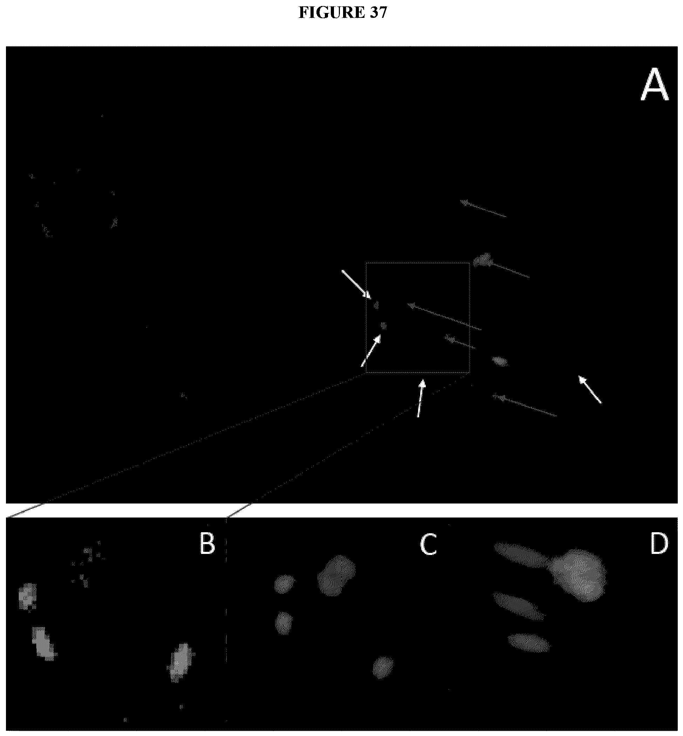

FIGURE 37 shows A) Confocal laser-scanning fluorescence micrograph (Z-stack projection) of xylem channels and fibroblast cells within HDMC/CL. Channels are visualized in red (autofluorescence; false color), GFP-3T3 fibroblasts are visualized in green (green fluorescent protein; false color). White arrows indicate the presence of cells outside of the channels. Blue arrows indicate the presence of cells inside the channels. B) Close-up view of red-outlined single channel in panel A. C) 3D-volume rendering (Fiji/ImageJ; 3D Viewer Plugin) of green fluorescent protein signal from the perspective of panel B. D) Rotated view of 3D rendering, displaying directionality of cells along the length of the channel;
FIGURE 38 shows histological staining of celery-derived HDMC bundle from subcutaneous injection studies of Example 8. Longitudinal cut. Hematoxylin and Eosin (A, B);
FIGURE 39 shows histological staining of celery-derived HDMC bundle from subcutaneous injection studies of Example 8. Transverse cut. Hematoxylin and Eosin (A, B);
FIGURE 40 shows histological staining of asparagus-derived HDMC bundle from subcutaneous injection studies of Example 8. Longitudinal cut. Hematoxylin and Eosin (A, B);
FIGURE 41 shows histological staining of asparagus-derived HDMC bundle from subcutaneous injection studies of Example 8. Transverse cut. Hematoxylin and Eosin (A, B);
FIGURE 42 is a graph showing percentage of bundles that separated into individual VBs after treatments as a function of varying ratios of acetic acid : 30% hydrogen peroxide and boiling times;
FIGURE 43 shows decellularised celery boiled for 20 minutes at varying concentrations of acid and peroxide solution, then laid out on a clear plastic plate. Ratios of glacial acetic acid to 30% hydrogen peroxide are depicted (FIG. 43A - 3:1; FIG. 43B - 2:1; FIG. 43C - 1:1; FIG. 43D - 1:2; FIG. 43E - 1:3).
FIGURE 44 shows asparagus strips treated with a 1:1 acid and peroxide solution of glacial acetic acid : 30% hydrogen peroxide. Fig. 44A shows freshly cut asparagus in slices in acid/peroxide solution. Fig. 44B shows resulting extracted bundles removed from acid/peroxide solution and washed in distilled water. Fig. 44C shows a small subset of bundles placed in a subsequent water wash.
FIGURE 45 is a graph showing percentage of LiCl-treated bundles that separated into individual VBs as a function of salt concentration and boiling times;
FIGURE 46 is a graph showing percentage of NaCl-treated bundles that separated into individual VBs as a function of salt concentration and boiling times;
FIGURE 47 is a graph showing percentage of NaOH-treated bundles that separated into individual VBs as a function of NaOH concentration and boiling times;
FIGURE 48 shows size distribution of CL vascular bundles derived from native tissue immersed in a boiling salt solution of LiCl for 15 minutes. Similar microstructure distributions were observed for the other treatments. Fig. 48A: the vascular bundles were stained with 0.1% Calcofluor White and the image was thresholded and segmented for size analysis. Fig. 48B: histogram of the vascular bundle diameters. The mean size was 23.7 ± 1.5 µm. Values are mean and standard error of the mean;
FIGURE 49 shows fluorescent microscopy images of preserved CL vascular bundle (VB) microstructure after boiling in 0.5 M LiCl for 10, 15, 20, and 30 minutes. The material was stained with Calcofluor White for 10 minutes and the images were converted to grey-scale;
FIGURE 50 shows fluorescent microscopy images of preserved CL vascular bundle (VB) microstructure after boiling in 1 M LiCl for 10, 15, 20, and 30 minutes. The material was stained with Calcofluor White for 10 minutes and the images were converted to grey-scale;
FIGURE 51 shows fluorescent microscopy images of preserved CL vascular bundle (VB) microstructure after boiling in 2 M LiCl for 10, 15, 20, and 30 minutes. The material was stained with Calcofluor White for 10 minutes and the images were converted to grey-scale;
FIGURE 52 shows fluorescent microscopy images of preserved CL vascular bundle (VB) microstructure after boiling in 3 M LiCl for 10, 15, 20, and 30 minutes. The material was stained with Calcofluor White for 10 minutes and the images were converted to grey-scale;
FIGURE 53 shows fluorescent microscopy images of preserved CL vascular bundle (VB) microstructure after boiling in 0.5 M NaCl for 10, 15, 20, and 30 minutes. The material was stained with Calcofluor White for 10 minutes and the images were converted to grey-scale;
FIGURE 54 shows fluorescent microscopy images of preserved CL vascular bundle (VB) microstructure after boiling in 1 M NaCl for 10, 15, 20, and 30 minutes. The material was stained with Calcofluor White for 10 minutes and the images were converted to grey-scale;
FIGURE 55 shows fluorescent microscopy images of preserved CL vascular bundle (VB) microstructure after boiling in 2 M NaCl for 10, 15, 20, and 30 minutes. The material was stained with Calcofluor White for 10 minutes and the images were converted to grey-scale;
FIGURE 56 shows fluorescent microscopy images of preserved CL vascular bundle (VB) microstructure after boiling in 3 M NaCl for 10, 15, 20, and 30 minutes;
FIGURE 57 shows fluorescent microscopy images of preserved CL vascular bundle (VB) microstructure after boiling in 0.5 M NaOH for 10, 15, 20, and 30 minutes. The material was stained with Calcofluor White for 10 minutes and the images were converted to grey-scale;
FIGURE 58 shows fluorescent microscopy images of preserved CL vascular bundle (VB) microstructure after boiling in 1 M NaOH for 10, 15, 20, and 30 minutes. The material was stained with Calcofluor White for 10 minutes and the images were converted to grey-scale;
FIGURE 59 shows fluorescent microscopy images of preserved CL vascular bundle (VB) microstructure after boiling in 2 M NaOH for 10, 15, 20, and 30 minutes. The material was stained with Calcofluor White for 10 minutes and the images were converted to grey-scale;
FIGURE 60 shows fluorescent microscopy images of preserved CL vascular bundle (VB) microstructure after boiling in 3 M NaOH for 10, 15, 20, and 30 minutes. The material was stained with Calcofluor White for 10 minutes and the images were converted to grey-scale;
FIGURE 61 shows fluorescent microscopy images of preserved CL vascular bundle (VB) microstructure after boiling in a 1:1 (v/v) ratio of peroxide and acetic acid for 10, 15, and 20 minutes. The material was stained with Calcofluor White for 10 minutes and the images were converted to grey-scale;
FIGURE 62 shows fluorescent microscopy images of preserved CL vascular bundle (VB) microstructure after boiling in a 1:2 (v/v) ratio of peroxide and acetic acid for 10, 15, and 20 minutes. The material was stained with Calcofluor White for 10 minutes and the images were converted to grey-scale;
FIGURE 63 shows fluorescent microscopy images of preserved CL vascular bundle (VB) microstructure after boiling in a 2:1 (v/v) ratio of peroxide and acetic acid for 10, 15, and 20 minutes. The material was stained with Calcofluor White for 10 minutes and the images were converted to grey-scale;
FIGURE 64 shows HDMC assembled from isolated CL vascular bundles boiled in water for 30 minutes (control conditions);
FIGURE 65 shows HDMC assembled from NaOH treated CL vascular bundles boiled for 30 minutes;
FIGURE 66 shows macerated CL in diluted acetic acid and peroxide boiled for 15 minutes, as described in Example 10; and
FIGURE 67 shows macerated celery with only xylem and phloem strands visible.

### DETAILED DESCRIPTION

There has previously been a lack of materials (biomaterials) that can mimic the directional, channel-like microscopic structures of certain tissues in humans, plants, and animals. Examples of such directional tissues include the extracellular matrices (ECM) of the spinal cord, vascular channels, lymphatic tissue, nervous tissue, and many others. Biocompatible materials possessing directionality and/or channel-like structure are desirable in the field.

Decellularised plant tissue may be used to provide scaffold biomaterials. The plant tissue may include microchannels, such as xylem and phloem channels, which may impart some directionality to the scaffold biomaterials. However, in decellularised plant tissue, the number of functional structural elements (microchannels) may be limited by their natural density within the plant tissue.

Material performance, in terms of functional recovery following an SCI for example, may be limited by the number of available microchannels for neuronal growth in certain applications. The present inventors have now developed methods for densification of these channels to provide high-density microchannel (HDMC) bundles, which may be used in a wide variety of applications including treatment and/or repair of spinal cord injury (SCI) and many others.

As described herein, in certain embodiments microchannels (i.e. xylem and phloem) present in vascular bundles of plant tissue may provide a guiding scaffold for the growth and/or regeneration of cells (such as neurons). Such decellularised plant structures may provide a more suitable surface (in terms of adherence, biocompatibility, etc.) for cell growth as compared with certain artificial or synthetic substrates. By extracting, isolating, and repackaging these channels, it is shown herein that these guiding structural components may be densified and may provide more routes for proliferating cells. It is contemplated that in certain embodiments densified bundles of microchannels may provide superior performance in certain applications compared to decellularised plant material without further modifications, stemming from increased densification of the microchannels. In certain examples where densification is beneficial, it is contemplated that decellularised material without modification may be less effective as a scaffold material per unit volume compared to densified vascular tissue.

In certain embodiments, the biomaterials described herein may be derived from cell wall architectures and/or vascular structures found in the plant and fungus kingdoms to create 3D scaffolds which may promote and/or directionally guide cell infiltration, cell growth, angiogenesis, tissue repair, and/or tissue reconstruction, etc. As will be understood, biomaterials as described herein may be produced from any suitable part of plant or fungal organisms having vascular structures or microchannels (such as, for example, xylem and/or phloem), including, for example, seed, root, bark, leaf, stem, fruit, pulp, core. Biomaterials may comprise, for example, substances such as cellulose, chitin, lignin, lignocellulosic polymers, hemicellulose, pectin, and/or any other suitable biochemicals/biopolymers which are naturally found in these organisms.

In certain embodiments, unlike many commercial biomaterials, plant/fungus derived biomaterials as described herein may be substantially non-resorbable or poorly resorbable (i.e. they will not substantially breakdown and be absorbed by the body), although in embodiments where glue is used the glue may be biodegradable in certain embodiments. The non-resorbable characteristic of these scaffolds may offer certain benefits. For example, in certain embodiments, biomaterials described herein may be resistant to shape change, and/or may hold their intended geometry over long periods of time. In certain embodiments, since they may have a minimal footprint compared to certain other products, they may be considered effectively invisible to the body, eliciting almost no immune response. When resorbable biomaterials break down, their by-products often illicit an adverse immune response, as well as induce oxidative stress and result in an increase of pH in the recovering tissue, which may be avoided by using a non-resorbable biomaterial.

As will be understood, unless otherwise indicated, the meaning/definition of plant and fungi kingdoms used herein is based on the Cavalier-Smith classification (1998).

### Scaffold Biomaterials

Described herein are scaffold biomaterials, methods and uses thereof, as well as methods for the production thereof. It will be appreciated that embodiments and examples are provided for illustrative purposes intended for those skilled in the art, and are not meant to be limiting in any way.

In an embodiment, there is provided herein a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle.

As will be understood, the scaffold biomaterial may in certain embodiments be a material having a 3-dimensional structure (although any of the x, y, and z dimensions may be very thin in certain embodiments), in which arrangement of the decellularised channels provides a directional porosity determined by the size, density, and orientation of the microchannels. In certain embodiments, the scaffold biomaterial may be suitable for providing a scaffold upon or through which living cells may infiltrate and/or grow and/or proliferate.

In certain embodiments, the microchannels may comprise generally any suitable vascular or other channel-type structure derived or isolated from plant or fungal tissue. In certain embodiments, the microchannels may comprise, for example, xylem and/or phloem channels. In certain embodiments, the microchannels may be decellularised, such that cellular materials and nucleic acids of the plant or fungal tissue are removed from the microchannels. In certain embodiments, the microchannels may be individually isolated from the plant or fungal tissue, may be grouped into one or more vascular bundles isolated from plant or fungal tissue, or any combination thereof. In certain embodiments, the microchannels may be cellulose-based, chitin-based, lignin-based, hemicellulose-based, lignocellulosic polymer-based, or pectin-based, or any combination thereof.

In certain embodiments, the microchannels may be about 50 to about 150 microns in diameter, or any sub-range or value occurring therebetween, with a continuous length based on the size of the biomaterial source or determined by cutting of the microchannels to one or more desired lengths. In certain embodiments, a mix of microchannels of different diameters and/or lengths may be used, or microchannels may be sorted/selected such that microchannels having a similar diameter and/or length are used.

As will be understood, cellular materials and nucleic acids of the plant or fungal tissue may include intracellular contents such as cellular organelles (e.g. chloroplasts, mitochondria), cellular nuclei, cellular nucleic acids, and/or cellular proteins. These may be substantially removed, partially removed, or fully removed from the plant or fungal tissue, from the microchannels, and/or from the scaffold biomaterial. It will be recognized that trace amounts of such components may still be present in the decellularised plant or fungal tissues described herein. As will also be understood, references to decellularised microchannels herein are intended to reflect that such cellular materials found in the plant or fungal source of the microchannels have been substantially removed from the microchannels - this does not preclude the possibility that the decellularised microchannels may in certain embodiments contain or comprise subsequently introduced, or reintroduced, cells, cellular materials, and/or nucleic acids of generally any kind, such as animal or human cells.

Indeed, in certain embodiments, the decellularised microchannels and/or scaffold biomaterials as described herein may further comprise living cells on and/or within at least one of the decellularised microchannels. In certain embodiments, the living cells may be animal cells, mammalian cells, or human cells. In certain embodiments, the living cells may comprise plant and/or fungi cells.

In certain embodiments, it is contemplated that plant and/or fungi cells may be added to microchannels to further modify the channels, after which decellularisation may be performed prior to implantation, for example. By way of example, in certain embodiments, decellularisation processes may denature and/or remove native cell wall proteins from the scaffold, and it is contemplated that selected and/or engineered moieties such as polysaccharide proteins (for example, polysaccharides-conjugated to mammalian surface anchorage and ligand-binding domains) may be added to the walls of the decellularised scaffold. In certain embodiments, anchorage protein(s) may be added. In certain embodiments, cells that may be added/deposited onto the decellularised scaffold may include those which may produce a useful or functional protein or macromolecule of interest into the microchannel structure. In certain embodiments, proteins with mammalian surface anchorage points may be added to the scaffold to speed up integration into surrounding tissue, for example. In certain embodiments, cells may be genetically modified to produce such moieties or proteins, and the cells may be added to the scaffold for a period of time and then removed, washed out, or killed. Examples of proteins or moieties that may be added may include mammalian growth factors for particular cell type(s) (for example, to encourage neuron proliferation).

In certain embodiments, a bundle of microchannels may comprise any suitable grouping or cluster of decellularised microchannels. In the examples described hereinbelow, bundles were formed using gluing. However, it will be understood that in certain embodiments bundles may instead be formed using a variety of other glue-free techniques such as, but not limited to, techniques using sealants, mechanical packing or entanglement, or chemical crosslinking of neighbouring isolated vascular structures, for example. In certain embodiments, bundles may be formed by suspending microchannels in cellulose-based hydrogels, or other such hydrogels. In certain embodiments, individual microchannels and/or vascular bundles may be combined together by threading them around each other, for example. In certain embodiments, it is contemplated that compression/packing of isolated (optionally, decellularised) vascular structures into larger enveloping and/or fastening vascular structures (optionally, decellularised) may be performed.

In certain embodiments of any of the above methods, the step of bundling may include physically binding or physically cross-linking the microchannels and/or VBs without the use of glue. Such a process may be referred to as entanglement of the microchannels. The microchannels may be physically tied or otherwise wrapped such that separation of the microchannels and/or VBs requires the use of force. Different dimensions and using different lengths (Kuhn segments or persistence length) may make different entanglements and alignment profiles. Entanglement may occur from stirring with a stirring device, such as a rod. Entanglement may occur during liquid-based extraction.

As described in detail herein, in certain embodiments isolated decellularised plant or fungal vascular structures (i.e. xylem, phloem, and/or vascular bundles thereof) may be bundled (and, optionally, glued) together, providing channel densification beyond what is found in the plant or fungal source material. In certain embodiments, these bundles may designed to be biocompatible, and may be amenable to further functionalization. It is contemplated that in certain embodiments such high-density microchannel (HDMC) bundles may be used in a wide variety of applications which may include, but are not limited to, use as extracellular matrices, cell scaffolds, and specialized structural supports.

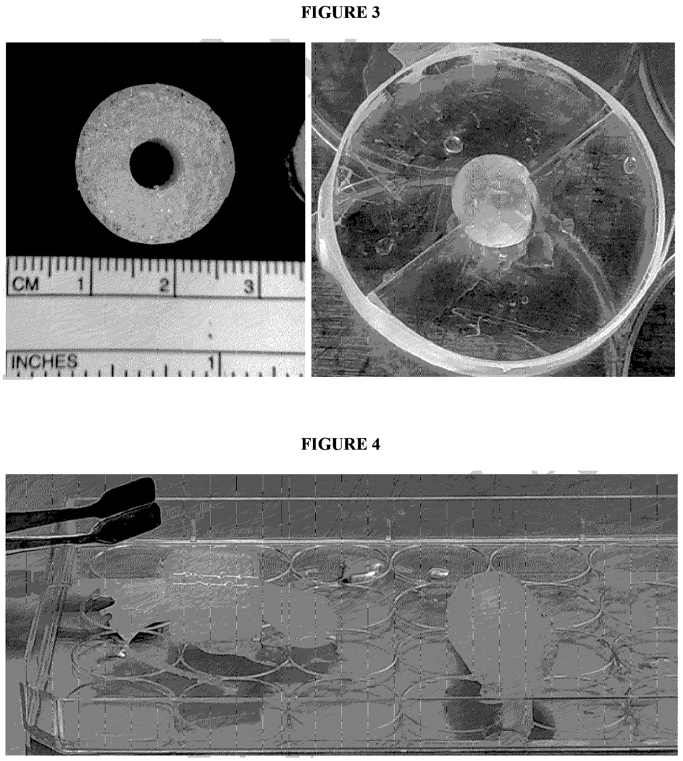

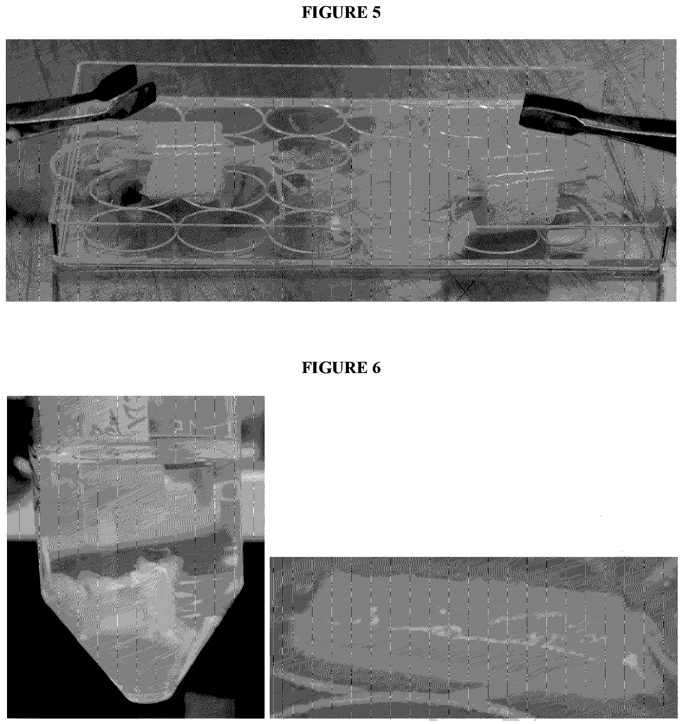

As will be understood, in certain embodiments the decellularised microchannels of the bundle may be arranged substantially parallel to each other within the bundle, so as to provide a substantially common longitudinal directionality or alignment of the channels of the bundle. Figures 4 and 5, described in further detail below, depict examples of bundles having decellularised microchannels arranged substantially parallel to each other (i.e. all microchannels in the bundle to the left of Figure 4 are arranged substantially parallel to each other, oriented with substantially the same directionality (left-right); and all microchannels in the bundle to the right of Figure 4 are arranged substantially parallel to each other, oriented with substantially the same directionality (front-back). in cases where the decellularised microchannels are bundled via entanglement, substantially parallel may refer to a common directionality or alignment of the bundle.

In certain embodiments, the plurality of decellularised microchannels of the bundle of the scaffold biomaterial may be glued together within the bundle. In certain embodiments, the plurality of decellularised microchannels may be glued together within the bundle by a biocompatible, optionally biodegradable, glue. Typically, it is desirable for the glue to be biodegradable such that it degrades over a period of time following implantation of the scaffold biomaterial, however embodiments where the glue is not biodegradable are also contemplated herein. In certain embodiments, the glue may be a glue having good adherence under moist conditions. In certain embodiments, the biocompatible glue may comprise a PEG-based, polyurethane-based, gelatin-based, or a fibrin-based glue. In certain embodiments, the biocompatible glue may comprise a fibrin-based glue.

In the examples described below, fibrin glue was shown to be particularly effective for gluing to produce HDMC bundles and scaffold biomaterials. However, it will be understood that a variety of other glues and/or sealants are also contemplated. For example, in certain embodiments, it is contemplated that fibrin glue may be substituted for other glue/sealant types, such as synthetic glues. Such glues may be advantageous in certain examples, due for example to less pronounced immune response following implantation.

PEG-based glues with additives may be used to allow for their biodegradation, however the present inventors have found that expansion of PEG-based glues makes these glues less preferable as compared with fibrin glue. In general, in certain embodiments a wide variety of suitable glue systems may be used in the fabrication of HDMC bundles, however it is noted that biodegradability of the glue is typically desired, although not necessary. In certain embodiments, it is contemplated that a PEG, polyurethane, or gelatin-based glue or sealant may be used, for example. In certain embodiments, the glue may be selected to be both biocompatible, and degradable following implantation, for example.

In certain embodiments, the glue may be a fibrin glue, or a cyanoacrylate glue. In certain embodiments, the glue may comprise a fibrin sealant. In certain embodiments, the glue may comprise TISSEEL from Baxter, Evicel from Ethicon, Vitagel from Stryker. In certain embodiments, the glue may comprise a PEG sealant. In certain embodiments, the glue may comprise Coseal from Baxter, or Duraseal from Covidien. Examples of glues are also described in Vyas et al., Comparison of Hemostatic Agents used in Vascular Surgery, Expert Opin Biol Ther., 2013, 13(12): 1663-1672, herein incorporated by reference in its entirety.

In embodiments where gluing, and especially molding, of the HDMC bundle(s) is desired, the glue may be selected so as to have low adherence to the mold structure itself, so as to have low expansion properties so as to prevent crushing the microchannels when the glue sets/cures, or both. In certain embodiments, it is contemplated that a non-expanding glue, or a glue having low expansion, may be used. In certain embodiments, a glue may be selected having a volume expansion of less than about 20%, less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2%, less than about 1%, or substantially no (i.e. 0%) expansion by volume, or any range bounded at an upper end of any of these values, or any range falling between any two of these values, for example.

In certain embodiments where gluing, a contracting glue (or a glue which contracts when drying) may be used. In such embodiments, the glue may typically be selected so as to provide little or minimal contraction, so as to avoid damaging bundles.

In certain embodiments, the glue may be biocompatible and/or bioinert. In certain embodiments, the glue may: adhere well to lignocellulose; not adhere strongly to non-lignocellulosic materials; may be substantially non-immunogenic; may allow for sterile preparation; may not involve extensive preparation and/or processing steps prior to use; may set relatively quickly (for example, within about 10 minutes); may have low or no endotoxin load; may not permeate lignocellulose such that it does not block microchannels; may not degrade lignocellulose, modify its surface chemistry, and/or impact or degrade its material properties; may not degrade, or may degrade very slowly, in storage conditions (such as 4°C, phosphate-buffered saline); may not be subject to autocatalytic chemical changes over time which would significantly alter its properties; or any combinations thereof.

In certain embodiments, a glue may be selected which is substantially biocompatible and substantially non-toxic. In certain embodiments, it may be desirable for the glue to be substantially bioinert, causing little or no immune response. In certain embodiments, it may be desirable for the glue to be chosen such that the glue is resorbable following cell invasion and/or implantation. In embodiments where molding is desired and a silicone-based mold is to be used, it may be desirable for the glue to be substantially non-stick toward silicone. In embodiments where therapeutic and/or *in vivo* applications are intended, it may be desirable to use a glue approved by a regulatory body such as the FDA. A variety of surgical glues, for example, are commercially available. A review of surgical glues/sealants may be found in Yepremyan et al., Surgical Glue: A Brief Overview, Austin Journal of Biomedical Engineering, 2014, 1(4): 1020, and Annabi, et al., Elastic Sealants for Surgical Applications, European Journal of Pharmaceutics and Biopharmaceutics, 2015, http://dx.doi.org/10.1016/i.eipb.2015.05.022, which are herein incorporated by reference in their entireties. In certain embodiments, it may be desirable to select a synthetic glue rather than a natural-origin glue, since bioinertness is more common among synthetic glues. While fibrin-based glue was shown to be particularly effective in the Examples below, in embodiments where immune response sensitivity is particularly high it may be desirable to use a synthetic glue or other bioinert glue to avoid even small immune response near the implantation site, for example. Fibrin based glues and synthetic adhesives have been investigated in, for example, Srinivasan et al., Novel Synthetic Adhesive as an Effective Alternative to Fibrin Based Adhesives, World Journal of Hepatology, 2017, 9(24): 1030-1039, which is herein incorporated by reference in its entirety.

Examples of natural origin glues that are contemplated may include:
- Natural polymers (such as polysaccharides, polypeptides, and others). Various types are known, including: Collagen/gelatin-based; Chitosan-based; Dextran-based; and Alginate-based glues.

Gelatin type glues are known for their biocompatibility and degradability; an example of a gelatin-type glue which is approved for sale in Europe and has FDA fast-track status is LifeSeal^{™}, available from Life-Bond (www.life-bond.com/index.php/lifesealtm/).

Fibrin-type glues, also known as fibrin sealants, are commonly used, and FDA-approved glues exist. There are many different types of fibrin glues, including glues based on bovine, porcine, and/or human thrombin, for example.

Biomimetic glues are also contemplated. These types of glues, typically inspired by geckos, caddisflies, various worms, and others, are mostly at the research stage but are known in the field.

Examples of synthetic glues that are contemplated may include:
- PEG-based glues: PEG-based glues are considered synthetic, and generally biocompatible. Coseal (Baxter Inc) is a widely available example of a PEG-based glue. Glue volume expansion and/or adherence to silicone molds (where used) was encountered by the present inventors in initial testing with Coseal (not shown), but with proper adjustments it is contemplated that impact of expansion and sticking may be minimized.
- Polyurethane glues: Polyurethane glues are considered synthetic, and generally biocompatible. These types are glues are being actively researched. Examples may include VIVO (Adhesys Medical GmbH) (based on polyurethane) and/or TissuGlu (Cohera Medical Inc) (based on polyurethane, FDA-approved).
- Cyanoacrylates: cyanoacrylates are considered synthetic, although may be associated with toxic degradation products and therefore may be somewhat limited in applicability. It is contemplated that such glues may still be of use where toxicity may be avoidable, such as in topical use, for example.
- Other glues: Examples of other synthetic glues being contemplated may include MeTro (Elastagen Ltd) - based on recombinant tropoelastin, and/or UV-curable adhesives, for example.

In certain embodiments, it is contemplated that gluing may be reduced or avoided entirely by chemically crosslinking (such as simple cellulose crosslinking) of the microchannels to one another; by wrapping the microchannels within an exterior enclosure, wrapping, or membrane; by sewing or tying the microchannels together; or any combinations thereof, for example. In certain embodiments, decellularised microchannels may be interwoven or meshed together, which may in certain embodiments improve performance under tension. In certain embodiments, channels may be flexible so as to allow such weaving or meshing without being significantly damaged. In certain embodiments, weaving may be performed during the molding phase.

In certain embodiments, it is contemplated that use of glues and/or sealants may be reduced or eliminated by shaping methods, allowing for individual pieces to fit together tightly, for example.

In certain embodiments of the scaffold biomaterials described herein, a density of decellularised microchannels within the bundle may be greater than a density of microchannels within the plant or fungal tissue source. In certain embodiments, density of decellularised microchannels may be based on a measurement of number of channels per cm², for example. In certain embodiments, this may be determined based on number of vascular bundles per cm², based on number of vascular structures per cm², or based on number of individual xylem and/or phloem channels per cm², for example. In certain embodiments, density may be measured in terms of number of microchannels in a circular cross-sectional area of a certain diameter. In certain preparations, samples have been measured at about 20 channels per 5mm diameter circular cross-section area, for example, and higher densities are also contemplated.

In certain embodiments, mimicking the axonal projections of the motor and/or sensory systems may be desirable. In certain embodiments, aligning the cortispinal tract, rubrospinal tract, raphespinal tract, reticulospinal tract, propriospinal tract, dpiniothalamic tract, and/or dorsal column sensory axons may be desired. It is contemplated that HDMCs as described herein may, in certain embodiments, be used to mimic 3D positions of injured axons within a subject. HDMCs may comprise multiple subunits/channels, rather than one fixed graft in certain embodiments. Additionally, each SCI injury is typically unique and rarely affects the entire spinal cord. As such, an ability to specifically place the HDMCs/channels in a 3D orientation that mimics the individual subject's specific injured axonal tract(s) may be of particular interest in certain embodiments.

In certain embodiments, the plant or fungal tissue may comprise generally any suitable plant or fungal tissue or part containing at least some microchannels and/or vascular structures (such as, but not limited to, xylem and/or phloem channels) which may be isolated from the surrounding plant or fungal tissue/structure.

In certain embodiments of the scaffold material or materials above, the plant or fungal tissue may comprise an apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue. Additional examples of plant and fungal tissues are described in Example 18 of WO2017/136950, entitled "Decellularised Cell Wall Structures from Plants and Fungus and Use Thereof as Scaffold Materials", herein incorporated by reference in its entirety.

In certain embodiments, the plant or fungal tissue may be genetically altered via direct genome modification or through selective breeding, to create an additional plant or fungal architecture which may be configured to physically mimic a tissue and/or to functionally promote a target tissue effect. The skilled person having regard to the teachings herein will be able to select a suitable scaffold biomaterial to suit a particular application.

In certain embodiments, a suitable tissue may be selected for a particular application based on, for example, physical characteristics such as size, structure (porous/tubular), stiffness, strength, hardness and/or ductility, which may be measured and matched to a particular application.

Moreover, chemical properties such as reactivity, coordination number, enthalpy of formation, heat of combustion, stability, toxicity, and/or types of bonds may also be considered for selection to suit a particular application. Such characteristics (physical and chemical) may also be directly modified before or after decellularisation and/or functionalization to respond to the specific application.

In certain embodiments, microchannels may be sourced from the same tissue or part of the plant or fungus, or from different parts or tissues of the plant or fungus. In certain embodiments, microchannels may be sourced from the same individual plant or fungus, or from multiple plants or fungi of the same species. In certain embodiments, the microchannels may be sourced from plants or fungi of different species, such that the bundles comprise vascular structures from more than one species. In certain embodiments, the microchannels may be selected so as to provide particular features. For example, in certain embodiments, microchannels having diameters falling within a certain size range may be selected for inclusion in the bundle, so as to impart particular structural characteristics to the scaffold biomaterial.

In certain embodiments, the plant or fungal tissue may comprise celery, asparagus, or both.

In certain embodiments, the scaffold biomaterial may be a scaffold biomaterial configured to physically mimic a tissue of the subject and/or to functionally promote a target tissue effect in the subject. Methods of using such scaffold biomaterials as are described herein may, in certain embodiments, include a step of selecting a scaffold biomaterial as described herein for which the decellularised microchannels are configured to physically mimic a tissue of the subject and/or to functionally promote a target tissue effect in the subject. The skilled person having regard to the teachings herein will be able to select a suitable scaffold biomaterial to suit a particular application.

Various methods may be used for producing scaffold biomaterials as described herein. By way of example, in certain embodiments of the scaffold biomaterials above, the decellularised microchannels may comprise microchannels which have been decellularised by thermal shock, treatment with detergent (e.g. SDS, Triton X, EDA, alkyline treatment, acid, ionic detergent, non-ionic detergents, and zwitterionic detergents), osmotic shock, lyophilisation, physical lysing (e.g. hydrostatic pressure, critical point decellularisation, CO₂ critical point decellularisation), electrical disruption (e.g. non thermal irreversible electroporation), or enzymatic digestion, or any combination thereof. In certain embodiments, biomaterials as described herein may be obtained from plants and/or fungi by employing decellularisation processes which may comprise any of several approaches (either individually or in combination) including, but not limited to, thermal shock (for example, rapid freeze thaw), chemical treatment (for example, detergents), osmotic shock (for example, distilled water), lyophilisation, physical lysing (for example, pressure treatment), electrical disruption and/or enzymatic digestion.

In certain embodiments, the microchannels will typically be isolated from the plant tissue first, and then decellularised. In such manner, decellularisation may be easier since the microchannels are typically similar in size and may be decellularised at a similar rate. However, it will be understood that in certain embodiments the plant tissue may be decellularised first, and then the microchannels isolated from the decellularised plant tissue. In such approaches, decellularisation conditions may be adjusted accordingly to suit the properties of the plant tissue being decellularised (for example, in certain embodiments larger samples of plant tissue may be subjected to longer or more aggressive decellularisation protocol as compared with smaller samples of plant tissue more readily accessible to decellularisation reagents).

In certain embodiments, the decellularised microchannels may comprise microchannels which have been decellularised by treatment with a detergent or surfactant. Examples of detergents may include, but are not limited to sodium dodecyl sulphate (SDS), Triton X, EDA, alkaline treatment, acid, ionic detergent, non-ionic detergents, and zwitterionic detergents.

In still further embodiments, the decellularised microchannels may comprise microchannels which have been decellularised by treatment with SDS. In still another embodiment, residual SDS may be removed from the microchannels by washing with an aqueous divalent salt solution. The aqueous divalent salt solution may be used to precipitate/crash a salt residue containing SDS micelles out of the solution/scaffold, and a dH₂O, acetic acid or dimethylsulfoxide (DMSO) treatment, or sonication, may have been used to remove the salt residue or SDS micelles.In certain embodiments, the divalent salt of the aqueous divalent salt solution may comprise, for example, MgCl₂ or CaCl₂.

In another embodiment, the microchannels may be decellularised by treatment with an SDS solution of between 0.01 to 10%, for example about 0.1% to about 1%, or, for example, about 0.1% SDS or about 1% SDS, in a solvent such as water, ethanol, or another suitable organic solvent, and the residual SDS may have been removed using an aqueous CaCl₂ solution at a concentration of about 100mM followed by incubation in dH₂O. In certain embodiments, the SDS solution may be at a higher concentration than 0.1%, which may facilitate decellularisation, and may be accompanied by increased washing to remove residual SDS. In particular embodiments, the microchannels may be decellularised by treatment with an SDS solution of about 0.1% SDS in water, and the residual SDS may have been removed using an aqueous CaCl₂ solution at a concentration of about 100mM followed by incubation in dH₂O.

Further examples of decellularisation protocols which may be adapted for producing decellularised microchannels for scaffold biomaterials as described herein may be found in WO2017/136950, entitled "Decellularised Cell Wall Structures from Plants and Fungus and Use Thereof as Scaffold Materials", herein incorporated by reference in its entirety.

In certain embodiments, the decellularised microchannels and/or scaffold biomaterials as described herein may further comprise living cells on and/or within at least one of the decellularised microchannels. In certain embodiments, the living cells may be animal cells, mammalian cells, or human cells. In certain embodiments, the cells may be cells introduced or seeded into and/or onto the scaffold biomaterials and/or decellularised microchannels, or may be cells infiltrating into or onto the scaffold biomaterials and/or decellularised microchannels following implantation of the scaffold biomaterials and/or decellularised microchannels into a living animal or plant subject, for example.

### Methods and Uses of Scaffold Biomaterials

The scaffold biomaterials as described herein may comprise one or more high-density microchannel bundles. While many of the design considerations, and contemplated uses, of the presently described scaffold biomaterials may be related to those described for the scaffold biomaterials of WO2017/136950, entitled "Decellularised Cell Wall Structures from Plants and Fungus and Use Thereof as Scaffold Materials" (herein incorporated by reference in its entirety), the presently described biomaterials and may provide benefit arising from increased density of channels and/or from the controllable and directionally aligned nature of the channels which may be substantially parallel to one another. Thus, the presently described biomaterials may be particularly advantageous for applications where channel directionality is desirable, for example.

In certain embodiments, biomaterials as described herein may have applications in biomedical laboratory research and/or clinical regenerative medicine in human and/or veterinary applications, for example. Such biomaterials may be effective as scaffolds which may be used as investigative tools for industrial/academic biomedical researchers, for biomedical implants, in sensing devices and pharmaceutical delivery vehicles, and/or in other suitable applications in which scaffolds may be used.

In certain embodiments, scaffold biomaterials as described herein may be used for regeneration of blood vasculature. The wide range of structures available may allow for the artificial production of blood vessel-like structures, and/or may provide conditions suitable for angiogenesis (natural blood vessel formation).

In certain embodiments, scaffold biomaterials as described herein may be used as simple or complex tissues. By way of example, scaffolds may be used to replace/regenerate simple (skin, bone) or complex (spinal cord, muscle, nerves, blood vessels, etc.) tissues following accident, malformation, esthetic, injury, or other damage to the tissue.

In certain embodiments, there are provided herein methods for supporting animal cell growth, for promoting tissue regeneration, for promoting angiogenesis, for replacement of a tissue, or for providing a structural scaffold in a cosmetic surgery, in a subject in need thereof, said methods comprising:
providing a scaffold biomaterial according to any of the scaffold biomaterials described herein; and
implanting the scaffold biomaterial into the subject.

In certain embodiments, high density microchannel (HDMC) approaches as described herein may provide directional pathways for cell infiltration. The "directionality" may arise not only from the interior of the microchannels, but also their exterior surfaces and the interchannel space. Figures 36 and 37 show evidence of cells proliferating along both the inside and the outside of the microchannels within an HDMC. HDMC may thus be of particular interest in tissue repair applications where a cellular connection or link is severed. A directional biomaterial may be particularly desirable in such instances, for example. It is contemplated that HDMC may be of particular interest for spinal cord injury (SCI) treatment, for example, since the neurons in the spinal cord are highly organized in tracts, and their proper functioning relies on directional connections. In certain embodiments, it is contemplated that HDMC may provide an end-to-end support structure that may provide for neuron reconnections along the proper tracts. Other structures that may be of particular interest for repair by HDMC as described herein may include vascular system channels. In certain embodiments, it is contemplated that HDMC may act as joints and/or fittings to repair particularly fine vascular systems.

In certain embodiments, bi-directional microchanels may provide nano- and/or micro-topogrpahical cues that may drive neurons and/or axonal guidance (see Straley KS, Foo CWP, Heilshorn SC (2010) Biomaterial design strategies for the treatment of spinal cord injuries. J Neurotrauma 27:1-19; Hoffman-Kim D, Mitchel J a, Bellamkonda R V (2010) Topography, cell response, and nerve regeneration. Annu Rev Biomed Eng 12:203-31; Hoffman-Kim D, Mitchel J a, Bellamkonda R V (2010) Topography, cell response, and nerve regeneration. Annu Rev Biomed Eng 12:203-31, which are herein incorporated by reference).

In certain embodiments, HDMC may allow individual channels to be aligned with the subject's own damaged spinal tract and/or may provide a better biomimic for the lost architecture of the damaged tissue. (see also Ige, O. O., Umoru, L. E., & Aribo, S. (2012). Natural Products: A Minefield of Biomaterials. ISRN Materials Science, 2012, 1-20. https://doi.org/10.5402/2012/983062, herein incorporated by reference). Rostral axons may migrate through scar tissue to bypass the lession and reconnect with a caudal axons. However, there is low chance that the exact axonal tract has reconnected with same rostral stump as the 3D cordinates of the lesion may be lost thru the random axonal migration thru the dense fibrin fibrils of the formed scar tissue. Whatever recovery may be gained from the axonal tract reconnection may be due to natural plasticity of the central nervous system that is established through long term physiotherapy (see also Anderson, M. A., O'Shea, T. M., Burda, J. E., Ao, Y., Barlatey, S. L., Bernstein, A. M., ... Sofroniew, M. V. (2018). Required growth facilitators propel axon regeneration across complete spinal cord injury. Nature, 561(7723), 396-400. https://doi.org/10.1038/s41586-018-0467-6, herein incorporated by reference). If the 3D coordinates across the damaged tissue were maintained, as may be provided by the HDMC approaches described herein, it is contemplated that in certain embodiments the ability for the exact axonal rostral and caudal stump tracts may be reestablished which may increase the motor recovery of the subject as well as decrease reliance of CNS plasticity during patient physiotherapy.

In certain embodiments, the scaffold biomaterial may be implanted at the spinal cord, and may promote repair or regeneration following spinal cord injury; may provide a structural implant for tissue replacement and/or for tissue regeneration in the subject; may provide a structural implant for skin graft and/or skin regeneration in the subject; may provide a structural implant for regeneration of blood vasculature in a target tissue or region or the subject; may provide a bone replacement, bone filling, or bone graft material, and/or may promote bone regeneration, in the subject; may provide a tissue replacement for skin, bone, muscle, nerve, blood vessel, or other damaged or malformed tissue in the subject; and/or may provide a structural implant for cosmetic surgery.

In certain embodiments, the scaffold biomaterial may be implanted at the spinal cord, and may promote repair and/or regeneration following acute and/or chronic spinal cord injury in the central and/or peripheral nervous system.

In an embodiment, there is provided herein a use of any of the scaffold biomaterials and/or microchannel bundles as described herein for supporting animal or plant cell growth; promoting tissue regeneration; promoting angiogenesis; treatment and/or repair of spinal cord injury; repair or reconstruction or replacement of plant or animal tissue; solution or material filtration and/or separation; plant modification or growth modulation; material transport; assembly for mimicking a desired shape or object; and/or microfluidics; or any suitable application in which biocompatible micrometer-scale channels may be of use.

In certain embodiments, the scaffold biomaterial may be for repair or reconstruction or replacement of plant or animal tissue, wherein the plant or animal tissue comprises damaged microvasculature, or plant tissue damaged by vascular disease (such as wilt disease), or damaged vascular structure of trees infested with an insect (such as emerald ash borer).

In certain embodiments, the scaffold biomaterial may be for repair or reconstruction of plant or animal tissue, wherein the plant or animal tissue comprises damaged microvasculature, wherein angiogenesis is impaired or compromised.

In another embodiment, the scaffold biomaterial may be for repair or reconstruction of animal tissue, wherein the animal is human.

In yet another embodiment, the scaffold biomaterial may be for plant modification or growth modulation, wherein the modification is modification of a grafting process for accelerated growth.

In still another embodiment, the scaffold biomaterial may be for material transport, wherein the material transport is a drug delivery application.

In yet another embodiment, the scaffold biomaterial may be for heat transfer or heat exchange microfluidics.

In still another embodiment, there is provided herein a method for supporting cell growth, said method comprising:
providing a scaffold biomaterial as described herein; and
introducing one or more cells to the scaffold biomaterial.

In still another embodiment, there is provided herein a method for promoting tissue regeneration; promoting angiogenesis; treatment and/or repair of spinal cord injury; or repair or reconstruction or replacement of plant or animal tissue, said method comprising:
providing a scaffold biomaterial as described herein; and
implanting said scaffold biomaterial at a site in need thereof.

In another embodiment, there is provided herein a method for solution or material filtration and/or separation, said method comprising:
providing a scaffold biomaterial as described herein; and
passing a solution or material through the scaffold biomaterial so as to filter and/or separate components from the solution based on size exclusion.

In yet another embodiment, there is provided herein a method for plant modification or growth modulation, said method comprising:
providing a scaffold biomaterial as described herein; and
grafting the scaffold biomaterial into the plant to provide for accelerated growth.

In certain embodiments, grafting may be done surgically, by identifying and replacing damaged or malfunctioning vascular structures within plants with the scaffold biomaterial. In certain embodiments, such repair may prevent loss of one or more distal parts of the plant. In certain embodiments, the scaffold biomaterial may be functionalized with one or more plant growth factors to promote or accelerate growth. In certain embodiments, grafts may incorporate genetically modified cells from the intended host, or another host. Such cells may accelerate growth, or confer resistance to certain plant diseases, for example. In certain embodiments, plant R-genes may be included, and grafts may be used to incorporate cells with engineered R-genes, for example.

In another embodiment, there is provided herein a method for material transport, said method comprising:
providing a scaffold biomaterial as described herein; and
using the scaffold biomaterial to transport a material to a site in need thereof.

In another embodiment, the material may be or comprise a drug, and the scaffold biomaterial may be used for drug delivery. In certain embodiments, the scaffold biomaterial may be loaded with the drug, and administered to the subject at a specific site. In certain embodiments, the scaffold biomaterial may provide for a controlled slow release of the drug over time, thus avoiding subjecting the subject to multiple injections, for example.

In certain embodiments, it is contemplated that scaffold biomaterials as described herein may be used for drug delivery to provide localization in drug delivery schemes. Vehicles, such as alginate beads, have been investigated as a means to encapsulate drugs and release drugs over time in a controlled fashion. However, this sort of delivery system may have issues with delivering the drugs to a specific location in need thereof, as the encapsulating material may drift. In certain embodiments, scaffold biomaterials as described herein may be designed to anchor drug encapsulation systems, remaining in-place such that the drug(s) may be delivered to the intended target(s). Removal of the scaffold biomaterials may be used in order to stop drug delivery if desired. In certain embodiments, scaffold biomaterials as described herein may be used to anchor drug encapsulators/vehicles, or may act as drug encapsulators/vehicles directly themselves, or both.

In another embodiment, there is provided herein a method for preparing a structure mimicking a desired shape or object, said method comprising:
providing a scaffold biomaterial as described herein;
carving or arranging the scaffold biomaterial so as to mimic the desired shape or object; and
optionally, gluing the scaffold biomaterial for structural reinforcement.

In yet another embodiment, there is provided herein a method for exchanging or transferring heat in a microfluidics process, said method comprising:
providing a scaffold biomaterial as described herein; and
using the microchannels to carry one or more fluids, wherein the one or more fluids are in close proximity so as to allow for heat exchange or heat transfer.

In certain embodiments, the one or more fluids may be carried by counter or parallel flow in different microchannels, or a combination thereof.

It is contemplated that in certain embodiments, HDMC bundles and/or scaffold biomaterials as described herein may be used in a variety of applications which may include, but are not limited to, any one or more of the following: spinal cord injury treatment; repair/reconstruction of human, plant, or animal tissues (for example, repair/reconstruction of damaged microvasculature, especially in cases where angiogenesis is not possible or compromised in any way, and/or in repair of plant tissue damaged by vascular disease such as wilt disease, and/or to replace damaged vascular structures in trees infested by insects such as emerald ash borer); solution/material filtration and/or separation; plant modification/growth modulation (for example, modification of the grafting process for accelerated growth); material transport (for example, drug delivery applications); assembled for mimicking a desired shape or object; and/or microfluidics (for example, heat transfer/exchange); or any other suitable application in which biocompatible micrometer-scale channels may be of use.

### Methods for Isolation and decellularisation of Microchannels, and Methods for Preparing Scaffold Biomaterials:

Methods for the isolation and decellularisation of microchannels, and methods for preparing scaffold biomaterials are described in detail herein. As well, experimental examples of such methods are described in detail in the Examples section below.

Further examples of decellularisation protocols which may be adapted for producing decellularised microchannels for scaffold biomaterials as described herein may be found in WO2017/136950, entitled "Decellularised Cell Wall Structures from Plants and Fungus and Use Thereof as Scaffold Materials", herein incorporated by reference in its entirety.

In an embodiment, there is provided herein a method for isolation and decellularisation of microchannels from a plant or fungal tissue, said method comprising:
separating microchannels from the plant or fungal tissue; and
decellularising the microchannels; and
optionally, sterilizing the microchannels.

As will be understood, in certain embodiments the microchannels will typically be separated from the plant or fungal tissue before decellularisation, however it is also contemplated that in certain embodiments the order may be switched such that decellularisation may be performed before the microchannels are separated from the plant or fungal tissue.

In certain embodiments, decellularisation may be performed before separating the microchannels from the plant or fungal tissue. In certain embodiments, such an approach may benefit from performing a perfusion method to facilitate the decellularising agent penetrating all relevant areas of the plant or fungal tissue.

In certain embodiments, a perfusion method may be applied to decellularise the plant or fungal tissue before the channels are isolated, and the perfusion may use substantially the same solutions and reagents as already described herein for performing decellularisation. A path may be provided (using a needle tip, for example) for decellularisation solutions to enter the plant or fungal tissues vasculature. It is contemplated that such approaches may be of particular interest for certain tissues, such as celery, which may readily draw up fluids through their vasculature. In certain embodiments, such approaches may be used to mainly or only decellularise microchannel structures of the plant or fungal tissue. By way of example, in certain embodiments perfusion may be used for targeted decellularisation (relatively quick), or total decellularisation (longer time), based for example on using multiple fluid injection sites, etc.

In another embodiment of the above method, the step of separating the microchannels may comprise mechanical separation of the microchannels, or vascular bundles containing the microchannels, or both, from surrounding plant or fungal tissue. In still another embodiment, the step of separating may be performed by gentle peeling, or by cutting.

In certain embodiments, the separation of the one or more structures, such as microchannels, from the plant or fungal tissue may comprise one or more manual steps which may be performed to extract or separate the one or more structures of interest from the surrounding plant or fungal tissue. Such manual steps may involve cutting, slicing, peeling, and/or other physical separation techniques. As will be understood, for large scale operations, such manual steps may become burdensome. As described herein, the present inventors thus developed extraction techniques which may be less burdensome and/or readily amendable to scale-up, for example. Accordingly, in certain embodiments, the step of separating may comprise a liquid-based extraction to isolate the one or more structures from the plant or fungal tissue.

As will be understood, liquid-based extraction may comprise any suitable process for treating and extracting one or more structures from the plant or fungal tissue (which may be either native plant tissue or fungal tissue, or decellularized plant tissue or fungal tissue, or a combination thereof) using a liquid extraction solution. In some cases, the plant tissue or fungal tissue may be processed before liquid-based extraction, such as by segmenting into smaller pieces or strips by a suitable apparatus, such as a scalpel or mandoline.

Liquid-based extraction will be understood by a person of skill in the art as a process for chemically treating and extracting vascular bundles (VBs) and/or microchannels from native plant tissue or fungal tissue or decellularised plant tissue or fungi tissue using a liquid solution. Liquid-based extraction may be understood as a maceration of the native or decellularised plant or fungal tissue to yield intact substructures, such as microchannels. Liquid-based extraction may result in the isolation of single microchannels or bundles of microchannels. In cases where native plant or fungal tissues are used, liquid-based extraction conditions may result in partial or full decellularisation of the native tissue. In such cases, the tissues may be further treated with any of the decellularisation methods discussed herein.

In embodiments described herein, the liquid solutions may be an acid solution, acid and peroxide solution, salt solution or alkaline solution for an acid extraction, acid and peroxide extraction, salt extraction or alkaline extraction, respectively. In some cases, more than one treatment or solution is used simultaneously or sequentially.

The one or more solutions may be combined with the plant or fungal tissue and heated to a desired temperature, such as boiling. In some cases, the solutions are heated or boiled for a period of time, such as 0.1-30 minutes. Periods of longer than 30 minutes are also contemplated, such as up to an hour or greater. Any amount of time within the range is contemplated or any value therebetween (optionally rounded to the nearest 0.1), or any subrange spanning between any two of these values.

In another embodiment any of the above method or methods, acid extraction may comprise mixing the plant or fungal tissue in an acid solution comprising acid. The mixture of plant or fungal tissue and acid solution is then heated. As will be understood, the acid solution may comprise generally any suitable acid capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable acids include, but are not limited to, acetic acid, boric acid, carbonic acid, hydrochloric acid, citric acid, hydrofluoric acid, nitric acid, oxalic acid, phosphoric acid, sulfuric acid, boron trifluoride, oxalic acid, malonic acid, succinic acid, malic acid and others. Other categories of acids are contemplated, such as carboxylic acids including, but are not limited to, linear saturated dicarboxylic acids, branched dicarboxylic acids, unsaturated dicarboxylic acids, substituted dicarboxylic acids, and aromatic dicarboxylic acids. The acid may be dissolved/mixed in a suitable solvent to form the acid solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the acid solution may comprise an aqueous solution of 50% acetic acid.

In another embodiment any of the above method or methods, acid and peroxide extraction may comprise mixing the plant or fungal tissue in an acid and peroxide solution comprising acid and peroxide. The mixture of plant or fungal tissue and acid and peroxide solution is then heated. As will be understood, the acid/peroxide solution may comprise generally any suitable acid and peroxide capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable acids include, but are not limited to, acetic acid, boric acid, carbonic acid, hydrochloric acid, citric acid, hydrofluoric acid, nitric acid, oxalic acid, phosphoric acid, sulfuric acid, boron trifluoride, oxalic acid, malonic acid, succinic acid, malic acid and others. Other categories of acids are contemplated, such as carboxylic acids including, but are not limited to, linear saturated dicarboxylic acids, branched dicarboxylic acids, unsaturated dicarboxylic acids, substituted dicarboxylic acids, and aromatic dicarboxylic acids. Examples of suitable peroxides include, but are not limited to, hydrogen peroxide, lithium peroxide, barium peroxide, dibenzoyl peroxide, benzoyl peroxide, methyl ethyl ketone peroxide. Peroxides may form peroxy acids when combined with acids, such as peracetic acid. The acid and peroxide solution may comprise glacial acetic acid and 30% hydrogen peroxide in a ratio of 5:1 to 1:5, such as 3:1 (13.05M acetic acid and 2.45M hydrogen peroxide) to 1:3 (containing 4.35M acetic acid and 7.35M hydrogen peroxide). The acid and peroxide may be dissolved/mixed in a suitable solvent to form the acid and peroxide solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the acid/peroxide solution may comprise an aqueous solution of 1:1 glacial acetic acid:30% hydrogen peroxide (v/v).

In another embodiment any of the above method or methods, salt extraction may comprise mixing the plant or fungal tissue in a salt solution. The mixture of plant or fungal tissue and salt solution is then heated. As will be understood, the salt solution may comprises generally any suitable salt capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable salts may be monovalent, such as LiCl and NaCl; divalent, such as MgSO₄, and CaCl₂; trivalent, such as AlCl₃ and others. Suitable cations include, but are not limited to: lithium, sodium, potassium, magnesium, calcium, iron, copper, zinc, aluminum and ammonium. Suitable anions include, but are not limited to: chloride, bromide, acetate, carbonate, citrate, fluoride, nitrate, phosphate, sulfate, iodide and borate. Medicinal salts such as ibuprofenate are also contemplated. Selection of a suitable salt may also depend on desired properties, such as ionic activity, screening, coordination size, Debye length, or ionic strength of interest. The salt solution may have a suitable salt concentration, such as about 0.5M-10M or any value therebetween (optionally rounded to the nearest 0.1), or any subrange spanning between any two of these values. The salt may be dissolved/mixed in a suitable solvent to form the salt solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the salt solution may comprise an aqueous solution of NaCl or LiCl, having a salt concentration of about 0.5M - 3M.

In another embodiment any of the above method or methods, alkaline extraction may comprise mixing the plant or fungal tissue in an alkaline solution. In some cases, the mixture of plant or fungal tissue and alkaline solution is heated. As will be understood, the alkaline solution may comprise generally any suitable base capable of osmotic shock of plant/fungal tissue, degradation of outer plant tissue structure, disruption of hydrogen bonding within the plant/fungal tissue and/or disruption of plant/fungal polymer crystal structures to extract tissue components, such as VBs and/or microchannels. Examples of suitable bases include, but are not limited to, sodium hydroxide (NaOH), potassium hydroxide (KOH), carbonates, nitrates, phosphates, sulfates, ammonia, calcium hydroxide, magnesium hydroxide, lithium hydroxide, zinc hydroxide, sodium carbonate, sodium bicarbonate, butyl lithium, sodium azide, sodium amide, sodium hydride, sodium borohydride, and lithium diisopropylamine. The alkaline solution may have a suitable alkaline concentration, such as about 0.5-10M or any value therebetween (optionally rounded to the nearest 0.1), or any subrange spanning between any two of these values. The base may be dissolved/mixed in a suitable solvent to form the alkaline solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the alkaline solution may comprise an aqueous solution of NaOH, having a base concentration of about 0.5M - 1M.

In another embodiment any of the above method or methods, the liquid-based extraction may further comprise mechanically agitating, for example stirring, the plant or fungal tissue in the alkaline solution. The solutions may be stirred using a stir stick or other suitable method, such as a mechanical or magnetic stirrer.

The methods for liquid-based extraction may be tuned to produce VBs and/or microchannels of a desired morphology and/or ultimate yield strength. The method may also be tuned to isolate VBs and/or microchannels from a desired plant or fungi. For example, longer heating or boiling times may result in softened microchannels and/or VBs, see Tables 1-8. Softened VBs and/or microchannels may be desirable for applications in which a pliable microchannel is desired. Tuning the mechanical properties of the isolated microchannels and/or VBs may result in directing cell growth and cell differentiation and control of the mechanical properties of the resulting engineered tissues. Hardened VBs and/or microchannels may be desirable for applications in which strength is desired, for example as a structure for cell growth. Higher concentrations of acid, base, salt (such as greater than 10M), and acid/peroxide (such as higher than 5:1-1:5), with a shorter boiling time is contemplated.

In still another embodiment, there is provided herein a method for preparing a scaffold biomaterial, said method comprising:
separating microchannels from a plant or fungal tissue;
decellularising the microchannels;
bundling the microchannels together, the microchannels being arranged substantially parallel to each other;
thereby providing a scaffold biomaterial comprising bundled microchannels.

As will be understood, in certain embodiments the microchannels will typically be separated from the plant or fungal tissue before decellularisation, however it is also contemplated that in certain embodiments the order may be switched such that decellullarization may be performed before the microchannels are separated from the plant or fungal tissue.

In certain embodiments of the above method, the step of separating the microchannels may comprise mechanical separation of the microchannels, or vascular bundles containing the microchannels, or both, from surrounding plant or fungal tissue. In certain embodiments, the step of separating may be performed by gentle peeling, or by cutting.

In certain embodiments, the separation of the one or more structures, such as microchannels, from the plant or fungal tissue may comprise one or more manual steps which may be performed to extract or separate the one or more structures of interest from the surrounding plant or fungal tissue. Such manual steps may involve cutting, slicing, peeling, and/or other physical separation techniques. As will be understood, for large scale operations, such manual steps may become burdensome. As described herein, the present inventors thus developed extraction techniques which may be less burdensome and/or readily amendable to scale-up, for example. Accordingly, in certain embodiments, the step of separating may comprise a liquid-based extraction to isolate the one or more structures from the plant or fungal tissue.

As will be understood, liquid-based extraction may comprise any suitable process for treating and extracting one or more structures from the plant or fungal tissue (which may be either native plant tissue or fungal tissue, or decellularized plant tissue or fungal tissue, or a combination thereof) using a liquid extraction solution. In some cases, the plant tissue or fungal tissue may be processed before liquid-based extraction, such as by segmenting into smaller pieces or strips by a suitable apparatus, such as a scalpel or mandoline.

Liquid-based extraction will be understood by a person of skill in the art as a process for chemically treating and extracting vascular bundles (VBs) and/or microchannels from native plant tissue or fungal tissue or decellularised plant tissue or fungi tissue using a liquid solution. Liquid-based extraction may be understood as a maceration of the native or decellularised plant or fungal tissue to yield intact substructures, such as microchannels. Liquid-based extraction may result in the isolation of single microchannels or bundles of microchannels. In cases where native plant or fungal tissues are used, liquid-based extraction conditions may result in partial or full decellularisation of the native tissue. In such cases, the tissues may be further treated with any of the decellularisation methods discussed herein.

In embodiments described herein, the liquid solutions may be an acid solution, acid and peroxide solution, salt solution or alkaline solution for an acid extraction, acid and peroxide extraction, salt extraction or alkaline extraction, respectively. In some cases, more than one treatment or solution is used simultaneously or sequentially.

The one or more solutions may be combined with the plant or fungal tissue and heated to a desired temperature, such as boiling. In some cases, the solutions are heated or boiled for a period of time, such as 0.1-30 minutes. Periods of longer than 30 minutes are also contemplated, such as up to an hour or greater. Any amount of time within the range is contemplated or any value therebetween (optionally rounded to the nearest 0.1), or any subrange spanning between any two of these values.

In another embodiment any of the above method or methods, acid extraction may comprise mixing the plant or fungal tissue in an acid solution comprising acid. The mixture of plant or fungal tissue and acid solution is then heated. As will be understood, the acid solution may comprise generally any suitable acid capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable acids include, but are not limited to, acetic acid, boric acid, carbonic acid, hydrochloric acid, citric acid, hydrofluoric acid, nitric acid, oxalic acid, phosphoric acid, sulfuric acid, boron trifluoride, oxalic acid, malonic acid, succinic acid, malic acid and others. Other categories of acids are contemplated, such as carboxylic acids including, but are not limited to, linear saturated dicarboxylic acids, branched dicarboxylic acids, unsaturated dicarboxylic acids, substituted dicarboxylic acids, and aromatic dicarboxylic acids. The acid may be dissolved/mixed in a suitable solvent to form the acid solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the acid solution may comprise an aqueous solution of 50% acetic acid.

In another embodiment any of the above method or methods, acid and peroxide extraction may comprise mixing the plant or fungal tissue in an acid and peroxide solution comprising acid and peroxide. The mixture of plant or fungal tissue and acid and peroxide solution is then heated. As will be understood, the acid/peroxide solution may comprise generally any suitable acid and peroxide capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable acids include, but are not limited to, acetic acid, boric acid, carbonic acid, hydrochloric acid, citric acid, hydrofluoric acid, nitric acid, oxalic acid, phosphoric acid, sulfuric acid, boron trifluoride, oxalic acid, malonic acid, succinic acid, malic acid and others. Other categories of acids are contemplated, such as carboxylic acids including, but are not limited to, linear saturated dicarboxylic acids, branched dicarboxylic acids, unsaturated dicarboxylic acids, substituted dicarboxylic acids, and aromatic dicarboxylic acids. Examples of suitable peroxides include, but are not limited to, hydrogen peroxide, lithium peroxide, barium peroxide, dibenzoyl peroxide, benzoyl peroxide, methyl ethyl ketone peroxide. Peroxides may form peroxy acids when combined with acids, such as peracetic acid. The acid and peroxide solution may comprise glacial acetic acid and 30% hydrogen peroxide in a ratio of 5:1 to 1:5, such as 3:1 (13.05M acetic acid and 2.45M hydrogen peroxide) to 1:3 (containing 4.35M acetic acid and 7.35M hydrogen peroxide). The acid and peroxide may be dissolved/mixed in a suitable solvent to form the acid and peroxide solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the acid/peroxide solution may comprise an aqueous solution of 1:1 glacial acetic acid:30% hydrogen peroxide (v/v).

In another embodiment any of the above method or methods, salt extraction may comprise mixing the plant or fungal tissue in a salt solution. The mixture of plant or fungal tissue and salt solution is then heated. As will be understood, the salt solution may comprises generally any suitable salt capable of osmotic shock of plant/fungal tissue, degradation of outer plant/fungal tissue structure, disruption of hydrogen bonding within the plant/fungal tissue and/or disruption of plant/fungal polymer crystal structures to extract tissue components, such as VBs and/or microchannels. Examples of suitable salts may be monovalent, such as LiCl and NaCl; divalent, such as MgSO₄, and CaCl₂; trivalent, such as AlCl₃ and others. Suitable cations include, but are not limited to: lithium, sodium, potassium, magnesium, calcium, iron, copper, zinc, aluminum and ammonium. Suitable anions include, but are not limited to: chloride, bromide, acetate, carbonate, citrate, fluoride, nitrate, phosphate, sulfate, iodide and borate. Medicinal salts such as ibuprofenate are also contemplated. Selection of a suitable salt may also depend on desired properties, such as ionic activity, screening, coordination size, Debye length, or ionic strength of interest. The salt solution may have a suitable salt concentration, such as about 0.5M-10M or any value therebetween (optionally rounded to the nearest 0.1), or any subrange spanning between any two of these values. The salt may be dissolved/mixed in a suitable solvent to form the salt solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the salt solution may comprise an aqueous solution of NaCl or LiCl, having a salt concentration of about 0.5M - 3M.

In another embodiment any of the above method or methods, alkaline extraction may comprise mixing the plant or fungal tissue in an alkaline solution. In some cases, the mixture of plant or fungal tissue and alkaline solution is heated. As will be understood, the alkaline solution may comprise generally any suitable base capable of osmotic shock of plant/fungal tissue, degradation of outer plant tissue structure, disruption of hydrogen bonding within the plant/fungal tissue and/or disruption of plant/fungal polymer crystal structures to extract tissue components, such as VBs and/or microchannels. Examples of suitable bases include, but are not limited to, sodium hydroxide (NaOH), potassium hydroxide (KOH), carbonates, nitrates, phosphates, sulfates, ammonia, calcium hydroxide, magnesium hydroxide, lithium hydroxide, zinc hydroxide, sodium carbonate, sodium bicarbonate, butyl lithium, sodium azide, sodium amide, sodium hydride, sodium borohydride, and lithium diisopropylamine. The alkaline solution may have a suitable alkaline concentration, such as about 0.5-10M or any value therebetween (optionally rounded to the nearest 0.1), or any subrange spanning between any two of these values. The base may be dissolved/mixed in a suitable solvent to form the alkaline solution. Typically, the solvent may comprise water, although other solvents, or combinations of solvents, are also contemplated such as, but not limited to, propanol, ethanol, methanol, ammonia, acetic acid, acetone, dimethylformamide, dimethylsulfoxide and amphiphilic solvents or colloids. By way of example, in certain embodiments, the alkaline solution may comprise an aqueous solution of NaOH, having a base concentration of about 0.5M - 1M.

In another embodiment any of the above method or methods, the liquid-based extraction may further comprise mechanically agitating, for example stirring, the plant or fungal tissue in the alkaline solution. The solutions may be stirred using a stir stick or other suitable method, such as a mechanical or magnetic stirrer.

The methods for liquid-based extraction may be tuned to produce VBs and/or microchannels of a desired morphology and/or ultimate yield strength. The method may also be tuned to isolate VBs and/or microchannels from a desired plant or fungi. For example, longer heating or boiling times may result in softened microchannels and/or VBs, see Tables 1-8. Softened VBs and/or microchannels may be desirable for applications in which a pliable microchannel is desired. Tuning the mechanical properties of the isolated microchannels and/or VBs may result in directing cell growth and cell differentiation and control of the mechanical properties of the resulting engineered tissues. Hardened VBs and/or microchannels may be desirable for applications in which strength is desired, for example as a structure for cell growth. Higher concentrations of acid, base, salt (such as greater than 10M), and acid/peroxide (such as higher than 5:1-1:5), with a shorter boiling time is contemplated.

In certain embodiments, liquid-based extraction may result in substantial sterilization (concurrent sterilization). In other cases, the isolated microchannels and/or VBs are sterilized after using any of the sterilization methods described herein.

In certain embodiments HDMC bundles may be formed from a plurality of vascular bundles (comprising xylem and phloem channels) obtained from plants. In certain embodiments, the vascular bundles may be further processed to isolate individual xylem and/or phloem channels, which may then be used to form HDMC bundles, which may have even higher density. As will be understood, higher densities may be achieved by removing more of the non-vascular plant tissue surrounding the microchannels. Non-vascular tissue may typically refer to parenchyma and sclerenchyma tissue. In certain embodiments, it is contemplated that channel density may also be increased by, for example, selection of microchannels that are thinner-walled or smaller by cross-sectional area (which may be plant species-dependent, and may be selected with the desired application in mind such that size does not become smaller than the cells to be accommodated, for example), and/or by reduction in glue used at the interchannel-space through refinements (typically macro-scale, mechanical consideration) to the molding/gluing steps, for example.

In certain embodiments of any of the above methods, the step of bundling may comprise gluing the microchannels together. In certain embodiments, the microchannels may be glued together by a biocompatible, optionally biodegradable, glue. In certain embodiments, the biocompatible glue may comprise a PEG-based, polyurethane-based, gelatin-based, or a fibrin-based glue. In certain embodiments, the biocompatible glue may comprise a fibrin-based glue.

In certain embodiments of any of the above methods, the step of bundling may comprise molding the microchannels. In certain embodiments, the molding may be performed using a mold into which the microchannels are packed. In certain embodiments, the mold may comprise biomedical-grade silicone. In certain embodiments, the mold may comprise polypropylene plastic, or Teflon or another non-stick material or coating. In certain embodiments, the mold may: comprise a non-stick material to which the glue does not stick or only weakly sticks; be biocompatible; may be disposable; may be autoclavable; may be moldable or somewhat soft; or any combinations thereof. In certain embodiments, the mold may comprise a passage for receiving the microchannels therein, the passage having cross-sectional dimensions which are imparted to the microchannels being molded therein. In certain embodiments, the mold may be divided into two or more sections, which can be assembled around the microchannels for molding. In certain embodiments, glue may be added to a surface of the mold facing the microchannels, and the microchannels may be packed in the mold and held therein while the glue cures. In certain embodiments, the mold may comprise a first section and a second section, and the molding may comprise adding glue to a surface of the first section facing the microchannels and to a surface of the second section facing the microchannels; placing the microchannels in the first section and in the second section in contact with the glue (the microchannels may be divided between the first and second sections); adding glue to an exposed surface of the microchannels in the first section, the microchannels in the second section, or both; installing the second section with associated microchannels over the exposed surface of the microchannels associated with the first section, opposite the first section, thereby assembling the mold; allowing the glue to substantially cure; and removing the mold.

In certain embodiments of any of the above methods, the step of bundling may include physically binding or physically cross-linking the microchannels and/or VBs without the use of glue. Such a process may be referred to as entanglement of the microchannels. The microchannels may be physically tied or otherwise tangled such that separation of the microchannels and/or VBs requires the use of force. Different dimensions and using different lengths (Kuhn segments or persistence length) may make different entanglements and alignment profiles. Entanglement may occur from stirring with a stirring device, such as a rod. Entanglement may occur during liquid-based extraction.

In certain embodiments of any of the above methods, the method may further comprise a step of cutting the bundled microchannels to a desired length. In certain embodiments, the step of cutting the bundled microchannels to a desired length may be performed before the mold is removed.

In yet another embodiment of any of the above methods, the method may further comprise a step of sterilizing the microchannels. In another embodiment, the step of sterilizing may comprise exposing the microchannels to ethanol, or a mixture of ethanol and water.

In certain embodiments of any of the above method, the microchannels may comprise xylem and/or phloem channels.

In another embodiment of any of the above methods, the decellularised microchannels may be cellulose-based, chitin-based, lignin-based, hemicellulose-based, or pectin-based, or any combination thereof.

In still another embodiment of any of the above method or methods, a density of decellularised microchannels within the bundle may be greater than a density of microchannels within the plant or fungal tissue.

In still another embodiment of any of the above method or methods, the plant or fungal tissue may comprise a microchannel-containing tissue from apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, a rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue, or a genetically altered tissue produced via direct genome modification or through selective breeding, or any combinations thereof. In another embodiment, the plant or fungal tissue may comprise celery, asparagus, or both. Additional examples of plant and fungal tissues are described in Example 18 of WO2017/136950, entitled "Decellularised Cell Wall Structures from Plants and Fungus and Use Thereof as Scaffold Materials", herein incorporated by reference in its entirety.

Examples of decellularisation protocols which may be adapted for producing decellularised microchannels for scaffold biomaterials as described herein may be found in WO2017/136950, entitled "Decellularised Cell Wall Structures from Plants and Fungus and Use Thereof as Scaffold Materials", herein incorporated by reference in its entirety.

Various methods may be used for decellularisation. By way of example, in certain embodiments, decellularisation may include decellularisation by thermal shock, treatment with detergent (e.g. SDS, Triton X, EDA, alkaline treatment, acid, ionic detergent, non-ionic detergents, and zwitterionic detergents), osmotic shock, lyophilisation, physical lysing (e.g. hydrostatic pressure ), electrical disruption (e.g. non thermal irreversible electroporation), or enzymatic digestion, or any combination thereof. In certain embodiments, decellularisation processes may comprise any of several approaches (either individually or in combination) including, but not limited to, thermal shock (for example, rapid freeze thaw), chemical treatment (for example, detergents), osmotic shock (for example, distilled water), lyophilisation, physical lysing (for example, pressure treatment), electrical disruption and/or enzymatic digestion.

In certain embodiments, decellularisation may comprise treatment with a detergent or surfactant. Examples of detergents may include, but are not limited to sodium dodecyl sulphate (SDS), Triton X, EDA, alkaline treatment, acid, ionic detergent, non-ionic detergents, and zwitterionic detergents.

In still further embodiments, the decellularised microchannels may comprise microchannels which have been decellularised by treatment with SDS. In still another embodiment, residual SDS may be removed from the microchannels by washing with an aqueous divalent salt solution. The aqueous divalent salt solution may be used to precipitate/crash a salt residue containing SDS micelles out of the solution/scaffold, and a dH₂O, acetic acid or dimethylsulfoxide (DMSO) treatment, or sonication, may have been used to remove the salt residue or SDS micelles. In certain embodiments, the divalent salt of the aqueous divalent salt solution may comprise, for example, MgCl₂ or CaCl₂.

In another embodiment, the microchannels may be decellularised by treatment with an SDS solution of between 0.01 to 10%, for example about 0.1% to about 1%, or, for example, about 0.1% SDS or about 1% SDS, in a solvent such as water, ethanol, or another suitable organic solvent, and the residual SDS may have been removed using an aqueous CaCl₂ solution at a concentration of about 100mM followed by incubation in dH₂O. In certain embodiments, the SDS solution may be at a higher concentration than 0.1%, which may facilitate decellularisation, and may be accompanied by increased washing to remove residual SDS. In particular embodiments, the microchannels may be decellularised by treatment with an SDS solution of about 0.1% SDS in water, and the residual SDS may have been removed using an aqueous CaCl₂ solution at a concentration of about 100mM followed by incubation in dH₂O.

In another embodiment, any of the above method or methods may further comprise a step of introducing living plant or animal cells to the microchannels. In another embodiment, any of the above method or methods may further comprise a step of culturing the living plant or animal cells on and/or in the scaffold biomaterial. In an embodiment, the living cells may comprise mammalian cells, such as human cells.

In certain embodiments, microchannels and/or scaffold biomaterials may be functionalized with specific growth factors selected for particular cell types of interest. Functionalization may, in certain embodiments, include providing growth factors adhered to the scaffold, for example.

In certain embodiments, for spinal cord injury applications in particular, it is contemplated that patient-derived neural progenitor cells may be added to the scaffolds as described herein to promote repair and/or recovery.

In another embodiment, there is provided herein a decellularised microchannel produced by any of the above method or methods.

In still another embodiment, there is provided herein a scaffold biomaterial produced by any of the above the method or methods.

In another embodiment, there is provided herein a kit comprising any one, two, three, or more of:
a mold;
a glue;
one or more microchannels;
one or more decellularisation agents;
a scalpel or microtome;
sterile measuring device;
sterile saline;
tweezers; and/or
instructions for performing any of the methods as described herein.

In the examples described below, AS and CL plant tissue sources are identified as providing particularly desirable characteristics. AS is well-characterized and worked quite well, and CL provides benefits such as ease of vascular bundle extraction and a higher number of xylem channels per vascular bundle as compared to AS. However, it will be understood that a wide variety of other plant source tissues may be used. In the examples below, custom-designed molds made of biomedical grade silicone were used to package/densify and glue vascular bundles. It will be understood that a variety of other mold materials are also contemplated, and that generally any suitable material may be used which will not substantially adhere to the glue/sealant and/or HDMC bundles and which may be suitably biocompatible or otherwise compatible with the HDMC bundles being produced.

### EXAMPLE 1 - Preparation of High-Density Microchannels (HDMC) and Biocompatibility Analysis Thereof

Described herein are methods for isolation and decellularisation of individual vascular bundles or vascular tissues of any suitable plant, while aiming to avoid or minimize introduction of processing residuals or contaminants. Bundling/densification of these vascular channels or tissues by various glues and sealants (including natural-origin and/or synthetic glues) is investigated. Creation of a custom-designed mold technology providing for the gluing of vascular bundles (or generally any plant microchannel structure) in a 3D arrangement without substantially causing damage or introducing impurities is described.

In the present studies, transport structures present in plant vascular bundles (i.e. the xylem and phloem channels) were utilized to prepare high-density microchannel (HDMC) bundles for a variety of applications. Xylem and phloem channels chiefly transport water and sugars, respectively, in plants, and are physically and compositionally distinct from the surrounding tissue in the plant. They can be visualized as long contiguous tube-shaped structures, and their walls typically possess a relatively higher lignin content compared to other structures in the plant. Lignin may confer structural rigidity to these structures, and as described herein the rigidity may assist with isolation of these channels (mechanically) from the surrounding non-vascular ground tissue.

As will be understood, the chemical composition and structural layout of vascular bundles may vary, and may be highly dependent on the particular plant species. In these studies, asparagus-sourced (AS) and celery-sourced (CL) source materials are identified as providing favorable structural characteristics for the application of their decellularised vascular bundles as a guiding support matrix for the growth and regeneration of certain cell types, for example. As will be understood, selection of plant source may be tailored for the particular intended application of the resultant HDMC.

In these studies, Asparagus (family: Asparagaceae) and Celery (family: Apiaceae) were selected as source plant materials. These plants were found to possess certain favourable characteristics for HDMC fabrication, which may include the following: Vascular bundles which may be isolated from surrounding ground tissue (i.e. non-vascular tissue) due to differences in the makeup of structural carbohydrates between vascular tissue and ground tissue; Xylem channel diameter large enough to accommodate a variety of cell types and structures (including the soma of neurons, for example); and relatively high density of xylem and phloem channels within vascular bundles.

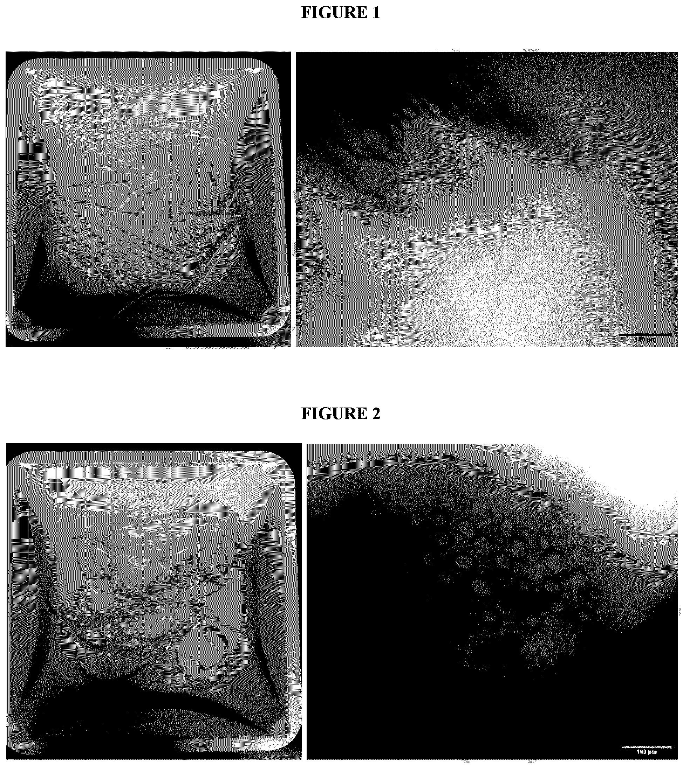

Figure 1 shows isolated AS vascular bundles, approx. 3 cm length (left panel), and microscopic cross-sectional view of an AS vascular bundle, with outlines of xylem channels clearly visible against the background (right panel). Figure 2 shows isolated CL vascular bundles, variable length 5 - 10 cm (left panel) and microscopic cross-sectional view of a CL vascular bundle, with outlines of xylem channels clearly visible against the ground tissue background (right panel).

As detailed herein, isolated vascular structures, such as plant vascular bundles, may be glued together to produce bundles of controllable (or arbitrary) diameter based on the materials selected and used, with controllable (or arbitrary) length typically limited on the upper end only by the length of the isolated individual structures used to prepare the HDMCs (unless consecutive lengthwise fusing or linking of two or more structures is performed to obtain longer lengths). In certain embodiments, HDMCs may be bundled together or cut and recombined into generally any desired shape and/or length. Typically, HDMCs will be cut to a desired length suitable for the desired application.

Plant vascular structures, comprising lignocellulose, are considered unique for their biocompatibility, their structural integrity, and their directionality. As described herein, it is contemplated herein these features may be imparted to HDMCs and harnessed for a number of different applications including, but not limited to, the repair and replacement of extracellular matrices even where functioning may be highly dependent on their directionality (for example, in the extracellular matrix of the spinal cord). In embodiments where glue is used for preparing HDMCs, it is contemplated that the glue may typically be biocompatible and (optionally, but preferably) biodegradable, so as to leave behind mainly the vascular plant structures in their desired orientation and location within a host or physical support, for example.

In the studies described in this Example, HDMCs were prepared from AS and CL, and their biocompatibilities were assayed.

### Method of HDMC Preparation:

Vascular bundles were first isolated from AS or CL stalks by scalpel blade or by gentle peeling, respectively. A dissection microscope may be used to facilitate visualization, but was not necessary for the trained eye. The vascular bundles in both AS and CL were visually distinguishable from the surrounding ground tissue. If desired, contrast may be further enhanced by leaving the stalks in colored water overnight at +4 C; such that the vascular bundles take up the colored water. In any case, excess ground tissue surrounding an isolated vascular bundle may be removed by scalpel blade. Successful isolation of undamaged individual vascular bundles was achieved in this manner.

The isolated vascular bundles may then, optionally, be cut to a specific length (by scalpel or microtome blade, for example). Thickness of vascular bundles may also be recorded by digital calipers, with care taken not to crush the channels between the caliper arms.

The vascular bundles were then decellularised in a 9-day process utilizing sodium dodecyl sulfate, based on decellularisation protocol as described in detail in WO2017/136950, entitled "Decellularised Cell Wall Structures from Plants and Fungus and Use Thereof as Scaffold Materials", herein incorporated by reference in its entirety. decellularised bundles were then sterilized by immersion in 70% ethanol and stored in sterile DPBS at 4 C until use.

Isolated vascular bundles were bundled together to provide high-density microchannels (HDMCs). In this study, bundling of the isolated vascular bundles was achieved using a custom-made mold and gluing. The custom mold was made of a biomedical-grade silicone, which was found not adhere to the fibrin glue used to glue the vascular bundles together in these studies. Prior to the development of the silicone mold, molds made from drinking straws were also successfully used. Drinking straw materials are food-grade, and these straws generally possess a small internal diameter comparable to the diameter of the spinal cord of rats and therefore may also be suitable for certain applications. However, plastic and silicone straws were less effective as molds than the biomedical-grade silicone mold, since the glue adhered to the plastic straw, and silicone straws were more difficult to manipulate due to their flexibility.

Figure 3 shows the custom biomedical-grade silicone mold (5-mm internal diameter, 1-cm length) used for vascular bundle packing in these studies (left panel). Mold halves are pressed together, and the right panel shows a 2 mm-thick slice of mold containing glued CL vascular bundles.

The bundling process was carried out under a laminar flow hood to minimize chances of contaminants becoming lodged in the glued bundle. All handling was done by autoclaved tweezers. Firstly, the fibrin glue kit and its dual-syringe application system was prepared following the manufacturer's instructions (TISSEEL Kit; Baxter Healthcare, catalog no. 1503152; document ID 0716820). Then, the individual channels (vascular bundles) to be glued together were laid out in a sterile staging area. The channels were kept moist to avoid drying out and collapse of the internal xylem and phloem. Finally, silicone mold halves were lined up in a secondary sterile staging area and packed with the vascular bundles using tweezers. Packing density depended on the internal diameter of the silicone molds, and the average thickness of the isolated plant vascular bundles selected for use.

Figure 4 shows AS channel bundles during the glue curing step, with mold halves pressed together, and Figure 5 shows CL channel bundles during the glue curing step, with mold halves pressed together.

Once packed in the mold halves, the channels were removed, but not separated. Two to three drops of glue were quickly deposited into each mold half's internal channel, and the packed channels were immediately placed back into the molds. Two to three drops of glue were then deposited on top of the packaged channels, and the mold halves were pressed together by manipulation with tweezers. The molds were then left undisturbed for about 15 min to allow for glue curing. The amount of glue to successfully bundle the channels was suitably selected, as too little glue may result in channels coming apart during the mold removal step, and too much glue may result in large excess "globes" of glue adhering to the exterior of bundles. Also, the location of the initial glue deposits was shown to be helpful to provide uniform gluing/adherence throughout the bundle.

In these studies, the glue was carefully selected. It was desirous that the glue be biocompatible, biodegradable, capable of adhering to moist lignocellulosic materials (but not mold material such as silicone), non-toxic for internal use, and preferably quick-setting. A cyanoacrylate glue was tested as well, but was less effective than the fibrin glue since molds proved difficult to remove from the cyanoacrylate glue. Also, although physical gluing was successful, it was noted that cyanoacrylate glue type may generate toxic degradation products under certain conditions for internal use according to literature reports on surgical glues and sealants.

After the glue was set, excess channel length may be cut away before the bundles are removed from their molds. In these studies, this was best achieved using a microtome blade (very sharp). At this stage, exact lengths of glued bundles may be produced generally without damage to the bundle by cutting directly through the molds. The molds may provide support as compared with exposed glued bundles which may be somewhat crushed under certain cutting pressure.

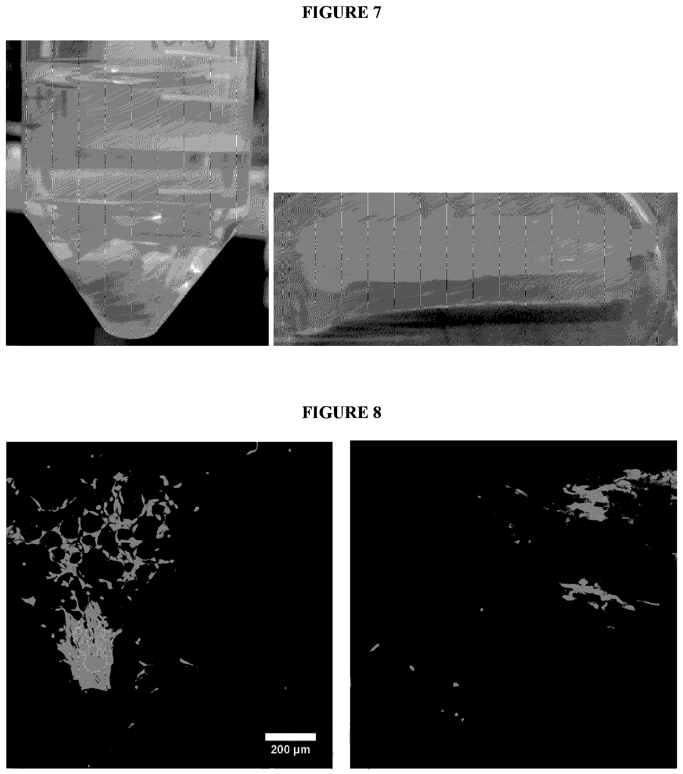

The mold halves were gently pulled apart by tweezer manipulation, revealing the final product: high density microchannels (HDMC) comprising glued plant vascular bundles. The HDMC bundles may be sterilized at this stage, for example by immersion in 70% ethanol for 30 min before storage in sterile DPBS at 4 C. Figure 6 shows HDMC (AS) bundles stored in DPBS (left panel) and individual HDMC (AS) immediately after removal from silicone mold (right panel). Figure 7 shows HDMC (CL) bundles stored in DPBS (left panel) and individual HDMC (CL) immediately after removal from silicone mold (right panel).

### Biocompatibility Analysis of HDMC:

Biocompatibility of HDMC was firstly assessed by 1 week of cell culture with a 3T3 fibroblast cell line expressing green fluorescent protein (GFP-3T3). Briefly, sections of HDMC were sliced and cells were seeded on the cross-sectional face of sections. After 1 week under standard cell culture conditions (37°C, 5% CO₂), the sections were fixed with a 4% paraformaldehyde solution for microscopy. Figures 8 (AS-based HDMC) and 9 (CL-based HDMC) evidence the presence of cells on the HDMC as well within the microchannels. Together, the results support that the HDMC bundles were biocompatible.

Figure 8 shows Confocal Laser-Scanning Micrographs of HDMC (AS-based) sample after 1 week of GFP-3T3 cell culture followed by fixing in 4% paraformaldehyde. Cells (green-green fluorescent protein) are visible in and around xylem microchannels (blue-autofluorescence). The left panel shows a cross-sectional view of single CL vascular channel within a glued bundle, with cells visible within xylem channels. Note cell density surrounding openings of xylem and phloem channels. The right panel shows a cross-sectional (partial) view of 2 vascular channels within a glued bundle, cells scattered throughout.

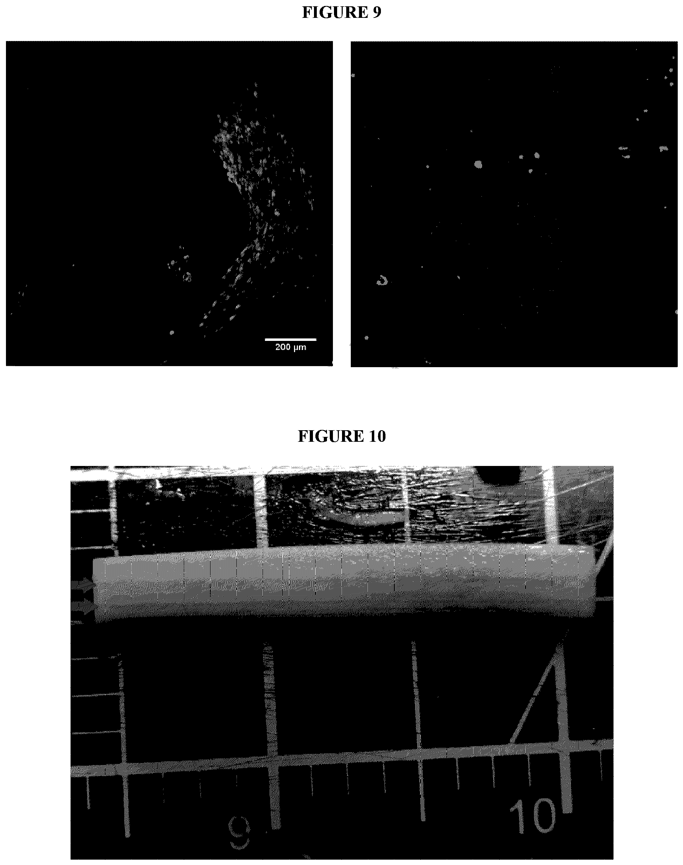

Figure 9 shows Confocal Laser-Scanning Micrographs of HDMC (CL-based) sample after 1 week of GFP-3T3 cell culture followed by fixing in 4% paraformaldehyde. Cells (green-green fluorescent protein) were visible in and around xylem microchannels (blue-autofluorescence). The left panel shows a cross-sectional view of single CL vascular channel within a glued bundle, with cells visible within xylem channels. Note the cell adherence to a fibrin glue deposit located to the right of the vascular channel. The right panel shows cross-sectional view of 2 vascular channels within a glued bundle, cells scattered throughout.

Figures 8 (AS-based HDMC) and 9 (CL-based HDMC) evidence the presence of cells on the HDMC as well within the microchannels. Together, the results support that the HDMC bundles were biocompatible.

### EXAMPLE 2 - Example of a Standard Operating Procedure (SOP) Developed for Extraction and Isolation of Plant or Fungal (i.e. AS) Vascular Bundles (VBs)

Described below is an example of a standard operating procedure (SOP) developed for extraction and isolation of plant or fungal vascular bundles and/or microchannels. This SOP was used for extraction and isolation of vascular bundles from AS sources.

### Safety Statement(s)

Guard against accidental cuts by maintaining awareness of scalpel blade position and sidedness of microtome blade. Consult all appropriate MSDS. Wear appropriate personal protection equipment such as vinyl gloves, lab coat, and goggles.

### Solutions

### Dulbecco's Phosphate-Buffered Saline

| | |
|---|---|
| DPBS (Hyclone, catalog no. 350-000-CL) | bottle 500 ml |
| Stored at 4°C in the dark. | |
| Do not use past expiration date. | |

### Procedure

### Selection of asparagus

An asparagus bundle was selected from the produce section of a grocer. The bundle consisting of large-diameter (> 1.0 cm) asparagus spears was preferably selected. Following selection and purchase, the asparagus was immediately transported to a refrigerated storage environment (4 C). An asparagus bundle that is not processed within 1 h of storage may be placed in shallow water (maintaining refrigeration) to prevent drying out of the spears.

An asparagus bundle was preferably processed within 24 h of purchase.

### Production of AS vascular bundles

[Note: if another plant or fungal tissue was previously processed, all tools and cutting mat may be disinfected with 70% ethanol and Accel TB solutions before proceeding]

Wearing new pair of gloves, the produce tag identifying the asparagus producer and the PLU number was removed from the bundle and recorded. The asparagus spears were unbundled from their packing elastic band and rinsed under tap water. The spears were placed in a beaker of shallow water next to the cutting mat.

A large weighing boat was placed next to the cutting mat and a small volume (~25 ml) of DPBS was poured into the boat. The boat was used to hold isolated AS vascular bundles as they were produced.

A scalpel (Feather #10) and a microtome blade were prepared for use.

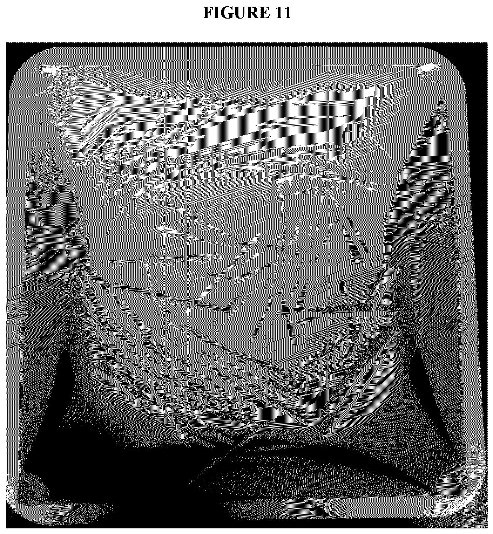

The spears were processed one-by-one. The following procedure applies to each individual spear:
A spear was removed from the shallow water beaker and placed onto the cutting mat.
The bottom 2 cm (approximately) of the spear was cut using the microtome blade. If the spear's new end still appears discolored, dry, or otherwise damaged, further segments of 1 cm are cut until the end consists of fresh undamaged tissue.
A 3-cm piece (termed "stump"), measured from freshly cut bottom end of the spear, was cut using the microtome blade. This stump corresponds to the spear's "bottom portion".
The 3-cm stump was sectioned into lengthwise halves using the microtome blade.
The resulting halves were examined for vascular bundles visible near the cut surfaces (See Figure 10 for example).
Each stump half was brought to the mandoline blade (; adjusted to approximately 1 mm-thick slicing) where it was sliced into approximately 1-mm-thick slices until no more slices could be produced. The slices were made to fall directly into a weighing boat containing DPBS.
The weighing boat containing the thin slices was brought to a cutting board. The slices were expected for vascular bundles running across the sheet. Vascular bundles running across at least ¾ of the slice length were selected for isolation in the next step.
The vascular bundles were individually cut-out from the slices by scalpel blade or microtome blade. Cuts with the scalpel blade were preferably made straight and even (no lifting of the blade, as this can create serrations on the sides of the isolated VB). Care was taken to remove as much surrounding ground tissue while extracting VBs.
Isolated VBs cuts were not retouched by any cutting tool, as this may create uneven edges or serrations.
Isolated vascular bundles were immediately visually inspected. Any of the following observations resulted in the isolated vascular bundle being discarded:
   - A kink or break in the vascular bundle.
   - Any VB with a continuous vasculature less than 1.5 cm long.
Isolated vascular bundles which pass visual inspection were immediately deposited into a weighing boat (containing DBPS) using clean tweezers (See Figure 11).
All slices were processed, one-at-a-time, for the isolation of VBs as described above.
After all slices had been processed, another 3-cm stump piece from the asparagus spear, was cut using the microtome blade. This stump corresponds to the spear's "middle" portion.
The 3-cm piece was cut and processed exactly as the first 3-cm stump, generating more isolated vascular bundles. Optionally, extracted VBs may be sorted depending on their origin in the asparagus spear (bottom, middle, or top stumps).
A final 3-cm stump piece was cut and processed exactly as the first and second 3-cm stumps, generating more isolated vascular bundles.

Asparagus spears were processed in the manner described above until the target number of VBs had bene generated.

If any single asparagus spear displayed any signs of the following, processing was halted, and the entire AS batch was discarded:
- Mold
- Rot
- Insect infestation

If a spear displayed any signs of the following, the spear was discarded and not processed (but processing of remaining spears may still proceed, and the batch was not discarded):
- Splitting or cracks due to dryness
- Excessively curved or otherwise misshapen spears.

Note: If the process was interrupted for longer than 0.5 h, the spear currently undergoing processing was discarded and the remaining spears (in shallow water) were returned to a refrigerated environment (4 C). All remaining spears were preferably processed within 24 h of their purchase.

### Preparation of AS vascular bundles for decellularisation

Using tweezers, isolated AS vascular bundles within the weighing boat were placed into a 50-ml falcon tube. The vascular bundles were counted; the total count per tube was indicated on the tube.

Note: A 50-ml centrifuge tube (Nunc) preferably contained no more than 60 AS vascular bundles. If the weighing boat contains more than 60 vascular bundles, multiple 50-ml tubes were used.

Each tube was labeled with the following information:
- The date in YYYY-MM-DD format.
- The operator's three-letter identifying initials.
- A production code starting with the letters "AS".
- The number of AS vascular bundles contained inside the tube was written on the tube cap.

If VB decellularisation is desired, the tubes were immediately processed for decellularisation following the SOP set out in Example 4 below.

### Disposal of plant tissue waste and cleaning of tools

Waste asparagus and celery tissue was collected into a disposable bag. The waste material was sorted as compostable material.

The cutting mat was rinsed with tap water. Dried plant tissue was gently removed. The mat was thoroughly disinfected with a 70% ethanol solution as well as an Accel TB solution.

The chef knife, tweezers, scalpel blade, and microtome blades were cleaned in same manner as the cutting mat above.

Metallic tools were gently wiped dry using a KimWipe.

DPBS solution in the weighing boat was collected in appropriate liquid waste jugs.

### EXAMPLE 3 - Example of a Standard Operating Procedure (SOP) Developed for Extraction and Isolation of Plant or Fungal (i.e. CL) Vascular Bundles (VBs)

Described below is an example of a standard operating procedure (SOP) developed for extraction and isolation of plant or fungal vascular bundles and/or microchannels. This SOP was used for extraction and isolation of vascular bundles from CL sources.

### Safety Statement(s)

Guard against accidental cuts by maintaining awareness of scalpel blade position and sidedness of microtome blade. Consult all appropriate MSDS. Wear appropriate personal protection equipment such as vinyl gloves, lab coat, and goggles.

### Solutions

### Dulbecco's Phosphate-Buffered Saline

| | |
|---|---|
| DPBS (Hyclone, catalog no. 350-000-CL) | bottle 500 ml |
| Stored at 4°C in the dark. | |
| Do not use past expiration date. | |

### Procedure

### Selection of celery

A celery bunch was selected from the produce section of a grocer. The bunch did not contain any obviously damaged stalks, and stalks were preferably firm. Following selection and purchase, the celery bunch was immediately transported to a refrigerated storage environment (4 C).

A celery bunch was preferably processed within 24 h of purchase.

### Production of CL vascular bundles

[Note: if another plant or fungal tissue was previously processed, all tools and cutting mat were disinfected with 70% ethanol and Accel TB solutions before proceeding]

Wearing a new pair of gloves, the produce tag identifying the celery producer and the PLU number (if present) was removed from the celery and recorded in a logbook. The celery was rinsed under tap water.

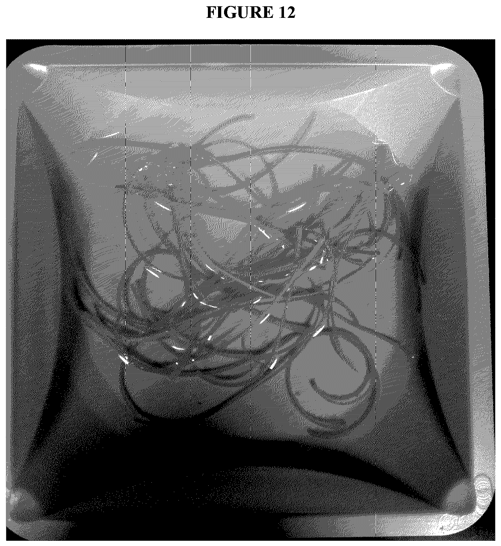

The celery was placed next to the cutting mat. A scalpel (Feather #10) and a chef's knife were prepared for use. A large weighing boat was placed next to the cutting mat and a small volume (~50 ml) of DPBS was poured into the boat. The boat was used to hold isolated AS vascular bundles as they were produced. A single celery stalk was manually snapped off from the bunch and was placed on the cutting mat. The following steps apply to all individual celery stalks:
The bottom 4 to 5 cm of the stalk were cut off using the chef's knife. This bottom portion of a stalk is typically thicker than the rest of the stalk and often harbors dirt particles on its surface.
If the top portion of the stalk has any leaves, the top portion was cut off using the chef's knife until no leaf-bearing portion remains.
A 1 to 2 cm section of the stalk, from the top, was gently manually snapped with care taken not to sever the vascular bundles running near the outside surface of the stalk.
The exposed vascular bundles were peeled and pulled off the entire length of the stalk. Peeling was performed slowly so as to avoid breakage of the vascular bundles while they were still embedded in the surrounding celery tissue. The exposed vascular bundles may be peeled away either as a group or individually.
The peeled vascular bundles were set on the cutting board. Excess loosely attached ground tissue was manually removed by gloved hand.
The vascular bundles were cut into 6-cm long sections using the scalpel.
Isolated vascular bundles were immediately visually inspected. Any of the following observations resulted in the isolated vascular bundle being discarded:
   - A kink or break in the vascular bundle.
   - Excessive coiling, such that the vascular bundle tends to naturally roll up into a stiff ring.
Freshly cut vascular bundles which passed visual inspection were deposited into a weigh boat containing Dulbecco's Phosphate Buffered Saline (DPBS) (see Figure 12).

All celery stalks, except the inner-most clear-colored stalks of the bunch, were processed in the manner described above. The unused inner stalks were discarded.

If any single celery stalk displayed any signs of the following, processing was halted, and the entire CL batch was discarded:
- Mold
- Rot
- Insect infestation

If a spear displays any signs of the following, the stalk was discarded and not processed (but processing of remaining spears may still proceed, and the batch was not discarded):
- Splitting or cracks due to dryness
- Broken or split stalks due to damage at the grocer or during transportation.

Note: If the process was interrupted for longer than 0.5 h, the stalk currently undergoing processing was discarded and the remaining stalks were returned to a refrigerated environment (4 C). All remaining stalks were preferably processed within 24 h of their purchase.

### Preparation of CL vascular bundles for decellularisation

Using tweezers, isolated CL vascular bundles within the weighing boat were placed into a 50-ml falcon tube. The vascular bundles were counted; the total count per tube was indicated on the tube.

Note: A 50-ml falcon tube contained no more than 50 CL vascular bundles. If the weighing boat contained more than 50 vascular bundles, multiple 50-ml falcon tubes were used.

Each tube was labeled with the following information:
- The date in YYYY-MM-DD format.
- The operator's three-letter identifying initials.
- A production code starting with the letters "CL".
- The number of CL vascular bundles contained inside the tube was written on the tube cap.

The tubes were immediately processed for decellularisation following SOP set out in Example 4 below.

### Disposal of plant tissue waste and cleaning of tools

Waste asparagus and celery tissue was collected into a disposable bag. The waste material was sorted as compostable material.

The cutting mat was rinsed with tap water. Dried plant tissue was gently removed. The mat was thoroughly disinfected with a 70% ethanol solution as well as an Accel TB solution.

The chef knife, tweezers, scalpel blade, and microtome blades were cleaned in same manner as the cutting mat above. Metallic tools were gently wiped dry using a KimWipe. DPBS solution in the weighing boat was collected in appropriate liquid waste jugs.

### EXAMPLE 4 - Example of a Standard Operating Procedure (SOP) Developed for decellularisation of Plant or Fungal (i.e. AS and CL) Vascular Bundles (VBs) by Sodium Dodecyl Sulfate (SDS)

Described below is an example of a standard operating procedure (SOP) developed for decellularisation of plant or fungal derived vascular bundles and/or microchannels using sodium dodecyl sulfate (SDS). This SOP was used for decellularisation of vascular bundles derived from AS and CL sources. While the following SOP is mainly focused on plant tissue, it is contemplated that fungal tissue may be similarly treated. In certain embodiments, it is contemplated that some adjustments to time-in-solution and/or concentration of SDS and/or CaCl₂ may be made for fungal tissue, for example.

### Solutions

### 0.1% SDS Solution

| | | |
|---|---|---|
| SDS powder (Fisher, catalog# BP166-500) | | 0.5 g |
| Sterile water (Baxter, catalog# JF7624) | *final volume* | 500 ml |
| Prepared in autoclaved bottle (Fisher, catalog# FB-800-500) | | |
| Stored at 20-25°C. | | |
| Use within 7 days. | | |

### 0.1 M CaCl₂ Solution

| | | |
|---|---|---|
| CaCl₂ powder (Acros Organics, catalog# 3496150000) | | 5.549 g |
| Sterile water (Baxter, catalog# JF7624) | *final volume* | 500 ml |
| Prepared in autoclaved bottle (Fisher, catalog# FB-800-500) | | |
| Stored at 20-25°C. | | |
| Use within 7 days. | | |

### Dulbecco's Phosphate-Buffered Saline

| | |
|---|---|
| DPBS (Hyclone, catalog no. 350-000-CL) | bottle 500 ml |
| Stored at 4°C in the dark. | |
| Do not use past expiration date. | |

### Sterilizing Ethanol Solution

| | | |
|---|---|---|
| HistoPrep 95% RA (Fisher, catalog# HC13001GL) | | 368.4 ml |
| Deionized water (Millipore) | *final volume* | 500 ml |
| Prepared in autoclaved bottle (Fisher, catalog# FB-800-500) under biosafety cabinet (BSC). | | |
| Stored at 20-25°C. Use only under BSC. | | |
| Stable indefinitely. | | |

### Procedure

Note 1: The origin and identifying information of all solutions used in the following procedure was recorded in the operator's logbook.

Note 2: The following procedure was preferably used immediately following the completion of SOPs set out in Examples 2 and/or 3 above.

### decellularisation by sodium dodecyl sulfate (SDS) (days 1 to 4):

Day 1:
- Freshly cut and isolated vascular bundles (VBs) were transferred from their DPBS storage solution to 50-ml centrifuge tubes (Nunc). Each tube was loaded with no more than 60 VBs, to ensure proper mixing of the VBs within the SDS solution.
- Each centrifuge tube was filled (to the 50-ml fill line) with 0.1% SDS solution. The tube caps were then tightly screwed into place.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The start time was recorded.

Day 2:
- 20-24h after the start of VBs exposure to SDS solution on an orbital shaker, the shaker was stopped, and the tubes were removed from the shaker.
- The caps of each tube was removed, and the SDS solution was decanted from each tube into a laboratory sink.
- The tubes are filled with unused 0.1% SDS solution, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

Day 3:
- 20-24h after the end of the SDS solution change in Day 2, the shaker was stopped, and the tubes were removed from the shaker.
- The caps of each tube was removed, and the SDS solution was decanted from each tube into a laboratory sink.
- The tubes were filled with unused 0.1% SDS solution, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

Day 4:
- 20-24h after the end of the SDS solution change in Day 3, the shaker was stopped, and the tubes were removed from the shaker.
- The caps of each tube were removed, and the SDS solution was decanted from each tube into a laboratory sink.
- The tubes were filled with unused 0.1% SDS solution, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

### Removal of sodium dodecyl sulfate (SDS) by CaCl₂ salt washes (days 5 to 6)

Day 5:
- 20-24h after the end of the SDS solution change in Day 4, the shaker was stopped, and the tubes were removed from the shaker.
- The caps of each tube were removed, and the SDS solution was decanted from each tube into a laboratory sink.
- The tubes were filled to the 50-ml fill line with DI water.
- The water was decanted from each tube into a laboratory sink.
- The DI-water rinse described in the previous 2 steps was repeated twice more.
- After decanting of the 3^{rd} DI water rinse, each tube was filled to the 50-ml fill line with 0.1 M CaCl₂ solution, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

Day 6:
- 20-24h after the end of the SDS to CaCl₂ solution change in Day 5, the shaker was stopped, and the tubes were removed from the shaker.
- The caps of each tube was removed, and the CaCl₂ solution was decanted from each tube into a laboratory sink.
- Each tube is filled to the 50-ml fill line with unused 0.1 M CaCl₂ solution, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

### Removal of CaCl₂ salt by water washes (days 7 to 8):

Day 7:
- 20-24h after the end of the CaCl₂ solution change in Day 6, the shaker was stopped, and the tubes were removed from the shaker.
- The caps of each tube were removed, and the SDS solution was decanted from each tube into a laboratory sink.
- The tubes were filled to the 50-ml fill line with DI water.
- The water was decanted from each tube into a laboratory sink.
- The DI-water rinse described in the previous 2 steps was repeated twice more.
- After decanting of the 3^{rd} DI water rinse, each tube was filled to the 50-ml fill line with water, and their caps are screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

Day 8:
- 20-24h after the end of the CaCl₂ to water solution change in Day 7, the shaker was stopped, and the tubes were removed from the shaker.
- The caps of each tube were removed, and the water was decanted from each tube into a laboratory sink.
- Each tube was filled to the 50-ml fill line with unused water, and their caps were screwed back on.
- The tubes were secured using tape to the plate of an orbital shaker (Corning LSE).
- The orbital shaker was set to rotate at 120 RPM with no time limit. The shaker was observed for at least 1 minute to ensure that the tubes were secured and not at risk of becoming loose.
- The time was recorded.

### Sterilization (day 9):

Day 9:
- 20-24h after the end of the water change in Day 8, the shaker was stopped, and the tubes were removed from the shaker. The time was recorded.
- The caps of each tube were removed, and the water was decanted from each tube into a laboratory sink. The tube caps were then screwed back on.
- The tubes were sprayed down with a 70% ethanol solution and were brought inside of a biosafety cabinet (BSC).
- Each tube was filled to the 30-ml fill-line with the sterilizing ethanol solution, and the tube caps were screwed back on. The tubes were positioned upright on a tube-rack, such that all decellularised VBs were immersed in the ethanol solution.
- The tubes were left undisturbed for 15 minutes.
- After 15 minutes, the tubes were inverted and were left undisturbed for a further 15 minutes.
- After 15 minutes, the tubes were repositioned upright, and the caps were removed.
- The ethanol solution in each tube was removed by a 10-ml serological pipette. The ethanol solution was collected in a glass bottle for disposal in a laboratory sink.
- Each tube was filled to the 30-ml fill-line with sterile DPBS using a 10-ml serological pipette, followed by a gentle swirling.
- The DPBS was aspirated out of each tube by sterile Pasteur pipette.
- The previous 2 steps were repeated twice more for a total of 3 sterile DPBS rinses.
- After the third DPBS rinse volume was aspirated out of each tube, each tube was filled with 30 ml of sterile DPBS, and the tube caps were screwed tightly back into place.
- Each tube was labeled with the following additional information: production code, sterilization date, liquid solution content, sterility status.
- The tubes were placed in the dark at 4C.

### EXAMPLE 5 - Fabrication of High-Density Microchannel (HDMC) Bundles from Lignocellulosic Materials (i.e. AS &CL) using Fibrin Glue

Described below is an example of a standard operating procedure (SOP) developed for fabrication of high-density microchannel (HDMC) bundles from lignocellulosic materials (i.e. AS and CL) using Fibrin glue. This SOP was used for bundling and gluing of vascular bundles derived from AS and CL sources.

### Safety Statement(s)

Always guard against needlestick injuries when handling hypodermic needles. Fibrin glue and fibrin glue-derived products (including protein vials), were treated as Biohazard Level 2 materials. Consult all appropriate MSDS. Wear appropriate personal protection equipment such as vinyl gloves, lab coat, and goggles, and perform all work under a laminar flow hood.

### Solutions & Reagents

### Dulbecco's Phosphate-Buffered Saline

| | |
|---|---|
| DPBS (Hyclone, catalog no. 350-000-CL) | bottle 500 ml |
| Stored at 4°C in the dark. | |
| Do not use past expiration date. | |

### TISSEEL Fibrin Sealant Kit (Baxter Healthcare, catalog# 1503152)

| | |
|---|---|
| Aprotinin (synthetic) solution | 1 ml at 3000 KIU/ml |
| Sealer protein concentrate (human) | Reconstituted to 96 - 125 mg/ml in aprotinin solution |
| Calcium chloride solution | 1 ml at 25 micromolar/ml |
| Thrombin (human) | Reconstituted to 500 IU/ml in calcium chloride solution |
| Components stored between 2 - 25°C. | |
| Solutions prepared in dual-syringe system as per manufacturer's instructions (see procedure below). | |

### Sterilizing Ethanol Solution

| | | |
|---|---|---|
| HistoPrep 95% RA (Fisher, catalog# HC13001GL) | | 368.4 ml |
| Deionized water (Millipore) | *final volume* | 500 ml |
| Prepared in autoclaved bottle (Fisher, catalog# FB-800-500) under biosafety cabinet (BSC). | | |
| Stored at 20-25°C. Used only under BSC. | | |
| Stable indefinitely. | | |

### Procedure - Glue Preparation

### TISSEEL and DUPLOJECT Preparation

Note: TISSEEL preparation follows the manufacturer's instructions (Baxter document ID 0716820). The following procedure is an equivalent restatement of these procedures suitable for local laboratory conditions.

All four vials in the TISSEEL kit were pre-warmed in wells of a FIBRINOTHERM (Baxter) device. The device warms vial contents to 37C and maintains this temperature. A signal light indicated when warming was complete. Warming may take up to 5 minutes if the vials were refrigerated.

After pre-warming was complete, all four vials were transferred to the biosafety cabinet (BSC).

The plastic flip-off plastic caps of the aprotinin and sealer protein vials were removed without touching the rubber vial stopper. The rubber stoppers are disinfected by a Kimwipe laden with Accel TB disinfecting solution. The rubber stoppers were allowed to dry.

Using the blue-scaled syringe and a needle tip in the kit, the aprotinin solution was transferred to the sealer protein vial.

The sealer protein vial was transferred to the FIBRINOTHERM device's stirring well (the vial already contains a small magnetic stir bar). The vial contents were stirred for 10 minutes. Stirring was complete when the vial solution was clear and free of bubbles and particles. The vial was left in a 37 C warming well until the DUPLOJECT dual-syringe system was assembled.

The plastic flip-off plastic caps of the calcium chloride and thrombin vials were removed without touching the rubber vial stopper. The rubber stoppers were disinfected by a Kimwipe laden with Accel TB disinfecting solution. The rubber stoppers were allowed to dry.

Using the black-scaled syringe and a needle tip in the kit, the calcium chloride solution was transferred to the thrombin vial.

The thrombin vial was gently swirled until clear, then transferred to the FIBRINOTHERM device for re-warming to 37C.

The two solution-containing vials (originally the sealer protein and thrombin vials) were transferred from the FIBRINOTHERM device to the BSC.

The rubber stoppers of both vials were wiped with a Kimwipe laden with Accel TB, then allowed to dry.

Using the second blue-scaled syringe in the kit and a needle tip, the solution from the sealer protein vial was drawn into the syringe.

The needle tip was removed from the syringe (discard into sharps container), and the syringe was clipped into the red dual-syringe-holding DUPLOJECT assembly.

Using the second black-scaled syringe in the kit and a needle tip, the solution from the thrombin vial was drawn into the syringe.

The needle tip was removed from the syringe (discard into sharps container), and the syringe was clipped into the red DUPLOJECT assembly.

The plastic joining piece was connected to the ends of the clipped syringes.

An application cannula was connected to the end of the joining piece.

The completed DUPLOJECT assembly was positioned upright against a stabilizing surface (such as a tube rack) until fibrin glue was required. Fibrin glue was preferably used within the following 4 hours.

### Bonding of Vascular Bundles (VBs)

Note: all tools described in this subsection were autoclaved for sterility and were only removed from their autoclave once inside the BSC.

A tube containing the correct type (AS or CL) and amount of decellularised & sterilized vascular bundles (20 VBs per HDMC bundle) was selected from storage and brought inside the BSC, along with:
- Microtome blade
- Custom silicone molds (1 mold per HDMC bundle)
- Tweezers
- 24-well plates (in sterile packaging; minimum 2 plates were used if fabricating HDMC from a single type of VB)

The solid contents of the VB tube were transferred by sterile tweezers to the interior surface of a 24-well plate lid. 5 ml of sterile DPBS was added to this interior surface to prevent drying out of the VBs.

Up to two silicone molds were removed from their autoclave pouches using sterile tweezers and were positioned within the interior surface of a second 24-well plate lid such that their interior channel was open and facing upwards (towards the operator). No more than 4 mold halves (totaling 2 HDMCs) were manipulated within a single plate lid.

Using a pair of sterile tweezers, 10 VBs were packed into each mold half's channel.

The packed VBs were removed from each mold half as a bundle, and the bundle from each half was placed directly below the mold.
3 droplets of fibrin glue were deposited along the length of each mold half's interior channel using the DUPLOJECT system.

Immediately following glue deposition, the packaged VB bundles were repositioned into their mold channels. Gentle pressure was applied by tweezer tip to ensure a tight packing of VBs within the glue-loaded mold channels.

The fibrin glue was allowed to set for 10 minutes. During this time, pictures of the molds were taken.

After 10 minutes, the applicator tip on the DUPLOJECT system was exchanged for a new, unclogged tip.

3 droplets of fibrin glue were deposited along the length of each packed VB bundle.

Immediately following glue deposition, the mold halves (containing 10 packed VBs each) were pressed together by tweezer manipulation. The molds were oriented such that the halves remained together without tweezer contact required.

The fibrin glue was allowed to set for 10 minutes. During this time, pictures of the molds were taken.

After 10 minutes, the ends of the VBs protruding from the silicone mold were sliced off by microtome blade and discarded.

The mold halves were gently separated by tweezer manipulation. The HDMC bundle product may take gentle prying from the one of the mold halves to be freed. Pictures of the freed HDMC were taken.

Note: HDMC bundle may be optionally sterilized at this stage by 30 min immersion in a 30 ml of a 70% ethanol solution followed by 3x20 ml rinses in sterile DPBS.

The HDMC bundle products were placed into 50-ml centrifuge tubes (Nunc) containing 10 ml of sterile DPBS (one tube per HDMC).

The HDMC tubes were stored in the dark at 4C.

### EXAMPLE 6 - Examples Testing Different Glues

Described below are examples of fabricating glued high-density microchannel (HDMC) bundles testing a variety of different types of glues in a molding-type process.

### AS Gluing with PEG-Based Synthetic Glue

### Methods:

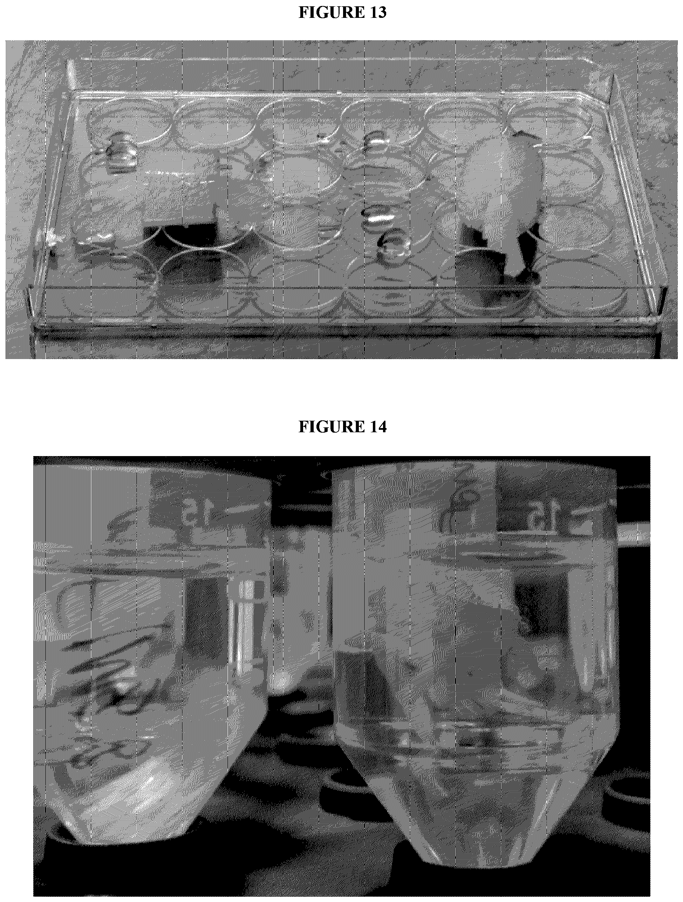

decellularised AS vascular bundles (VBs) produced by SOP of Example 4 (decellularisation) were used.
Molding: VBs were packed into two halves of a biomedical-grade silicone (Dow Corning Silastic MDX4-4210) mold. 10 VBs per mold half; 20 VBs per HDMC.
Glue: Coseal glue from Baxter was used. 2 droplets were deposited into each mold half's interior, then VBs were packed into channels. After 5 minutes, 2 more glue droplets deposited onto packed VBs of each mold half before mold halves were pressed together (Fig 13). 10 min settling time was allowed before attempt at mold half separation.
Sterile assembly within BSC was performed.

### Results:

Coseal glue produced gel which was immediately adhesive after deposition onto mold halves. Volume of expansion, however, was severe, with expansion estimated at about 400% by volume. Due to the mold being used and the particular setup of this experiment which was not designed to accommodate for expansion, the glue expansion was detrimental and resulted in separation of the mold halves, and therefore shape of the HDMC cylinder was not maintained. It was also found that the Coseal glue was more adhesive toward the silicone mold than fibrin glue was, and therefore splitting of the HDMC bundle in half was encountered if the glue was not carefully separated from the mold halves. Resulting HDMC bundles from this test were loosely bonded, and did not have a cylindrical shape once removed from the molds (see Figure 14).

While difficulties were encountered with PEG-based glue in this experimental system, it is contemplated that adaptations may be made to accommodate for the PEG-based glue expansion and stickiness, for example by using a bigger mold and/or foregoing molding; using lower microchannel density to allow for expansion; and/or using a mold of a different material which has less adherence with the PEG-based glue, for example.

### CL Gluing with PEG-Based Synthetic Glue

### Methods:

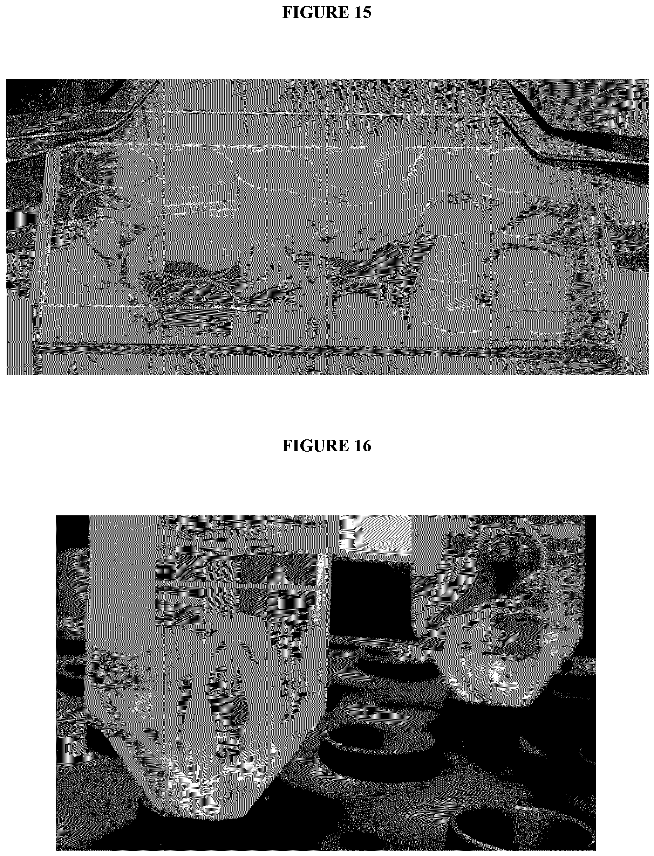

decellularised CL vascular bundles (VBs) were prepared by the SOP (decellularisation) of Example 4.
Molding: VBs were packed into two halves of a biomedical-grade silicone (Dow Corning Silastic MDX4-4210) mold. 10 VBs per mold half; 20 VBs per HDMC.
Glue: PEG-based Coseal glue from Baxter was used. 2 droplets were deposited into each mold half's interior, then VBs were packed into channels. After 5 minutes, 2 more glue droplets were deposited onto packed VBs of each mold half before mold halves were pressed together (Fig 15). 10 min settling time was allowed before attempting mold half separation.
Sterile assembly was performed within BSC.

### Results:

Coseal glue produced gel which was immediately adhesive after deposition onto mold halves. Volume of expansion, however, was severe, with expansion estimated at about 400% by volume. Due to the mold being used and the particular setup of this experiment which was not designed to accommodate for expansion, the glue expansion was detrimental and resulted in separation of the mold halves, and therefore the shape of the HDMC cylinder was not maintained. It was also found that the Coseal glue was more adhesive toward the silicone mold than fibrin glue was, and therefore splitting of the HDMC bundle in two was encountered if the glue was not carefully separated from the mold halves as each half may remain glued to its silicone mold half. Resulting HDMC bundles from this test were loosely bonded, and did not have a cylindrical shape once removed from the molds (see Figure 16, in which partial debonding of individual VBs is visible). While difficulties were encountered with PEG-based glue in this experimental system, it is contemplated that adaptations may be made to accommodate for the PEG-based glue expansion and stickiness, for example by using a bigger mold and/or foregoing molding; using lower microchannel density to allow for expansion; and/or using a mold of a different material which has less adherence with the PEG-based glue, for example.

### AS Gluing with Cyanoacrylate Glue (Krazy Glue)

### Methods:

decellularised AS vascular bundles (channels) were prepared according to SOP of Example 4 (decellularisation) as above.
Molding: Channels were packed into two halves of a plastic straw (5 mm internal diameter).
Gluing: Cyanoacrylate glue (Krazy Glue) was used. Droplets were deposited into packed channels before mold halves were pressed together.
Methods were non-sterile, by manual assembly.

### Results:

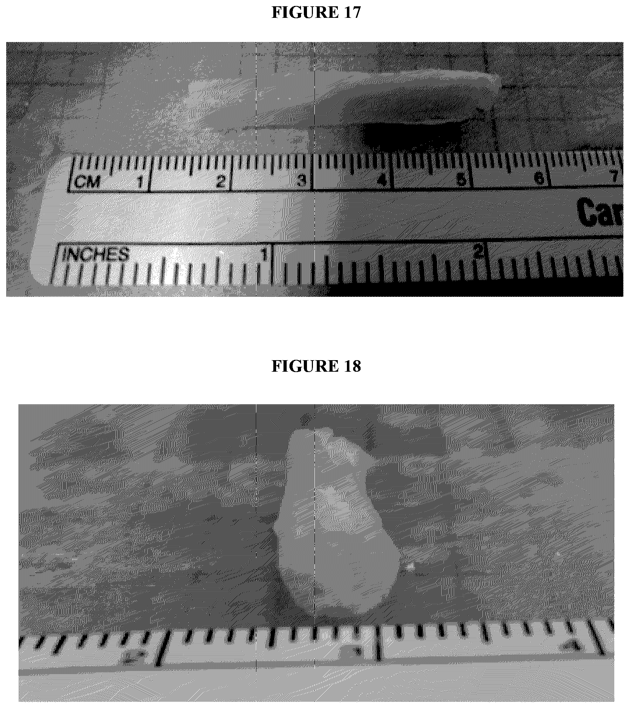

Bundles were successfully assembly. Maximum packing density under these conditions was about 34 channels in 5 mm diameter, which compares favorably to the 5 - 9 channels found in a native AS piece of comparable diameter (see Figures 17-18).

### AS Gluing with Biodegradable Fibrin Sealant (TISSEEL)

### Methods:

decellularised AS vascular bundles (channels) were prepared by SOP (decellularisation) of Example 4 above.
Molding: Channels were packed into two halves of a custom-made silicone (Sylgard 184) mold (4 mm internal diameter). Silicone material did not adhere to fibrin sealant, facilitating removal of glued AS channels from the mold.
Molds were autoclaved in autoclaving pouches before use.
Gluing: Glue was Fibrin Sealant. Droplets were applied using Baxter DUPLOJECT system for TISSEEL application into packed channels before mold halves were pressed together.
Sterile assembly by autoclaved tweezer manipulation within laminar flow hood was performed. Assembly was completed within the interior side of sterile 24-well plate lid.

### Results:

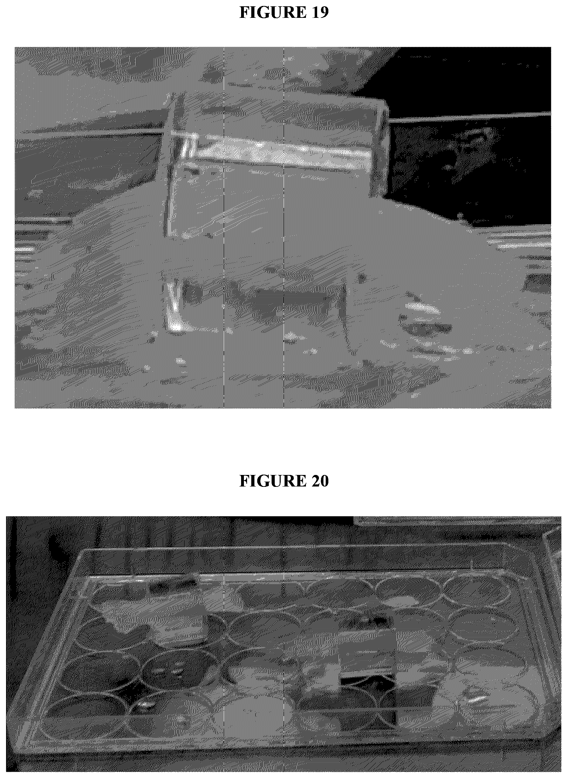

Glued bundles were successfully assembled. Maximum packing density under the conditions used was about 20 channels in 4 mm diameter. This was a slight reduction in density versus the cyanoacrylate glue. Figure 19 shows AS channels within joined silicone mold halves during the curing period.

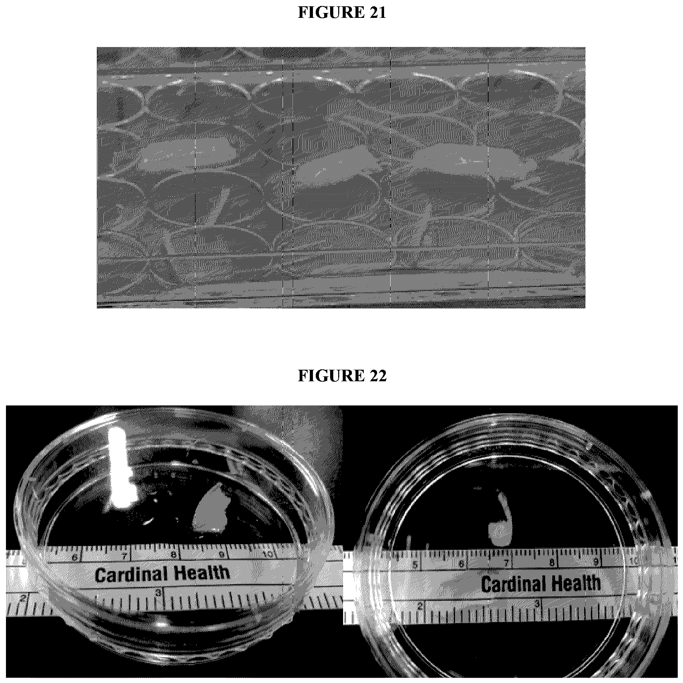

A repeated experiment was performed to that above, with slight modification. Channel modification was reduced to 18 channels per bundle, and average individual channel thickness was slightly increased. Figure 20 shows a view of AS channels within silicone molds during glue curing step. Figure 21 shows view of fibrin-glued AS channels after removal from silicone molds. Sterilization was performed by 30-min immersion of glued bundle samples in 70% ethanol followed by 3 washes in sterile DBPS. Sample was stored in sterile DPBS at 4C prior to *in vitro* tests.

*In vitro* biocompatibility testing was then performed. GPF-3T3 fibroblasts were cultured on glued bundle samples for 1 week, then fixed in 4% paraformaldehyde. Cells initially deposited on exposed channel openings. Confocal laser-scanning microscopy (CLSM) of fixed samples to determine presence or absence of cells was performed. Figure 22 (left panel) shows a perspective view of AS sample after 1 week of GFP-3T3 cell culture followed by fixing in 4% paraformaldehyde. Right panel shows top view of the same sample.

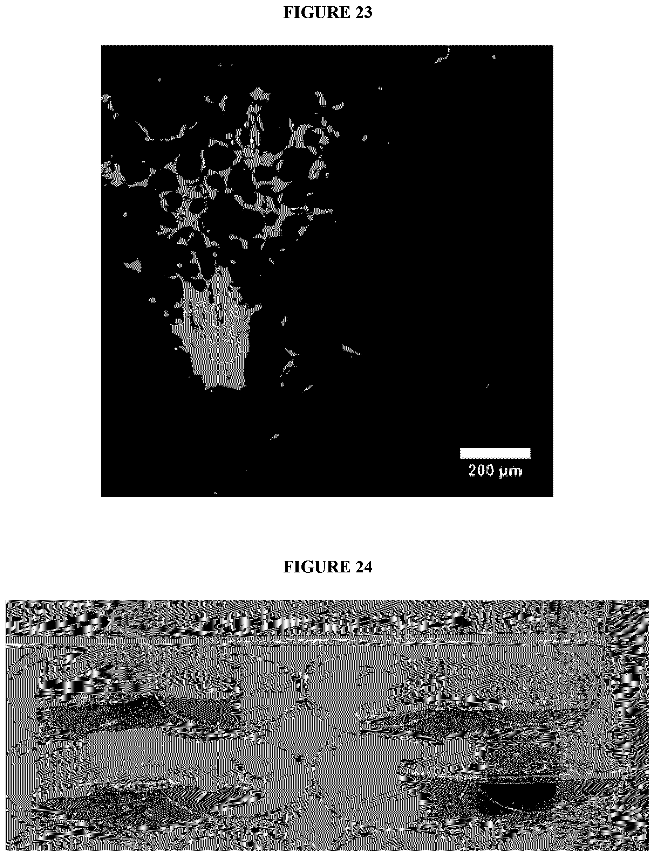

Figure 23 shows CLSM imagery of AS HDMC bundle after 1 week of GFP-3T3 cell culture followed by fixing in 4% paraformaldehyde. Cells (green) were visible in and around xylem microchannels (blue).

Results and discussion: Multiple glued AS HDMC bundle samples were successfully produced. After 1 week of culture, cells were visualized within channels and also on peripheral material (glue, channel exteriors). Cell visualization was possible due to presence of GFP protein; cellulose scaffold of AS was visible by autofluorescence of xylem microchannels.

These experimental were also repeated, with some modification: Mold halves were made from food-grade silicone drinking straws. Mold halves were autoclaved in autoclaving pouches prior to use, and contrasts with Sylgard 184 silicone which is not food-grade. It was noted that manipulation of the smaller mold halves by autoclaved tweezers was more difficult than manipulation of larger custom-made Sylgard 184 mold halves, for example. Same sterilization procedure as noted above was used, and resulting HDMC bundles were intended for use in implantation studies in rats (see Example 8 below).

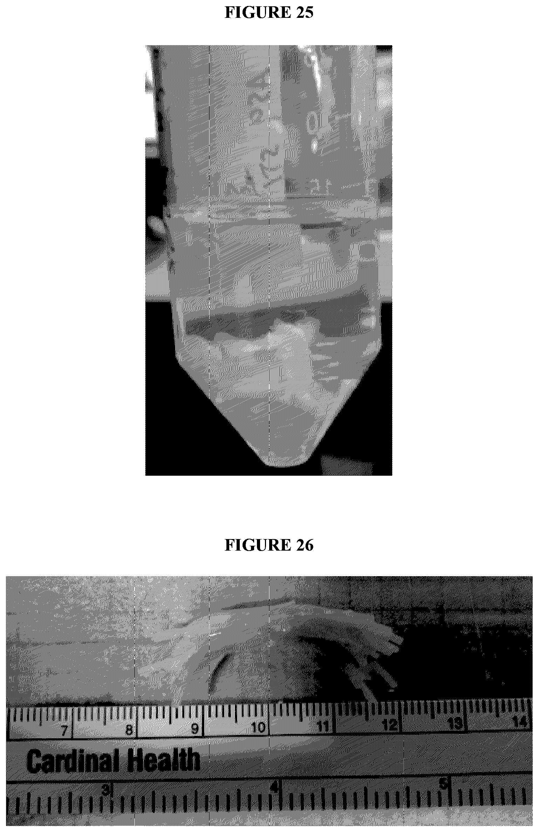

Multiple glued AS bundle samples were successfully produced. 7 samples were made, each about 5mm length, 5 mm diameter. Some of the sample become unglued/disjoined during storage at 4C, potentially due to insufficient fibrin glue in some bundles. Figure 24 shows a view of AS channels within food-grade silicone molds during glue curing step. Figure 25 shows glued AS channel samples stored in sterile DPBS after cutting to 5 mm length.

Due to the loss of some samples in storage, this procedure was repeated, but using slightly more glue to hold channels together within the silicone straw molds.

Multiple glued AS bundle samples were successfully prepared. 4 samples were produced, each 5 mm length, 5 mm diameter, and no loss of samples in storage was observed. Subcutaneous implantation results are shown in Example 8 below.

### CL Gluing ith Cyanoacrylate Glue (Super glue/"Krazy Glue")

### Methods:

decellularised CL vascular bundles (channels) were prepared by SOP (decellularisation) of Example 4.
Molding: Channels were packed into two halves of a plastic straw (5 mm internal diameter).
Gluing: Cyanoacrylate glue ("Krazy glue") was used. Droplets were deposited into packed channels before mold halves were pressed together.
Non-sterile, manual assembly was performed.

### Results:

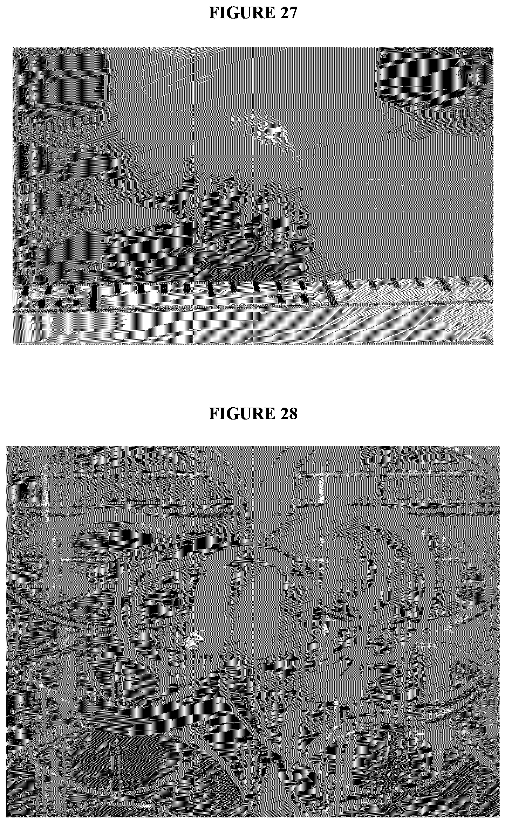

Successful assembly of glued bundles was achieved. Maximum packing density under these conditions was about 24 channels in 5 mm diameter. Figure 26 shows a side view of CL channel bundle glued with cyanoacrylate glue. Figure 27 shows a radial view of CL channel bundle glued with cyanoacrylate glue, in which individual channels are faintly visible.

### CL Gluing with Biodegradable Fibrin Sealant (TISSEEL)

### Methods:

decellularised CL vascular bundles (channels) were prepared by SOP (decellularisation) of Example 4.
Molding: Channels were packed into two halves of a custom-made silicone (Sylgard 184) mold (4 mm internal diameter). Silicone material did not adhere to fibrin sealant, facilitating removal of glued CL channels from the mold.
Molds were autoclaved in autoclaving pouches before use.
Gluing: Biodegradable Fibrin sealant was used as glue. Droplets were applied using Baxter DUPLOJECT system for TISSEEL application into packed channels before mold halves were pressed together.
Sterile assembly was performed by autoclaved tweezer manipulation within laminar flow hood. Assembly was completed within the interior side of sterile 24-well plate lid.

### Results:

Glued bundle was successfully assembled. Maximum packing density under the conditions tested was about 20 channels in 4 mm diameter. Figure 28 shows CL channels joined together in silicone mold halves during curing period.

This experiment was repeated to produce more bundle samples for further testing. The same fabrication procedure was used, with the modification of channel packing density reduced to 18 channels per bundle to account for variability (increase) in average size of CL channel thickness. Sterilization was performed by 30-min immersion of glued bundle samples in 70% ethanol followed by 3 washes in sterile DBPS. Sample was stored in sterile DPBS at 4 C prior to *in vitro* tests.

*In vitro* biocompatibility testing was performed as follows: GFP-3T3 fibroblasts were cultured on glued bundle samples for 1 week, then fixed in 4% paraformaldehyde. Cells were initially deposited on exposed channel openings. Confocal laser-scanning microscopy (CLSM) of fixed samples was used to determine the presence or absence of cells.

### Results:

Multiple glued CL bundle samples were successfully produced. After 1 week of culture, cells were visualized within channels and also on peripheral material (glue, channel exteriors). Cell visualization was possible due to presence of GFP protein; cellulose scaffold of CL was visible by autofluorescence of xylem microchannels.

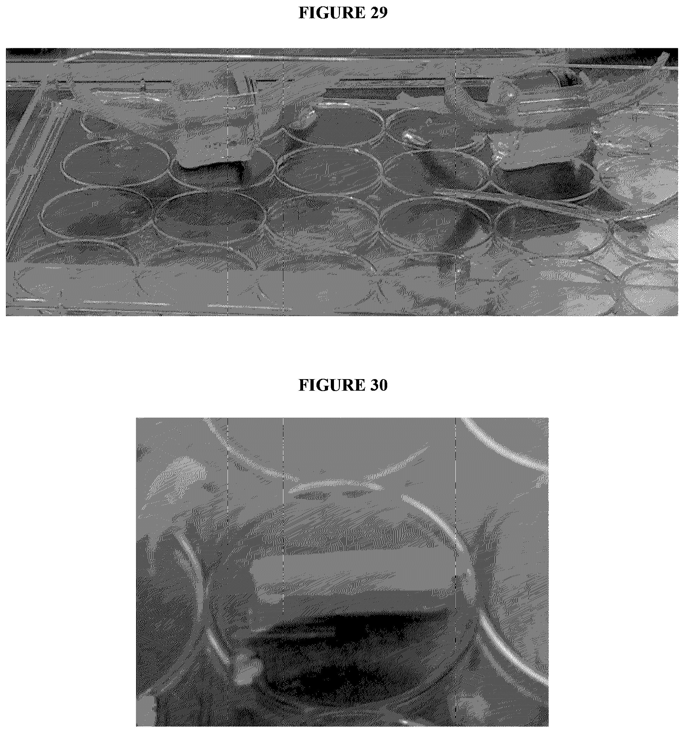

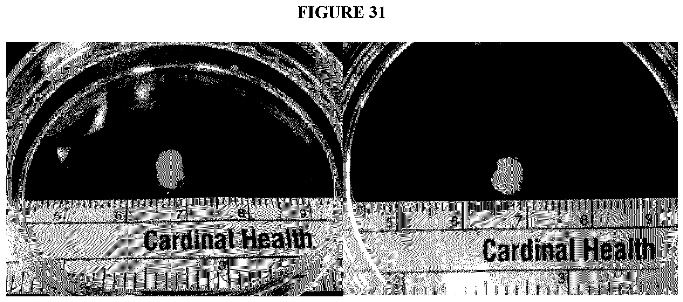

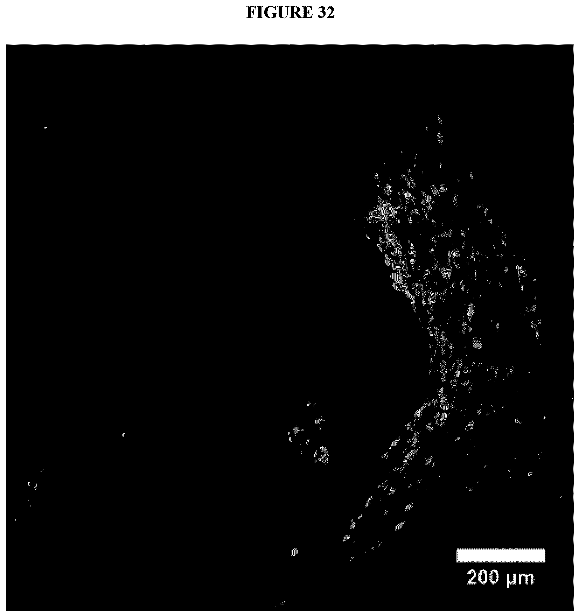

Figure 29 shows a view of CL channels within silicone molds during glue curing step. Figure 30 shows view of fibrin-glued CL channels after removal from silicone mold. Figure 31 shows a perspective view of CL bundle sample after 1 week of GFP-3T3 cell culture followed by fixing in 4% paraformaldehyde (left panel) and a top view of the same sample (right panel). Figure 32 shows CLSM imagery of CL sample after 1 week of GFP-3T3 cell culture followed by fixing in 4% paraformaldehyde. Cells (green) were visible in and around xylem microchannels (blue).

This experiment was repeated, but with a change to using mold halves made from food-grade silicone drinking straws so as to study using medical or food-grade silicone molds and provide samples for subcutaneous implantation in rats (see Example 8). Mold halves were autoclaved in pouches prior to use. It was noted that manipulation of smaller mold halves by autoclaved tweezers was more difficult than manipulation of larger custom-made Sylgard 184 mold halves. Same sterilization procedure as above was used, and samples were intended for subcutaneous implantation in rats (see Example 8).

Results: production of multiple glued CL bundle samples was successfully achieved. 9 samples were produced, each 5 mm length, 5 mm diameter. 3 of the 9 samples eventually became unglued/disjoined during storage at 4 C, likely due to insufficient fibrin gluing of these 3 bundles.

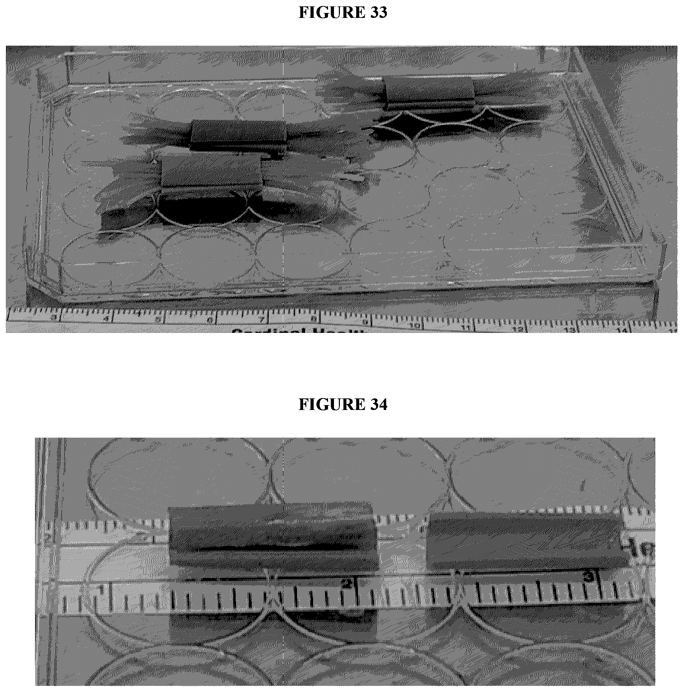

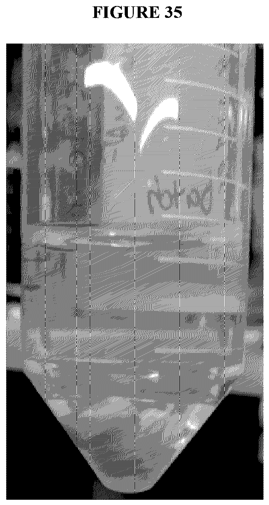

Figure 33 shows a view of CL channels within food-grade silicone molds during glue curing step. Figure 34 shows a view of glued CL channel bundle after removal of top silicone mold half. Figure 35 shows a view of glued CL channel samples (stored in sterile DPBS) after cutting to 5 mm length.

### EXAMPLE 7 - Guiding Properties and Directionality of HDMC Bundles

Experiments were performed to further investigate the ability of HDMC bundles as described herein to guide cellular directionality. As described herein, there are many examples, both therapeutic and non-therapeutic, where a scaffold biomaterial capable of imparting directionality and/or guiding of cellular growth/invasion/proliferation is desirable. While many scaffolds in the field are unable to impart directionality, the present inventors have now developed scaffold biomaterials comprising high-density microchannel bundles as described herein, in which decellularised microchannels are arranged substantially parallel to each other within the bundle, which may impart directionality to cells growing thereon and/or therein.

A description of one such study is provided below.

### Methods:

(HDMC fabrication): HDMC were fabricated using decellularised vascular bundles (VBs) of AS and CL source material generally following the SOPs of Examples 3, 4, and 5. Briefly, isolated AS and CL VBs were decellularised as described herein, then shaped and glued (Baxter Healthcare, TISSEEL fibrin sealant, catalog no. 1503152) into HDMC/AS and HDMC/CL, respectively, by a custom-made silicone (Dow, Sylgard 184) mold. The VB density was 18 AS or CL VBs per HDMC. HDMC were sterilized by a 30-min immersion in a 70% ethanol solution prior to 3x washes with sterile Dulbecco's Phosphate-Buffered Saline (DPBS; Hyclone, catalog no. 350-000-CL) and storage in DPBS at 4 C.

(Cell culture): GFP-3T3 fibroblasts (producing green fluorescent protein) were cultured with HDMC/AS and HDMC/CL for a 1 week (with 2 equally-spaced media changes) period in Dulbecco's Modified Eagle's Medium (DMEM; Hyclone) supplemented with 10% fetal bovine serum (FBS; Hyclone) and 1% penicillin-streptomycin (P/S). Sections of HDMC/AS and HDMC/CL approximately 5 mm long and 5 mm diameter were deposited in the wells of a 24-well plate, and approximately 50 000 GFP-3T3 cells were deposited (in a small media droplet) directly onto open-channel faces of the HDMC. 4 hours after initial cell deposition by small droplet (to allow for cell infiltration and adherence onto HDMC), HDMC-containing wells received 1 ml of media, and 2 media changes were effectuated before the end of the 1-week culture period. The samples were incubated in standard cell culture conditions in a humidified 37 C, 5% CO₂ incubator.

(Fixing): At the end of the 1-week cell culture period, media was removed from the HDMC wells. The wells were washed 3x with DPBS, then filled 1 ml/well with a 4% paraformaldehyde (PFA) fixing solution for 10 minutes shielded from light. At the end of the 10-minute fixation period, the PFA solution was removed and the wells were washed 3x with DPBS, before being filled 1/ml with DPBS and stored in 4 C and shielded from light. (Microscopy): HDMC samples were removed from their 24-well plate, DPBS-containing wells and placed into 5-mm plastic cell culture dishes. The open-channel ends (those onto which cells were deposited) were examined by laser-scanning confocal microscopy. The surface was scanned using the 10X objective lens for areas where cells had adhered in and around xylem channel openings. Both single optical sections and vertically-stacked sections ("Z-stacks"; 63 sections spaced 2.8 microns apart) were captured of areas of interest. Optical sections were visualized using Fiji/ImageJ. 3D visualizations of the Z-stacks were rendered by the 3D Viewer Plugin, a standard package in Fiji.

### Results:

Xylem channel structures in both HDMC/AS and HDMC/CL were directly visible by autofluorescence (by 405 nm excitation laser), and the presence of cells was indicated by green fluorescent signal (by 488 nm excitation laser) due to the presence of GFP in cells. Cells were visualized in and around xylem channels in both HDMC/AS (Figures 36A and 36B) and HDMC/CL (Figures 37A and 37B). A 3D reconstruction of the optical slices revealed cellular infiltration in and around the xylem channels (Figures 36C and 37C), directionally towards the interior of the HDMC (Figures 36D and 37D).

### Discussion:

This cell culture study of the biocompatibility of HDMC/AS and HDMC/CL indicates that fibroblasts may adhere to, and survive on, HDMC structures, supporting that HDMC are a biocompatible material. Fibroblasts were visualized both in and around xylem microchannels, suggesting that the channel spaces of AS and CL source materials were appropriately-sized for cellular infiltration and growth. Notably, 3D renderings of optical sections demonstrated evidence for cell masses proliferating deeper into the HDMC material by microchannel-mediated guidance. This supports that the HDMC material may provide the substrate for directionally-controlled proliferation of cells both inside and outside of lignocellulosic microchannels. HDMC may therefore provide a scaffold engineered to promote cellular reconnections across formerly severed links, for example.

Figure 36A) shows confocal laser-scanning fluorescence micrograph (Z-stack projection) of xylem channels and fibroblast cells within HDMC/AS. Channels are visualized in red (autofluorescence; false color), GFP-3T3 fibroblasts are visualized in green (green fluorescent protein; false color). Figure 36B) shows close-up view of red-outlined multi-channel in panel A. Figure 36C) shows 3D-volume rendering (Fiji/ImageJ; 3D Viewer Plugin) of green fluorescent protein and xylem autofluorescence signals from the perspective of panel B. Figure 36D) shows a rotated view of 3D rendering, displaying directionality of cells along the length of the channels.

Figure 37A) shows confocal laser-scanning fluorescence micrograph (Z-stack projection) of xylem channels and fibroblast cells within HDMC/CL. Channels are visualized in red (autofluorescence; false color), GFP-3T3 fibroblasts are visualized in green (green fluorescent protein; false color). White arrows indicate the presence of cells outside of the channels. Blue arrows indicate the presence of cells inside the channels. Figure 37B) shows a close-up view of red-outlined single channel in panel A. Figure 37C) shows a 3D-volume rendering (Fiji/ImageJ; 3D Viewer Plugin) of green fluorescent protein signal from the perspective of panel B. Figure 37D) shows a rotated view of 3D rendering, displaying directionality of cells along the length of the channel.

Accordingly, this testing indicates that scaffold biomaterials comprising an HDMC bundle with decellularised microchannels from either of AS and CL plant tissue sources may be used to provide a guidance effect on cells. In these results, cells were located both inside and outside of the xylem channels of the HDMC bundles, and they were directional (i.e. guided by the directionality of the channels).

### EXAMPLE 8 - In Vivo Biocompatibility of Scaffold Biomaterials and HDMC Bundles

Studies were performed *in vivo* to further study the biocompatibility of scaffold biomaterials and HDMC bundles as described herein.

### Methods:

Surgical procedures: Rats were given subcutaneous injections of saline 0.9% and buprenorphine (0.05mg/kg) prior to surgical implantation. Rats were anesthetized using Isoflurane. Eyes were protected by application of ophthalmic liquid gel. The rats were shaved from hips to shoulder, on their right side. The skin was washed and sterilized with aseptic solutions. Three 1-2cm incisions were performed (one near in the lower, middle and upper back), parallel anterior to the spinal column. Incisions were made through the epidermis, dermis, and subcutaneous fat layers to the underlying muscle. HDMC bundle implants (CL and AS) were implanted into each incision (1 implant per incision). The incisions were sutured and transdermal bupivacaine 2% was applied to the suture sites. Additionally, buprenorphine was administered subcutaneously 4-6 hours following the first injection. Specimens were collected after 4 and 8 weeks.

Histology: Collected samples were fixed in 10% formalin and placed in 70% ethanol before paraffin embedding. Serial sections were cut and stained with either hematoxylin-eosin (H&E) or Masson's trichrome.

### Results and discussion:

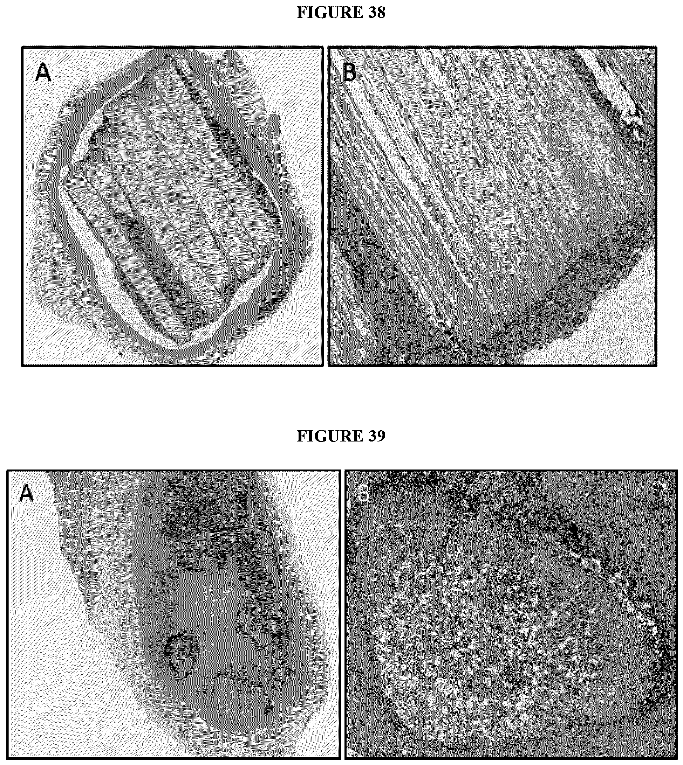

Figures 38 and 39 show histological stains (hematoxylin and eosin; H&E) of longitudinal and transverse cuts, respectively, of retrieved HDMC/CL (celery-derived) material from an implantation site. In all cases, panel B is a close-up of a region in panel A. The CL-based microchannels (visible in Figure 39A) remain bundled together and clearly-defined.

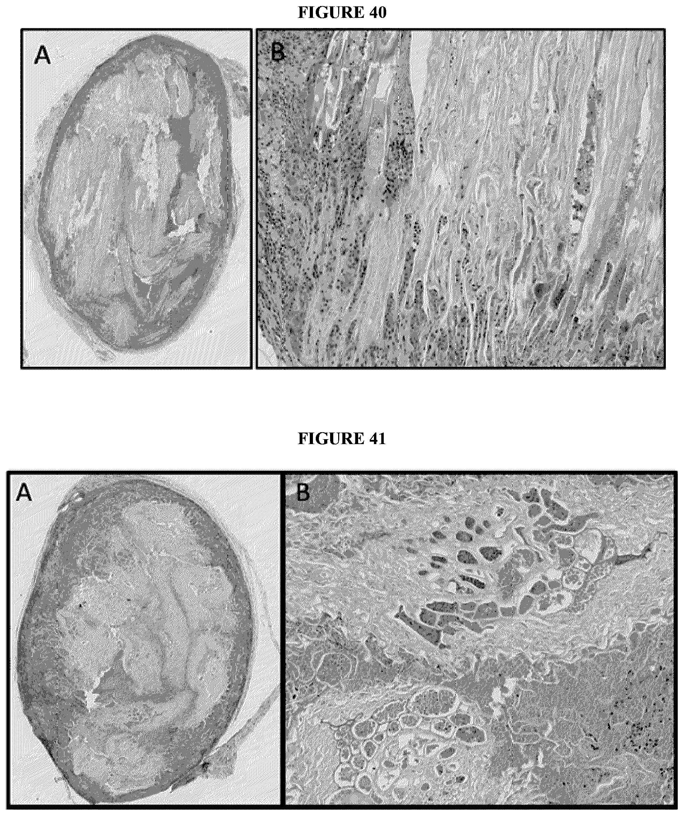

Figures 40 and 41 show histological stains (H&E) of longitudinal and transverse cuts, respectively, of retrieved HDMC/AS (asparagus-derived) material from an implantation site. The AS-based microchannels (visible in Figure 40A) remain bundled together, but the boundaries between the microchannels were less evident than in HDMC/CL (Figure 39A) under the conditions tested.

A cursory direct comparison of HDMC/CL (Figures 38 and 39) and HDMC/AS (Figures 40 and 41) histological stains suggests that the HDMC/AS material may be somewhat more biocompatible under the conditions tested.

### EXAMPLE 9 - Methods and Procedures for Liquid-based Extraction of Intact Vascular Bundle Microchannels from Native or Decellularised Vascular Plants for HDMC Production

Studies were performed to determine alternative methods for isolating VB microchannels from native or decellularised vascular plants for HDMC production. Employed herein are a variety of different chemical treatments such as osmotic shock, temperature changes, mechanical agitation, and maceration in acid and base solutions. Depending on the goal of the procedure, optimal solution, concentration and times are determined. A guiding principle of these steps is to deconstruct plant tissues into underlying intact structures which can then be utilized alone, or in combination with other plant materials.

### Methods:

### Preparation of buffers and solutions

1. For NaCl, 50mL solutions of 0M, 0.5M, 1M, 2M and 3M are prepared.
2. For LiCl, 50mL solutions of 0M, 0.5M, 1M, 2M and 3M are prepared.
3. For acid/peroxide, 50mL solutions of 3:1, 2:1, 1:1, 1:2, and 3:1 ratios of glacial acetic acid to 30% hydrogen peroxide are prepared.
4. For base, 50mL solutions of 0M, 0.5M, 1M, 2M and 3M of NaOH are prepared.

### Preparation of Plant Tissues

5. Prepare strips of tissue by slicing off any skin or removing ends. For fruits like apples or pear, peel skin and slice into equal pieces. For celery, asparagus and the like, cut off white ends and tops.
6. Cut into 3cm (or appropriate size) pieces then slice on a mandoline slicer.
7. For plants such as asparagus, celery and the like, keep slices containing visible vascular bundles of xylem and phloem. For fruits such as apple, pear and the like, use all of the inner pulp staying away from the core.
8. At this point, the native tissues can be treated with the conditions described below. Alternatively, the native tissues can also first be decellularised according to the SDS-based methods in our previous work and patents prior to the additional treatments described below.
9. Remaining strips are placed in a beaker filled with the desired solution for microstructure isolation.

### Extraction Procedure

### Materials Required:

a. 50mL tubes for each solution type and/or concentration
b. 200mL beakers (use exact same beakers for entirety of procedure)
c. hot plate
d. thermometer
e. 4x lids from 6 well plate (or any clear rectangular plate lid for images)
f. 1x 1L beaker filled with distilled water (I recommend having another beaker on the side to pour the 200mL of distilled water into to stop the reaction quickly).
g. forceps
h. timer

Procedure: This procedure can be applied to any vascular plant tissue. Here we demonstrate the results utilizing Celery and Asparagus
1. Obtain 5x 10 CL strips from the initial batch for each beaker. Take a picture of strips laid out on the table. Count the number of VBs in the strips (total).
2. Prepare beakers with solutions for each respective treatment (e.g. 1M NaCl = 5x beakers each filled with 50mL 1M NaCl) - allow to come to room temperature, or prepare solutions in advance.
3. Place 4 beakers on a hot plate (for salt treatments, the hot plate may be kept on the counter, but for acid and base treatments, the entire procedure must occur in the fume hood).
4. Put the 10 CL strips into each beaker, then increase the temperature of the hot plate to 100°C.
5. Once bubbles can be seen, start a timer for 30 minutes, (or intervals). Remove each beaker after 10, 15, 20 and 30 mins respectively.
6. After time is up, remove the beaker from the hot plate and add 200mL of room temperature water (Caution: do NOT add cold water to the hot beaker as this could cause the glass to shatter - add slowly)
7. Using a thermometer, make 30 figure-8 turns through the bundles thoroughly to gently separate loose bundles. (**NOTE:** avoid shearing or separating strips that are not easily loosened).
8. Pour out the solution - do NOT lose any loose bundles and place strips / excised bundles on a clear plate lid. Take a picture, and note the number of bundles extracted. (Note if the strip simply was separated, or if a VB was clearly excised. Note % bundle extraction)

### Results:

The effects of temperature and boiling times shows effects on the rate of bundle liquid-based extraction on plant material. The following results show that the parenchyma between xylem and phloem bundles can be degraded cutting the slices into smaller pieces, and that prolonged treatments can enable the xylem and phloem bundles to be extracted.

Experiments disclosed herein demonstrate that a variety of liquid-based chemical treatments, such as salt (NaCl, LiCl), maceration (with acetic acid and peroxide) and base treatment (NaOH) were able to change the mechanical and structural properties of the plant material and were able to extract various parts of the plant such as the vascular bundles. Conditions such as concentrations and boiling times were evaluated for their ability to extract these materials as well as the effects of these solutions on the structural and mechanical properties of the materials.

Maceration is commonly used in the pulp and paper and food industries to soften and digest plant materials such as wood, bark, leaves, fruits, and others. In the food industry fruits are often soaked in a liquid to absorb the flavour of the liquid into the food for example strawberries that are sprinkled with sugar so that they may release their juices and soften the strawberry. However, in all cases, these industrial processes cause the degradation of plant tissue ultrastructures which are valuable for tissue engineering applications *in vitro* and *in vivo.*

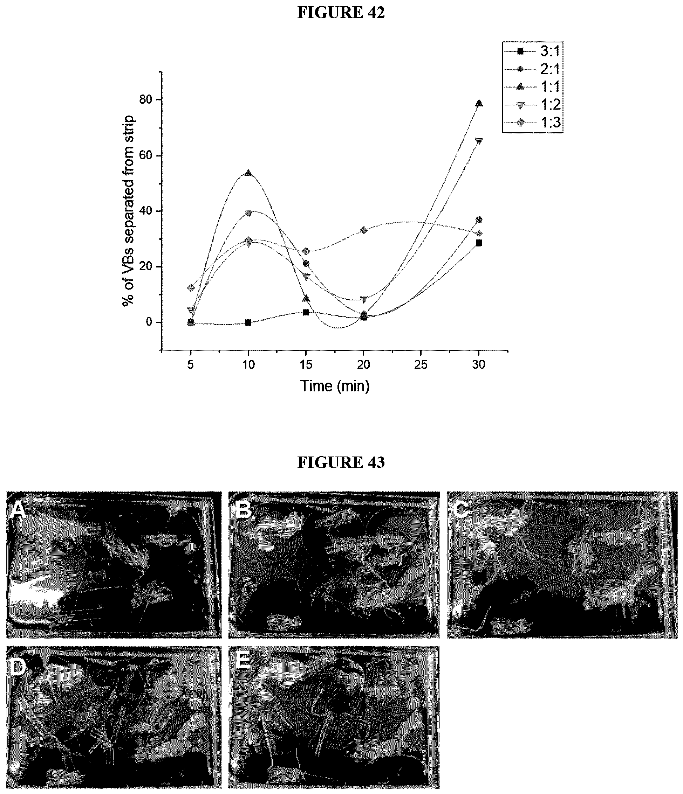

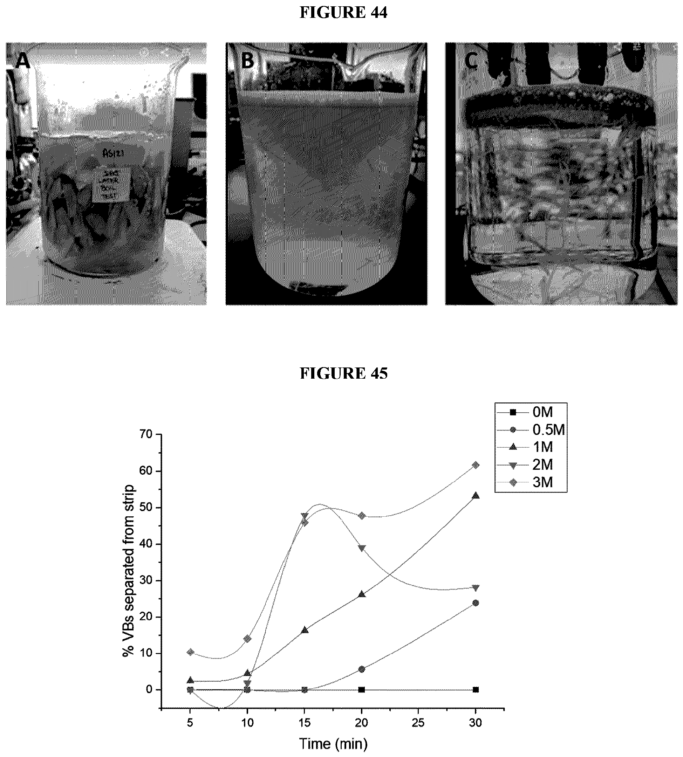

Acid and Peroxide Treatment Results: Acid and peroxide can be used to both soften plant material and to extract different parts of the plant in efforts to preserve the microstructures. This has been done with asparagus and celery, results of which are shown below. Figure 42 shows the results of acid and peroxide extraction and boiling of celery strips in efforts to remove the vascular bundles contained within each strip. We show here that a ratio of 1:1 Acetic acid to 30% hydrogen peroxide boiled for 30 minutes, results in the release of the largest number of vascular bundles (Figs. 43 and 44). It can be seen that ratios of 1:1, 1:2 and 1:3 were able to release bundles from strips from decellularised strips. Similar results are also obtained for salt and alkaline treatments (discussed further below). Fig. 44 depicts asparagus strips treated with a 1:1 acid and peroxide solution of glacial acetic acid:30% hydrogen peroxide. The approaches described herein can also be applied to the vascular tissues of other plants to achieve the same extraction results. Fig. 44 depicts asparagus strips treated with a 1:1 acid and peroxide solution of glacial acetic acid : 30% hydrogen peroxide.

It should be noted that maceration at room temperature did not yield any bundles nor did boiling without the maceration solutions. It is the combination of heating/boiling and maceration solutions that yield bundles. Additionally, the same treatments can be applied to decellularised bundles and yield similar results, however at different boiling times and concentrations. Figure 43 illustrates this whereby previously decellularised celery was subjected to the same conditions as the native strips in Figure 42. It can be noted that individual bundles were able to be extracted.

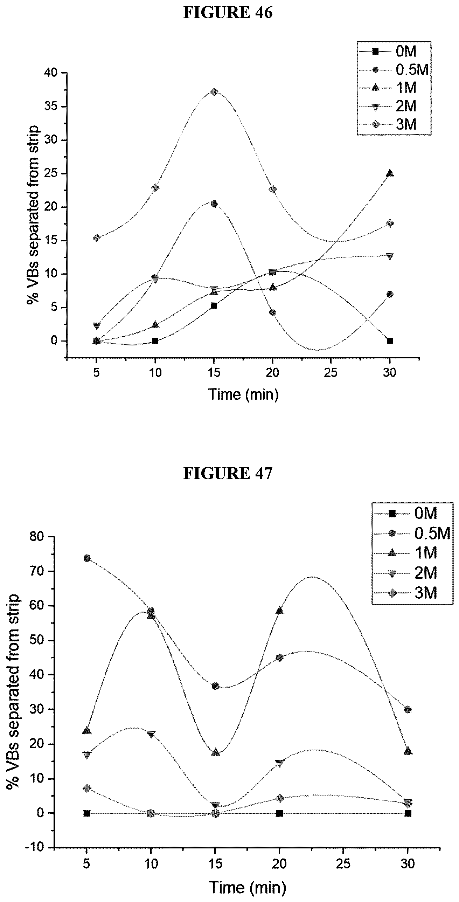

Salt Treatment Results: Treatment with salt has also shown to be useful for the removal of plant bundles. Similarly to acid and peroxide, a range of salt concentrations and boiling times were tested to determine whether alternative solutions were more or less effective at extracting various parts of the plant and preserving the microstructures. Figs. 45 and 46 show that increasing boiling times generally result in the successful extraction of the vascular bundles regardless of the salt concentration, however, the more concentrated solutions (3M and 1M) were shown to extract the largest number of vascular bundles. The 2M solution was also shown to release a large number of bundles after 15 minutes but then decreased after 20 and 30 minutes.

Alkaline Treatment Results: In addition to treatment with acids, excision of bundles from strips using varying concentrations of NaOH base was tested. The results revealed that it is a good candidate for separating bundles from strips. In this specific case, the 0.5M for shorter boiling times (less than 15 minutes) appeared to yield the highest amount of isolated vascular bundles (Fig. 47). The higher concentrations, specifically 2 M and 3 M failed to separate the strips into single vascular bundles. Optimal bundle extraction, which yielded the largest amount of individually separated vascular bundles attached to surrounding parenchyma, or extracted cleanly with no surrounding tissue, appeared to have been around the range of 0.5 - 1M and between 10 and 20 minutes of boiling time.

Vascular Bundle Microstructure: The vascular bundles were stained with 0.1% Calcofluor White for 10 minutes. Cross-sectional cuts of the HDMC were performed with a microtome blade in the PDMS molds. The images were acquired at 2.5X and 10X on a SZX16 Stereo Microscope (1600x1200, ISO100). Image processing was completed on Fiji (ImageJ). The raw images were converted to binary and were then thresholded with adaptive thresholding techniques. The images were processed with denoise, despeckle, and watershed functions. The images were then segmented for particle size analysis. SEM images of the microchannels revealed that there are interconnected pores between the individual channels. This analysis is not focused on those structures, as on the sub-micron scale, the pore size distribution would be much smaller.

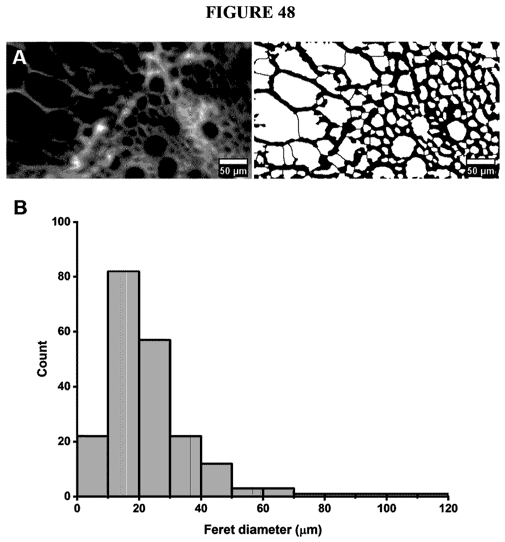

Fig. 48 shows size distribution of CL vascular bundles derived from native tissue immersed in a boiling salt solution of LiCl for 15 minutes. Similar microstructure distributions were observed for the other treatments. In Fig. 48A, the vascular bundles were stained with 0.1% Calcofluor White and the image was thresholded and segmented for size analysis. Fig. 48B is a histogram of the vascular bundle diameters. The mean size was 23.7 ± 1.5 µm. Values are mean and standard error of the mean.

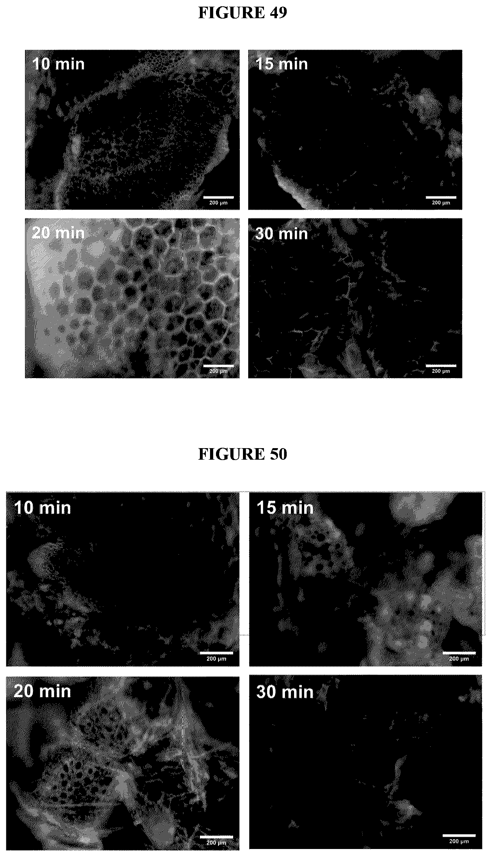

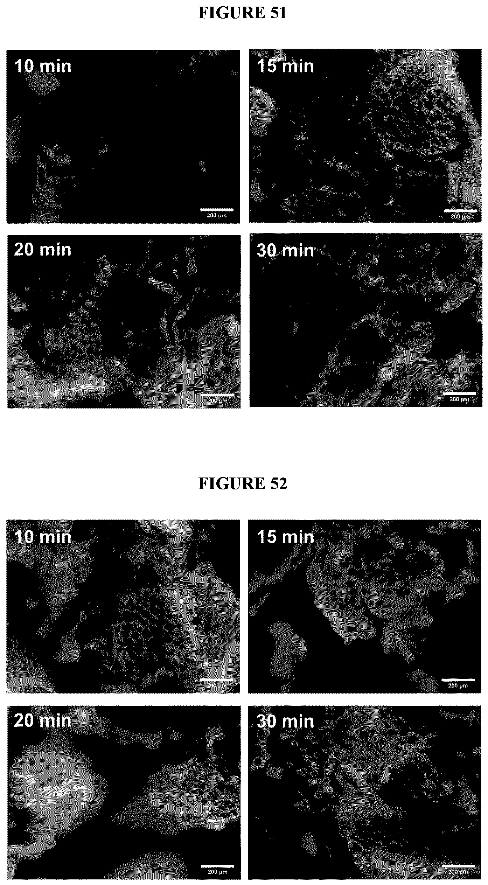

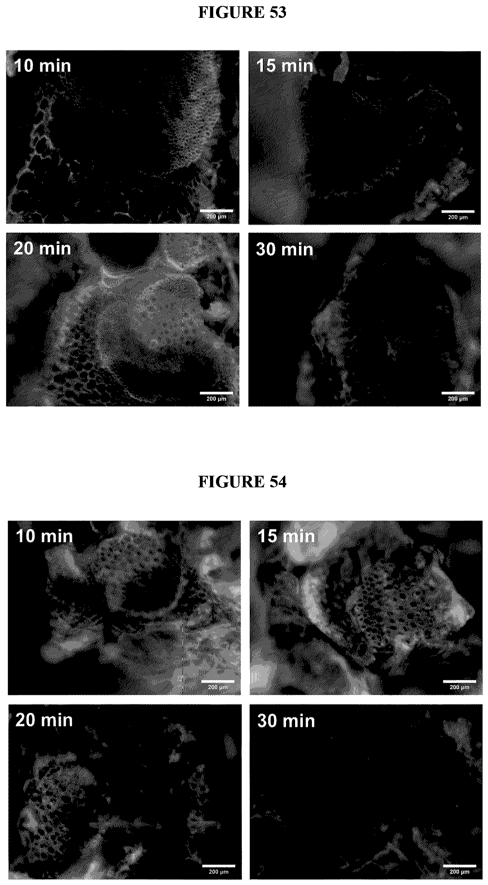

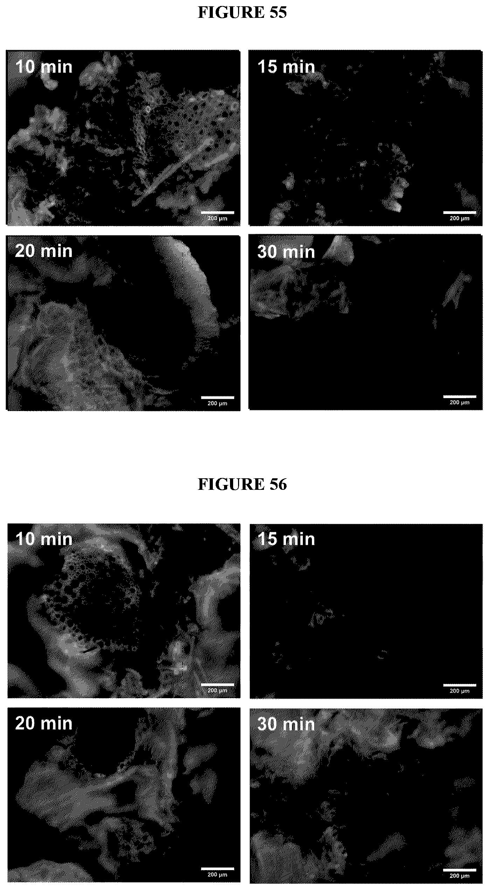

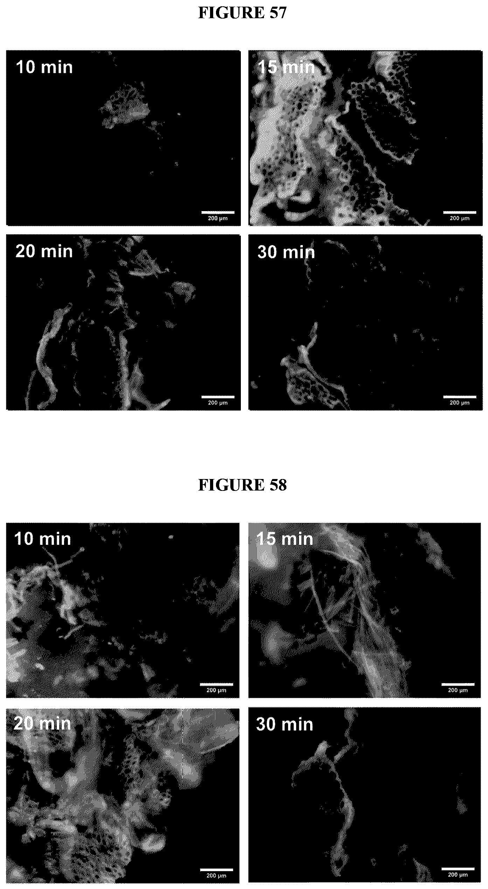

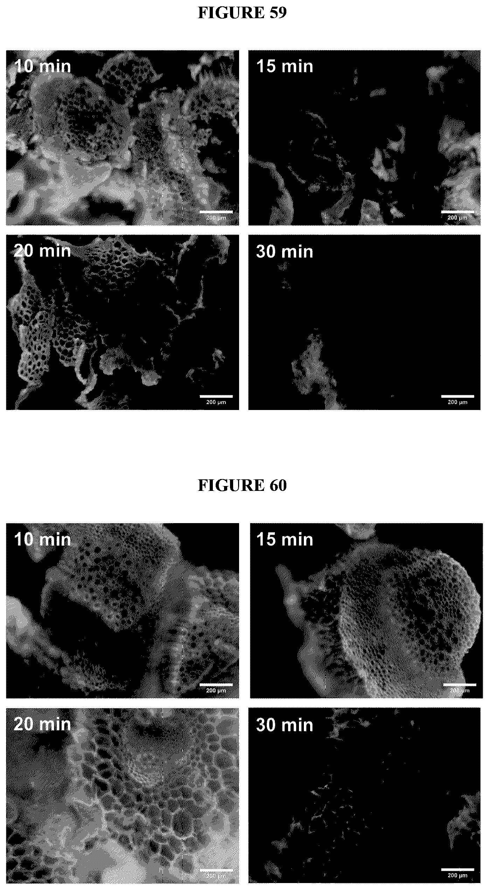

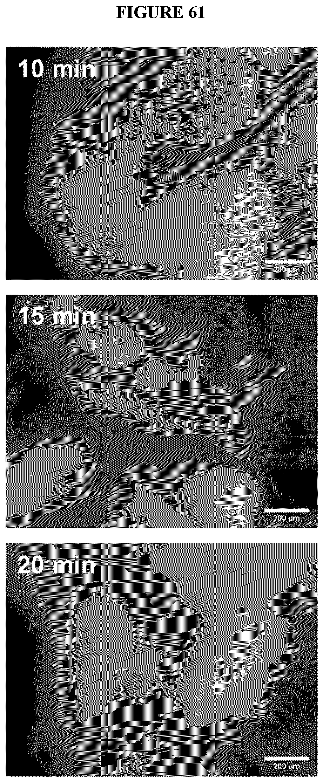

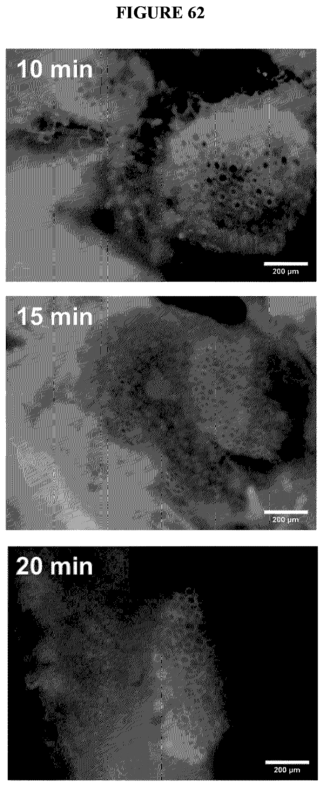

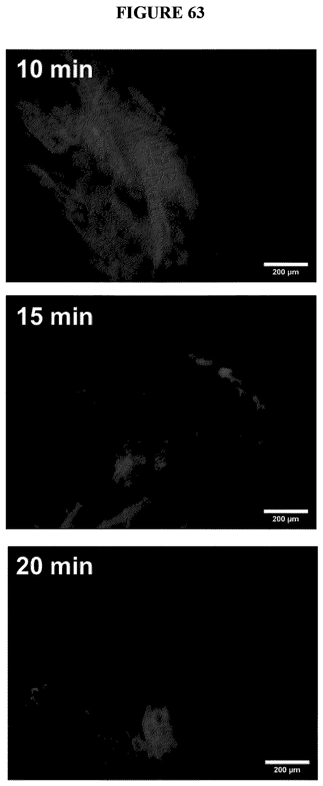

The images provided in Figs. 49-63 summarize the morphology under several acid and peroxide (Figs. 61-63), salt (Figs. 49-56) and alkaline (Figs. 57-60) treatments. Tables 1-4 summarize the major observation and quantitative outcomes. Tables 5-8 summarize mechanical properties of vascular bundles following treatments.

**Table 1: Bundle morphology of LiCl treated native celery.**

| **Time (min)** | **[0.5 M]** | **[1 M]** | **[2 M]** | **[3 M]** |
|---|---|---|---|---|
| 10 | Intact | Intact | Intact | Slightly degraded |
| 15 | Intact | Intact | Intact | Slightly degraded |
| 20 | Intact | Slightly degraded | Slightly degraded | Slightly degraded |
| 30 | Intact | Slightly degraded | Slightly degraded | Slightly degraded |

**Table 2: Bundle morphology of NaCl treated native celery.**

| **Time (min)** | **[0.5 M]** | **[1 M]** | **[2 M]** | **[3 M]** |
|---|---|---|---|---|
| 10 | Intact | Intact | Intact | Intact |
| 15 | Intact | Intact | Slightly degraded | Slightly degraded |
| 20 | Intact | Intact | Slightly degraded | Slightly degraded |
| 30 | Intact | Intact | Slightly degraded | Slightly degraded |

**Table 3: Bundle morphology of NaOH treated native celery.**

| **Time (min)** | **[0.5 M]** | **[1 M]** | **[2 M]** |
|---|---|---|---|
| 10 | Slightly degraded | Slightly degraded | Slightly degraded |
| 15 | Slightly degraded | Slightly degraded | Slightly degraded |
| 20 | Slightly degraded | Slightly degraded | Slightly degraded |
| 30 | Slightly degraded | Slightly degraded | Slightly degraded |

**Table 4: Bundle morphology of macerated native celery using different acetic acid:peroxide ratios.**

| **Time (min)** | **1:1** | **1:2** | **2:1** |
|---|---|---|---|
| 15 | Intact | Intact | Slightly degraded |
| 20 | Intact | Intact | Slightly degraded |
| 30 | Slightly degraded | Intact | Slightly degraded |

**Table 5. Ultimate Yield Strength (in MPa) of acid and peroxide solution-treated CL strips at various concentrations and boiled for 5, 10, 15, 20 and 30 minutes. Values are n=5 strips ± StDev.**

| | **3:1** | **2:1** | **1:1** | **1:2** | **1:3** |
|---|---|---|---|---|---|
| **5min** | N/A | N/A | N/A | N/A | N/A |
| **10min** | N/A | ~ 0 | 0.21 ± 0.12 | 0.20 ± 0.06 | 0.11 ± 0.02 |
| **15min** | N/A | 0.24 ± 0.08 | 0.62 ± 0.31 | 0.14 ± 0.06 | 0.06 ± 0.03 |
| **20min** | N/A | 0.68 ± 0.08 | 0.46 ± 0.09 | 0.38 ± 0.25 | 1.12 ± 0.50 |
| **30min** | N/A | N/A | N/A | N/A | N/A |

**Table 6. Ultimate Yield Strength (in MPa) of LiCl-treated CL strips at various concentrations and boiled for 5, 10, 15, 20 and 30 minutes. Values are n=5 strips ± StDev.**

| | **0M** | **0.5M** | **1M** | **2M** | **3M** |
|---|---|---|---|---|---|
| **5min** | 1.28 ± 0.86 | N/A | N/A | N/A | N/A |
| **10min** | 2.02 ± 1.06 | 1.20 ± 0.83 | 0.29 ± 0.08 | 0.38 ± 0.23 | 0.16 ± 0.09 |
| **15min** | 2.16 ± 1.22 | 0.41 ± 0.10 | 0.30 ± 0.12 | 0.16 ± 0.04 | 0.20 ± 0.06 |
| **20min** | 1.47 ± 0.97 | 0.40 ± 0.15 | 0.27 ± 0.09 | 0.21 ± 0.12 | 0.15 ± 0.07 |
| **30min** | 2.39 ± 1.20 | 0.15 ± 0.07 | 0.10 ± 0.06 | 0.17 ± 0.05 | 0.34 ± 0.15 |

**Table 7. Ultimate Yield Strength (in MPa) of NaCl-treated CL strips at various concentrations and boiled for 5, 10, 15, 20 and 30 minutes. Values are n=5 strips ± StDev.**

| | **0M** | **0.5M** | **1M** | **2M** | **3M** |
|---|---|---|---|---|---|
| **5min** | 1.28 ± 0.86 | N/A | N/A | N/A | 0.43 ± 0.13 |
| **10min** | 2.02 ± 1.06 | 0.85 ± 0.36 | 0.57 ± 0.10 | 0.91 ± 0.64 | 0.34 ± 0.11 |
| **15min** | 2.16 ± 1.22 | 0.40 ± 0.10 | 0.45 ± 0.19 | 0.65 ± 0.47 | 0.66 ± 0.17 |
| **20min** | 1.47 ± 0.97 | 1.72 ± 0.91 | 0.23 ± 0.11 | 0.46 ±0.09 | 0.25 ± 0.35 |
| **30min** | 2.39 ± 1.20 | 0.60 ± 0.48 | 0.38 ± 0.13 | 0.46 ± 0.28 | 0.25 ± 0.16 |

**Table 8. Ultimate Yield Strength (in MPa) of NaOH-treated CL strips at various concentrations and boiled for 5, 10, 15, 20 and 30 minutes. Values are n=5 strips ± StDev.**

| | **0M** | **0.5M** | **1M** | **2M** | **3M** |
|---|---|---|---|---|---|
| **5min** | 1.28 ± 0.86 | 0.16 ± 0.10 | 0.25 ± 0.19 | 0.64 ± 0.17 | N/A |
| **10min** | 2.02 ± 1.06 | 0.24 ± 0.10 | 0.35 ± 0.19 | 0.43 ± 0.29 | N/A |
| **15min** | 2.16 ± 1.22 | 0.15 ± 0.05 | 0.26 ± 0.04 | 0.61 ± 0.17 | N/A |
| **20min** | 1.47 ± 0.97 | 0.17 ± 0.06 | 0.12 ± 0.02 | 0.68 ± 0.12 | N/A |
| **30min** | 2.39 ± 1.20 | 0.21 ± 0.07 | 0.25 ± 0.06 | 1.73 ± 0.37 | N/A |

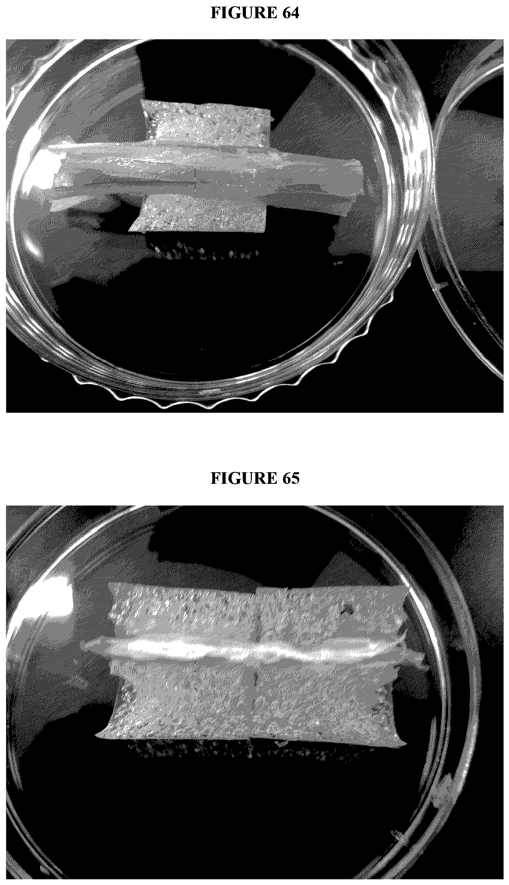

The VBs extracted using the procedures described in this Example may be used in the bundling of HDMC as described herein. Figs. 64 and 65 depict HDMCs assembled from isolated CL vascular bundles boiled in water for 30 min (control) and 1M NaOH for 30 min. The NaOH bundles rapidly dehydrated and glued quickly. In this example, a cyanoacrylate glue was used to bundle the HDMC.

### Example 10: Alternative Example of Methods and Procedures for Liquid-based Extraction of Intact Vascular Bundle Microchannels from Native or Decellularised Vascular Plants for HDMC Production

Procedure:
1. Celery was cut into 1-inch pieces followed by slicing on a mandoline kitchen slicer;
2. ~ 50-60 Pieces were placed in a 1L beaker filled to the top with maceration solution (see treatment solutions 1-7 below) until all slices were covered.
3. The solution was brought to a boil (95-100°C) and was mixed thoroughly every 5 minutes.
4. Bundles began separating from the rest of the strip after 10 minutes of boiling.
5. After maceration was completed, samples were removed from macerating solution and are washed several times with distilled water until acetic acid and peroxide was removed.

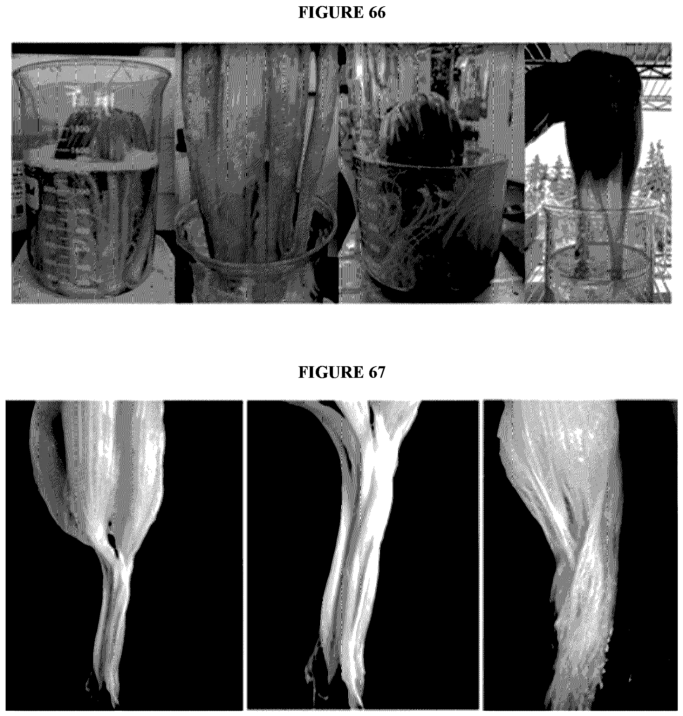

It was observed that after increased boiling times, more bundles were seen to separate from the rest of the slice, however bundles were observed to soften due to increased boiling time. Images of macerated CL in diluted acetic acid and peroxide boiled for 15 minutes are shown in Figure 66.

Additionally, a variety of solutions were tested for their potential as macerating products. These included:

| | |
|---|---|
| Treatment Solution 1: | 1:1 glacial acetic acid and 30% hydrogen peroxide |
| Treatment Solution 2: | 1:1 glacial acetic acid and peroxide (as above), diluted 50% in water |
| Treatment Solution 3: | Glacial acetic acid |
| Treatment Solution 4: | 50% diluted acetic acid |
| Treatment Solution 5: | 95% ethanol |
| Treatment Solution 6: | 3 Molar Sodium Chloride (NaCl) |
| Treatment Solution 7: | 4 Molar Hydrochloric Acid (HCl) |

Of these treatments, Treatment Solution #s 1, 2, 4, 6, and 7 were able to remove bundles, however the concentrated hydrochloric acid broke down the bundles beyond the point of use and chopped up the individual pieces, therefore the treatment condition was too strong. It was therefore diluted two-fold for subsequent testing.

It was also seen that celery and asparagus did not require slicing or prior preparation of the sample prior their addition into maceration solution. For these two, once samples were removed from the macerating solution, a significant amount of mixing was performed to excise bundles from pieces. The celery and asparagus were soft enough that the surrounding tissue could be squeezed off from the bundles (in celery with much more ease than asparagus). The long fibers were removed from the solution and placed into a new beaker with distilled water. The bundles were then cleaned with vigorous mixing and manual squishing of the sample while avoiding destruction of the bundles. Increasing amounts of surrounding tissue were removed after each wash with mixing (Figure 67).

As will be understood, a decellularization step (such as an SDS-based decellularization as described herein) may be performed either before or after the liquid extraction/maceration step, if/as desired.

Embodiments described herein include:
Embodiment 1: A scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle.
Embodiment 2: The scaffold biomaterial of embodiment 1, wherein the decellularised microchannels comprise decellularised xylem and/or phloem channels.
Embodiment 3: The scaffold biomaterial of embodiment 2, wherein the decellularised xylem and/or phloem channels are individually isolated from plant or fungal tissue, are grouped in one or more vascular bundles isolated from plant or fungal tissue, or any combination thereof.
Embodiment 4: The scaffold biomaterial of any one of embodiments 1-3, wherein the plurality of decellularised microchannels are glued together within the bundle.
Embodiment 5: The scaffold biomaterial of embodiment 4, wherein the plurality of decellularised microchannels are glued together within the bundle by a biocompatible, optionally biodegradable, optionally low-volume expansion, glue.
Embodiment 6: The scaffold biomaterial of embodiment 5, wherein the biocompatible glue comprises a PEG-based, polyurethane-based, gelatin-based, or a fibrin-based glue.
Embodiment 7: The scaffold biomaterial of embodiment 6, wherein the biocompatible glue comprises a fibrin-based glue.
Embodiment 8: The scaffold biomaterial of any one of embodiments 1-7, wherein the decellularised microchannels are cellulose-based, chitin-based, lignin-based, hemicellulose-based, or pectin-based, or any combination thereof.
Embodiment 9: The scaffold biomaterial of any one of embodiments 1-8, wherein a density of decellularised microchannels within the bundle is greater than a density of microchannels within the plant or fungal tissue.
Embodiment 10: The scaffold biomaterial of any one of embodiments 1-9, wherein the plant or fungal tissue comprises a microchannel-containing tissue from apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, a rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue, or a genetically altered tissue produced via direct genome modification or through selective breeding, or any combinations thereof.
Embodiment 11: The scaffold biomaterial of any one of embodiments 1-9, wherein the plant or fungal tissue comprises celery, asparagus, or both.
Embodiment 12: The scaffold biomaterial of any one of embodiments 1-11, further comprising living cells, in particular non-native cells, on and/or within at least one of the decellularised microchannels.
Embodiment 13: The scaffold biomaterial of embodiment 12, wherein the living cells are animal cells.
Embodiment 14: The scaffold biomaterial of embodiment 13, wherein the living cells are mammalian cells.
Embodiment 15: The scaffold biomaterial of embodiment 14, wherein the living cells are human cells.
Embodiment 16: Use of the scaffold biomaterial of any one of embodiments 1-15 for supporting animal or plant cell growth; promoting tissue regeneration; promoting angiogenesis; treatment and/or repair of spinal cord injury; repair or reconstruction or replacement of plant or animal tissue; solution or material filtration and/or separation; plant modification or growth modulation; material transport; assembly for mimicking a desired shape or object; and/or microfluidics; or any suitable application in which biocompatible micrometer-scale channels may be of use.
Embodiment 17: The use of embodiment 16, wherein the scaffold biomaterial is for repair or reconstruction or replacement of plant or animal tissue, wherein the plant or animal tissue comprises damaged microvasculature, or plant tissue damaged by vascular disease (such as wilt disease), or damaged vascular structure of trees infested with an insect (such as emerald ash borer).
Embodiment 18: The use of embodiment 16 or 17, wherein the scaffold biomaterial is for repair or reconstruction of plant or animal tissue, wherein the plant or animal tissue comprises damaged microvasculature, wherein angiogenesis is impaired or compromised.
Embodiment 19: The use of any one of embodiments 16-18, wherein the scaffold biomaterial is for repair or reconstruction of animal tissue, wherein the animal is human.
Embodiment 20: The use of embodiment 16, wherein the scaffold biomaterial is for plant modification or growth modulation, wherein the modification is modification of a grafting process for accelerated growth.
Embodiment 21: The use of embodiment 16, wherein the scaffold biomaterial is for material transport, wherein the material transport is a drug delivery application.
Embodiment 22: The use of embodiment 16, wherein the scaffold biomaterial is for heat transfer or heat exchange microfluidics.
Embodiment 23: A method for supporting cell growth, said method comprising:
   providing a scaffold biomaterial as defined in any one of embodiments 1-15; and
   introducing one or more cells to the scaffold biomaterial.
Embodiment 24: A method for promoting tissue regeneration; promoting angiogenesis; treatment and/or repair of spinal cord injury; or repair or reconstruction or replacement of plant or animal tissue, said method comprising:
   providing a scaffold biomaterial as defined in any one of embodiments 1-15; and
   implanting said scaffold biomaterial at a site in need thereof.
Embodiment 25: A method for solution or material filtration and/or separation, said method comprising:
   providing a scaffold biomaterial as defined in any one of embodiments 1-15; and
   passing a solution or material through the scaffold biomaterial so as to filter and/or separate components from the solution based on size exclusion.
Embodiment 26: A method for plant modification or growth modulation, said method comprising:
   providing a scaffold biomaterial as defined in any one of embodiments 1-15; and
   grafting the scaffold biomaterial into the plant to provide for accelerated growth.
Embodiment 27: A method for material transport, said method comprising:
   providing a scaffold biomaterial as defined in any one of embodiments 1-15; and
   using the scaffold biomaterial to transport a material to a site in need thereof.
Embodiment 28: The method of embodiment 27, wherein the material is a drug, and the scaffold biomaterial is used for drug delivery.
Embodiment 29: A method for preparing a structure mimicking a desired shape or object, said method comprising:
   providing a scaffold biomaterial as defined in any one of embodiments 1-15;
   carving or arranging the scaffold biomaterial so as to mimic the desired shape or object; and
   optionally, gluing the scaffold biomaterial for structural reinforcement.
Embodiment 30: A method for exchanging or transferring heat in a microfluidics process, said method comprising:
   providing a scaffold biomaterial as defined in any one of embodiments 1-15; and
   using the microchannels of the scaffold biomaterial to carry one or more fluids, wherein the one or more fluids are in close proximity so as to allow for heat exchange or heat transfer.
Embodiment 31: A method for isolation and decellularisation of microchannels from a plant or fungal tissue, said method comprising:
   separating microchannels from the plant or fungal tissue; and
   decellularising the microchannels; and
   optionally, sterilizing the microchannels.
Embodiment 32: The method of embodiment 31, wherein the step of separating the microchannels comprises mechanical separation of the microchannels, or vascular bundles containing the microchannels, or both, from surrounding plant or fungal tissue.
Embodiment 33: The method of embodiment 32, wherein the step of separating is performed by gentle peeling, or by cutting.
Embodiment 34: The method of embodiment 32, wherein the step of separating is performed by liquid-based extraction of the microchannels from the plant or fungal tissue.
Embodiment 35: The method of embodiment 34, wherein liquid-based extraction comprises at least one of acid extraction, acid and peroxide extraction, salt extraction or alkaline extraction.
Embodiment 36: The method of embodiment 35, wherein acid and peroxide extraction comprises heating the plant or fungal tissue in an acid and peroxide solution.
Embodiment 37: The method of embodiment 36, wherein the acid and peroxide solution comprises glacial acetic acid and 30% hydrogen peroxide in a ratio of 3:1 to 1:3.
Embodiment 38: The method of embodiment 37, wherein the acid and peroxide solution comprises 1:1 glacial acetic acid:hydrogen peroxide solution (30% v/v).
Embodiment 39: The method of any one of embodiments 36-38, wherein heating comprises heating up to 30 minutes.
Embodiment 40: The method of embodiment 39, wherein heating comprises heating for 30 minutes.
Embodiment 41: The method of any one of embodiments 36-40, wherein heating comprises boiling the acid and peroxide solution.
Embodiment 42: The method of embodiment 36, wherein the acid and peroxide solution comprises 1:1 glacial acetic acid:hydrogen peroxide solution (30% v/v) and the solution is heated to boiling for 30 minutes.
Embodiment 43: The method of any one of embodiments 36-42, wherein the liquid-based extraction further comprises mechanically agitating, for example stirring, the plant or fungal tissue in the acid and peroxide solution.
Embodiment 44: The method of embodiment 35, wherein salt extraction comprises heating the plant or fungal tissue in a salt solution.
Embodiment 45: The method of embodiment 44, wherein the salt solution comprises LiCl or NaCl.
Embodiment 46: The method of any one of embodiments 44-45, wherein the salt solution has a salt concentration of about 0.5M-3M.
Embodiment 47: The method of embodiment 46, wherein the salt solution comprises LiCl or NaCl at a salt concentration of about 3M.
Embodiment 48: The method of any one of embodiments 44-47, wherein heating comprises heating up to 30 minutes.
Embodiment 49: The method of embodiment 48, wherein heating comprises heating for 30 minutes.
Embodiment 50: The method of any one of embodiments 44-49, wherein heating comprises boiling the salt solution.
Embodiment 51: The method of embodiment 44, wherein the salt solution comprises about 3M LiCl and the solution is heated to boiling for 30 minutes.
Embodiment 52: The method of embodiment 44, wherein the salt solution comprises about 3M NaCl and the solution is heated to boiling for 15 minutes.
Embodiment 53: The method of any one of embodiments 44-52, wherein the liquid-based extraction further comprises mechanically agitating, for example stirring, the plant or fungal tissue in the salt solution.
Embodiment 54: The method of embodiment 35, wherein alkaline extraction comprises heating the plant or fungal tissue in an alkaline solution.
Embodiment 55: The method of embodiment 54, wherein the alkaline solution has an alkaline concentration of about 0.5-3M.
Embodiment 56: The method of embodiment 55, wherein the alkaline solution comprises sodium hydroxide (NaOH).
Embodiment 57: The method of embodiment 56, wherein the alkaline solution comprises sodium hydroxide at an alkaline concentration of about 0.5M-1M.
Embodiment 58: The method of any one of embodiments 54-57, wherein heating comprises heating up to 30 minutes.
Embodiment 59: The method of embodiment 58, wherein heating comprises heating for 30 minutes.
Embodiment 60: The method of any one of embodiments 54-59, wherein heating comprises boiling the alkaline solution.
Embodiment 61: The method of embodiment 54, wherein the alkaline solution comprises about 0.5M NaOH and the solution is heated to boiling for 5 minutes.
Embodiment 62: The method of any one of embodiments 54-61, wherein the liquid-based extraction further comprises mechanically agitating, for example stirring, the plant or fungal tissue in the alkaline solution.
Embodiment 63: The method of embodiment 35, wherein acid extraction comprises heating the plant or fungal tissue in an acid solution.
Embodiment 64: The method of embodiment 63, wherein the acid solution comprises acetic acid or hydrochloric acid.
Embodiment 65: The method of embodiment 64, wherein the acid solution comprises 50% acetic acid.
Embodiment 66: The method of any one of embodiments 63-65, wherein heating comprises heating up to 30 minutes.
Embodiment 67: The method of embodiment 66, wherein heating comprises heating for 30 minutes.
Embodiment 68: The method of any one of embodiments 63-67, wherein heating comprises boiling the acid solution.
Embodiment 69: The method of embodiment 63, wherein the acid solution comprises 50% acetic acid and the solution is heated to boiling for 30 minutes.
Embodiment 70: The method of any one of embodiments 63-69, wherein the liquid-based extraction further comprises mechanically agitating, for example stirring, the plant or fungal tissue in the acid and solution.
Embodiment 71: The method of any one of embodiments 34-70, wherein the step of decellularising occurs before the step of separating.
Embodiment 72: A method for preparing a scaffold biomaterial, said method comprising:
   separating microchannels from a plant or fungal tissue, or providing microchannels already separated from a plant or fungal tissue;
   decellularising the microchannels;
   bundling the microchannels together, the microchannels being arranged substantially parallel to each other;
   thereby providing a scaffold biomaterial comprising bundled microchannels.
Embodiment 73: The method of embodiment 72, wherein the step of separating the microchannels comprises mechanical separation of the microchannels, or vascular bundles containing the microchannels, or both, from surrounding plant or fungal tissue.
Embodiment 74: The method of embodiment 73, wherein the step of separating is performed by gentle peeling, or by cutting.
Embodiment 75: The method of embodiment 72, wherein the step of separating is performed by liquid-based extraction of the microchannels from the plant or fungal tissue.
Embodiment 76: The method of embodiment 75, wherein liquid-based extraction comprises at least one of acid extraction, acid and peroxide extraction, salt extraction or alkaline extraction.
Embodiment 77: The method of embodiment 76, wherein acid and peroxide extraction comprises heating the plant or fungal tissue in an acid and peroxide solution.
Embodiment 78: The method of embodiment 77, wherein the acid and peroxide solution comprises glacial acetic acid and 30% hydrogen peroxide in a ratio of 3:1 to 1:3.
Embodiment 79: The method of embodiment 78, wherein the acid and peroxide solution comprises 1:1 glacial acetic acid:hydrogen peroxide solution (30%).
Embodiment 80: The method of any one of embodiments 77-79, wherein heating comprises heating up to 30 minutes.
Embodiment 81: The method of embodiment 80, wherein heating comprises heating for 30 minutes.
Embodiment 82: The method of any one of embodiments 77-81, wherein heating comprises boiling the acid and peroxide solution.
Embodiment 83: The method of embodiment 77, wherein the acid and peroxide solution comprises 1:1 glacial acetic acid:hydrogen peroxide solution (30% v/v) and the solution is heated to boiling for 30 minutes.
Embodiment 84: The method of any one of embodiments 77-83, wherein the liquid-based extraction further comprises mechanically agitating, for example stirring, the plant or fungal tissue in the acid and peroxide solution.
Embodiment 85: The method of embodiment 76, wherein salt extraction comprises heating the plant or fungal tissue in a salt solution.
Embodiment 86: The method of embodiment 85, wherein the salt solution comprises LiCl or NaCl.
Embodiment 87: The method of any one of embodiments 85-86, wherein the salt solution has a salt concentration of about 0.5M-3M.
Embodiment 88: The method of embodiment 87, wherein the salt solution comprises LiCl or NaCl at a salt concentration of about 3M.
Embodiment 89: The method of any one of embodiments 85-88, wherein heating comprises heating up to 30 minutes.
Embodiment 90: The method of embodiment 89, wherein heating comprises heating for 30 minutes.
Embodiment 91: The method of any one of embodiments 85-90, wherein heating comprises boiling the salt solution.
Embodiment 92: The method of embodiment 85, wherein the salt solution comprises about 3M LiCl and the solution is heated to boiling for 30 minutes.
Embodiment 93: The method of embodiment 85, wherein the salt solution comprises about 3M NaCl and the solution is heated to boiling for 15 minutes.
Embodiment 94: The method of any one of embodiments 85-93, wherein the liquid-based extraction further comprises mechanically agitating, for example stirring, the plant or fungal tissue in the salt solution.
Embodiment 95: The method of embodiment 76, wherein alkaline extraction comprises heating the plant or fungal tissue in an alkaline solution.
Embodiment 96: The method of embodiment 95, wherein the alkaline solution comprises sodium hydroxide (NaOH).
Embodiment 97: The method of any one of embodiment 95-96, wherein the alkaline solution has an alkaline concentration of about 0.5-3M.
Embodiment 98: The method of embodiment 97, wherein the alkaline solution comprises sodium hydroxide at an alkaline concentration of about 0.5M-1M.
Embodiment 99: The method of any one of embodiments 95-98, wherein heating comprises heating up to 30 minutes.
Embodiment 100: The method of embodiment 99, wherein heating comprises heating for 30 minutes.
Embodiment 101: The method of any one of embodiments 95-100, wherein heating comprises boiling the alkaline solution.
Embodiment 102: The method of embodiment 95, wherein the alkaline solution comprises about 0.5M NaOH and the solution is heated to boiling for 5 minutes.
Embodiment 103: The method of any one of embodiments 95-102, wherein the liquid-based extraction further comprises mechanically agitating, for example stirring, the plant or fungal tissue in the alkaline solution.
Embodiment 104: The method of embodiment 76, wherein acid extraction comprises heating the plant or fungal tissue in an acid solution.
Embodiment 105: The method of embodiment 104, wherein the acid solution comprises acetic acid or hydrochloric acid.
Embodiment 106: The method of embodiment 104-105, wherein the acid solution comprises 50% acetic acid.
Embodiment 107: The method of any one of embodiments 104-106, wherein heating comprises heating up to 30 minutes.
Embodiment 108: The method of embodiment 107, wherein heating comprises heating for 30 minutes.
Embodiment 109: The method of any one of embodiments 104-108, wherein heating comprises boiling the acid solution.
Embodiment 110: The method of embodiment 104, wherein the acid solution comprises 50% acetic acid and the solution is heated to boiling for 30 minutes.
Embodiment 111: The method of any one of embodiments 104-110, wherein the liquid-based extraction further comprises mechanically agitating, for example stirring, the plant or fungal tissue in the acid and solution.
Embodiment 112: The method of any one of embodiments 76-111, wherein the step of decellularising occurs before the step of separating.
Embodiment 113: The method of any one of embodiments 72-112, wherein the step of bundling comprises gluing the microchannels together.
Embodiment 114: The method of embodiment 113, wherein the microchannels are glued together by a biocompatible, optionally biodegradable, glue.
Embodiment 115: The method of embodiment 114, wherein the biocompatible glue comprises a PEG-based, polyurethane-based, gelatin-based, or a fibrin-based glue.
Embodiment 116: The method of embodiment 115, wherein the biocompatible glue comprises a fibrin-based glue.
Embodiment 117: The method of any one of embodiments 72-116, wherein the step of bundling comprises molding the microchannels.
Embodiment 118: The method of embodiment 117, wherein the molding is performed using a mold into which the microchannels are packed.
Embodiment 119: The method of embodiment 118, wherein the mold comprises biomedical-grade silicone.
Embodiment 120: The method of embodiment 118 or 119, wherein the mold comprises a passage for receiving the microchannels therein, the passage having cross-sectional dimensions which are imparted to the microchannels being molded therein.
Embodiment 121: The method of any one of embodiments 118-120, wherein the mold is divided into two or more sections, which can be assembled around the microchannels for molding.
Embodiment 122: The method of any one of embodiments 118-121, wherein glue is added to a surface of the mold facing the microchannels, and the microchannels are packed in the mold and held therein while the glue cures.
Embodiment 123: The method of embodiment 122, wherein the mold comprises a first section and a second section, and the molding comprises adding glue to a surface of the first section facing the microchannels and to a surface of the second section facing the microchannels; placing the microchannels in the first section and the second section in contact with the glue; adding glue to an exposed surface of the microchannels in the first section, the microchannels in the second section, or both; installing the second section with microchannels over the exposed surface of the microchannels of the first section, opposite the first section, thereby assembling the mold; allowing the glue to substantially cure; and removing the mold.
Embodiment 124: The method of any one of embodiments 72-123, further comprising a step of cutting the bundled microchannels to a desired length.
Embodiment 125: The method of any one of embodiments 118-123, further comprising a step of cutting the bundled microchannels to a desired length, wherein the step of cutting is performed before the mold is removed.
Embodiment 126: The method of any one of embodiments 72-125, wherein the method further comprises a step of sterilizing the microchannels.
Embodiment 127: The method of any one of embodiments 31-33 or 126, wherein the step of sterilizing comprises exposing the microchannels to ethanol, or a mixture of ethanol and water.
Embodiment 128: The method of any one of embodiments 31-127, wherein the microchannels comprise xylem and/or phloem channels.
Embodiment 129: The method of any one of embodiments 31-128, wherein the decellularised microchannels are cellulose-based, chitin-based, lignin-based, hemicellulose-based, or pectin-based, or any combination thereof.
Embodiment 130: The method of any one of embodiments 31-129, wherein a density of decellularised microchannels within the bundle is greater than a density of microchannels within the plant or fungal tissue.
Embodiment 131: The method of any one of embodiments 31-130, wherein the plant or fungal tissue comprises a microchannel-containing tissue from apple hypanthium (Malus pumila) tissue, a fern (Monilophytes) tissue, a turnip (Brassica rapa) root tissue, a gingko branch tissue, a horsetail (equisetum) tissue, a hermocallis hybrid leaf tissue, a kale (Brassica oleracea) stem tissue, a conifers Douglas Fir (Pseudotsuga menziesii) tissue, a cactus fruit (pitaya) flesh tissue, a Maculata Vinca tissue, an Aquatic Lotus (Nelumbo nucifera) tissue, a Tulip (Tulipa gesneriana) petal tissue, a Plantain (Musa paradisiaca) tissue, a broccoli (Brassica oleracea) stem tissue, a maple leaf (Acer psuedoplatanus) stem tissue, a beet (Beta vulgaris) primary root tissue, a green onion (Allium cepa) tissue, a orchid (Orchidaceae) tissue, turnip (Brassica rapa) stem tissue, a leek (Allium ampeloprasum) tissue, a maple (Acer) tree branch tissue, a celery (Apium graveolens) tissue, a green onion (Allium cepa) stem tissue, a pine tissue, an aloe vera tissue, a watermelon (Citrullus lanatus var. lanatus) tissue, a Creeping Jenny (Lysimachia nummularia) tissue, a cactae tissue, a Lychnis Alpina tissue, a rhubarb (Rheum rhabarbarum) tissue, a pumpkin flesh (Cucurbita pepo) tissue, a Dracena (Asparagaceae) stem tissue, a Spiderwort (Tradescantia virginiana) stem tissue, an Asparagus (Asparagus officinalis) stem tissue, a mushroom (Fungi) tissue, a fennel (Foeniculum vulgare) tissue, a rose (Rosa) tissue, a carrot (Daucus carota) tissue, or a pear (Pomaceous) tissue, or a genetically altered tissue produced via direct genome modification or through selective breeding, or any combinations thereof.
Embodiment 132: The method of any one of embodiments 31-131, wherein the plant or fungal tissue comprises celery, asparagus, or both.
Embodiment 133: The method of any one of embodiments 31-132, wherein the step of decellularising the microchannels comprises decellularlising by thermal shock, treatment with detergent, osmotic shock, lyophilisation, physical lysing, electrical disruption, or enzymatic digestion, or any combination thereof, thereby removing cellular materials and nucleic acids to provide decellularised microchannels.
Embodiment 134: The method of embodiment 133, wherein the step of decellularising comprises treatment with sodium dodecyl sulphate (SDS).
Embodiment 135: The method of embodiment 134, wherein residual SDS is removed by using an aqueous divalent salt solution to precipitate a salt residue containing SDS micelles out of the microchannels.
Embodiment 136: The method of embodiment 135, wherein dH₂O, DI water, acetic acid, DMSO, or sonication treatment, or any combination thereof, is used to remove the aqueous divalent salt solution, the salt residue, and/or the SDS micelles.
Embodiment 137: The method of embodiment 136, wherein the divalent salt of the aqueous divalent salt solution comprises MgCl₂ or CaCl₂.
Embodiment 138: The method of embodiment 137, wherein the step of decellularising comprises treatment with an SDS solution of about 0.1% or about 1% SDS in water, and the residual SDS is removed following decellularisation using an aqueous CaCl₂ solution at a concentration of about 100mM, followed by incubation in dH₂O or DI water.
Embodiment 139: The method of any one of embodiments 31-138, further comprising a step of introducing living plant or animal cells to the microchannels
Embodiment 140: The method of embodiment 139, wherein the living cells are mammalian cells.
Embodiment 141: The method of embodiment 140, wherein the living cells are human cells.
Embodiment 142: A decellularised microchannel produced by the method of any one of embodiments 31-63 or 127-141.
Embodiment 143: A scaffold biomaterial produced by the method of any one of embodiments 34-141.
Embodiment 144: The scaffold biomaterial of embodiment 143, which is a scaffold biomaterial as defined in any one of embodiments 1-15.
Embodiment 145: A kit comprising one or more of:
   a mold;
   a glue;
   one or more microchannels;
   one or more decellularisation agents;
   a scalpel or microtome;
   sterile measuring device;
   sterile saline;
   tweezers; and/or
   instructions for performing a method as defined in any one of embodiments 23-141.

One or more illustrative embodiments have been described by way of example. It will be understood to persons skilled in the art that a number of variations and modifications can be made without departing from the scope of the invention as defined in the claims.

## Claims

1. A method for supporting cell growth, said method comprising:
providing a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels; and
introducing one or more cells to the scaffold biomaterial.

2. A method for isolation and decellularisation of microchannels from a plant or fungal tissue, said method comprising:
separating microchannels from the plant or fungal tissue; and
decellularising the microchannels; and
optionally, sterilizing the microchannels.

3. The method of claim 2, wherein the step of separating the microchannels comprises mechanical separation of the microchannels, or vascular bundles containing the microchannels, or both, from surrounding plant or fungal tissue, wherein the step of separating is performed by gentle peeling, or by cutting.

4. The method of claim 2, wherein the step of separating the microchannels comprises mechanical separation of the microchannels, or vascular bundles containing the microchannels, or both, from surrounding plant or fungal tissue, wherein the step of separating is performed by liquid-based extraction of the microchannels from the plant or fungal tissue, preferably wherein liquid-based extraction comprises at least one of acid extraction, acid and peroxide extraction, salt extraction or alkaline extraction; preferably wherein acid and peroxide extraction comprises heating the plant or fungal tissue in an acid and peroxide solution; further preferably wherein the acid and peroxide solution comprises glacial acetic acid and 30% hydrogen peroxide in a ratio of 3:1 to 1:3; further preferably wherein the acid and peroxide solution comprises 1:1 glacial acetic acid:hydrogen peroxide solution (30% v/v).

5. The method of claim 4, wherein heating comprises heating up to 30 minutes, preferably heating for 30 minutes, wherein heating comprises boiling the acid and peroxide solution, preferably wherein the acid and peroxide solution comprises 1:1 glacial acetic acid:hydrogen peroxide solution (30% v/v) and the solution is heated to boiling for 30 minutes, preferably wherein the liquid-based extraction further comprises mechanically agitating, for example stirring, the plant or fungal tissue in the acid and peroxide solution.

6. The method of claim 4, wherein salt extraction comprises heating the plant or fungal tissue in a salt solution, preferably wherein the salt solution comprises LiCl or NaCl, preferably wherein the salt solution has a salt concentration of about 0.5M-3M, preferably wherein the salt solution comprises LiCl or NaCl at a salt concentration of about 3M, further preferably wherein heating comprises heating up to 30 minutes, further preferably wherein heating comprises heating for 30 minutes.

7. The method of claim 6, wherein heating comprises boiling the salt solution, preferably wherein the salt solution comprises about 3M LiCl and the solution is heated to boiling for 30 minutes or wherein the salt solution comprises about 3M NaCl and the solution is heated to boiling for 15 minutes, wherein the liquid-based extraction further comprises mechanically agitating, for example stirring, the plant or fungal tissue in the salt solution.

8. The method of claim 4, wherein alkaline extraction comprises heating the plant or fungal tissue in an alkaline solution, preferably wherein the alkaline solution has an alkaline concentration of about 0.5-3M, preferably wherein the alkaline solution comprises sodium hydroxide (NaOH), preferably wherein the alkaline solution comprises sodium hydroxide at an alkaline concentration of about 0.5M-1M, preferably wherein heating comprises heating up to 30 minutes, further preferably wherein heating comprises heating for 30 minutes.

9. The method of claim 8, wherein heating comprises boiling the alkaline solution, preferably wherein the alkaline solution comprises about 0.5M NaOH and the solution is heated to boiling for 5 minutes, preferably wherein the liquid-based extraction further comprises mechanically agitating, for example stirring, the plant or fungal tissue in the alkaline solution.

10. The method of claim 4, wherein acid extraction comprises heating the plant or fungal tissue in an acid solution, preferably wherein the acid solution comprises acetic acid or hydrochloric acid, wherein the acid solution comprises 50% acetic acid, preferably wherein heating comprises heating up to 30 minutes, further preferably for 30 minutes, preferably wherein heating comprises boiling the acid solution, wherein the acid solution comprises 50% acetic acid and the solution is heated to boiling for 30 minutes, wherein the liquid-based extraction further comprises mechanically agitating, for example stirring, the plant or fungal tissue in the acid and solution, wherein the step of decellularising occurs before the step of separating.

11. A scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels; for use in a method for transport of a material such as a drug to a site in in a subject.

12. A method for solution or material filtration and/or separation, said method comprising:
providing a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels; and
passing a solution or material through the scaffold biomaterial so as to filter and/or separate components from the solution based on size exclusion.

13. A method for plant modification or growth modulation, said method comprising:
providing a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels; and
grafting the scaffold biomaterial into the plant to provide for accelerated growth.

14. A method for preparing a structure mimicking a desired shape or object, said method comprising:
providing a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels;
carving or arranging the scaffold biomaterial so as to mimic the desired shape or object; and
optionally, gluing the scaffold biomaterial for structural reinforcement.

15. A method for exchanging or transferring heat in a microfluidics process, said method comprising:
providing a scaffold biomaterial comprising at least one bundle of microchannels, the bundle of microchannels comprising a plurality of decellularised microchannels isolated from plant or fungal tissue, the decellularised microchannels being arranged substantially parallel to each other within the bundle, wherein the plurality of decellularised microchannels are bundled together by gluing, by using sealants, by mechanical packing, by mechanical entanglement, by crosslinking, by suspending the microchannels in hydrogels, by threading the microchannels around each other, or by compressing/packing the microchannels or by fastening the microchannels; and
using the microchannels of the scaffold biomaterial to carry one or more fluids, wherein the one or more fluids are in close proximity so as to allow for heat exchange or heat transfer.
